(19) Europäisches Patentamt
European Patent Office
Office européen des brevets

(11) Publication number: **0 066 987**
**B1**

(12) **EUROPEAN PATENT SPECIFICATION**

(45) Date of publication of patent specification: **27.08.86**

(21) Application number: **82302556.4**

(22) Date of filing: **19.05.82**

(51) Int. Cl.⁴: **C 07 C 127/00,**
C 07 C 157/00, A 61 K 31/17,
C 07 C 147/00, C 07 D 295/12
// C07C93/06

(54) **N-(aryloxyalkyl)-N'-(amino-alkyl) ureas.**

(30) Priority: **20.05.81 US 265510**

(43) Date of publication of application:
**15.12.82 Bulletin 82/50**

(45) Publication of the grant of the patent:
**27.08.86 Bulletin 86/35**

(84) Designated Contracting States:
**AT BE CH DE FR GB IT LI LU NL SE**

(56) References cited:
**GB-A-1 031 165**
**US-A-2 344 934**

**ARZNEIMITTELFORSCHUNG, vol. 9, nos. 1-12, 1959, pages 113-120 PAUL P. KOELZER et al.: "Beziehungen zwischen chemischer Konstitution und pharmakologischer Wirkung bei mehreren Klassen neuer Lokalanaesthetica"**

(73) Proprietor: **A.H. ROBINS COMPANY, INCORPORATED**
**1407 Cummings Drive P.O. Box 26609**
**Richmond Virginia 23261 (US)**

(72) Inventor: **Shanklin, James Robert, Jr.**
**8318 Brookfield Road**
**Richmond Virginia 23227 (US)**
Inventor: **Johnson, Christopher Peter, III**
**3906 West Franklin Street**
**Richmond Virginia 23221 (US)**

(74) Representative: **Kearney, Kevin David Nicholas et al**
**KILBURN & STRODE 30 John Street**
**London, WC1N 2DD (GB)**

**EP 0 066 987 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

1. Field of invention

The present invention relates to N - (aryloxyalkyl) - N′ - (aminoalkyl)ureas and thioureas, their acid addition salts and hydrates thereof and such compounds for use in treating cardiac arrythmias in living animal bodies due to its cardiac antiarrhythmic effect and is also concerned with pharmaceutical methods and compositions associated therewith.

2. Description of the prior art

Procainamide hydrochloride which has the formula

$$NH_2C_6H_4—CONH—CH_2CH_2—N—(C_2H_5)_2 \cdot HCl$$

has been used in the art to suppress certain cardiac arrhythmias.

Recently, Protiva, M. et al. have disclosed in Czeck Patent 146,873 [C.A. 79, 42205 g] compounds such as

$$ClC_6H_4—O(CH_2)_3—CO—NH(CH_2)_2—N—(C_2H_5)_2$$

useful for lowering blood sugar levels in rats.

Joullie, M. et al. in Ger. Offen, 2,801,187 also have disclosed trimethoxyphenoxy carbamoyl compounds of the general structure

$$(CH_3O)_3C_6H_2—X—(CH_2)_m—Y—(CH_2)_n—NR_1,R_2$$

wherein X is O or $NR_3$ and Y is CONH, $R_3$ is hydrogen, benzyl or morpholinoethyl, m and n are 0, 1 and 2. Some of the compounds disclosed are ureas but they are never urea at the same time X is O, i.e., phenoxy. Use of the compounds as tranquilizers, sedatives, muscle relaxants and spasmolytics is disclosed.

Koelzer, P. P. and Wehr, K. H. in Arzneim. Forsch *9*, 113—20 (1959) disclosed two unsubstituted phenoxy compounds which we have found are useful in treating arrythmias. These compounds are:

N′-[2-(Diethylamino)ethyl]-N-methyl-N-[2-(phenoxy)ethyl]urea and

N-Methyl-N-[2-(phenoxy)ethyl]-N′-[2-(pyrrolidinyl)ethyl]urea

Anaesthetic activity in animals was disclosed but clinical use of these ureas was said to be unlikely.

Summary of the invention

The present invention is concerned with compounds useful for treating cardiac arrhythmias in an animal, the said compounds being N - (aryloxyalkyl) - N - (aminoalkyl)urea having the following structure formula:

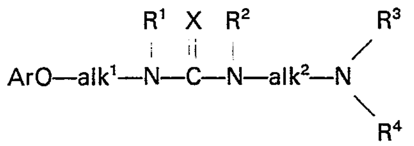

Formula I

wherein;

Ar represents a 1- or 2-naphthyl, 2,3-dihydro-1H-inden-4 (or 5)-yl, phenyl or substituted group, the said substituted phenyl group being phenyl substituted by 1—3 radicals which may be the same or different selected from halogen atoms or loweralkyl, loweralkoxy, trifluoromethyl, amino, nitro, loweralkylthio, loweralkylsulphinyl, loweralkylsulphonyl, loweralkanoyl, aroylamino or acylamino groups;

$R^1$ and $R^2$ each represents a hydrogen atom or a loweralkyl, cycloalkyl, phenyl or phenyl-loweralkyl wherein phenyl may be substituted by a halogen atom or a loweralkyl or lower alkoxy group;

X represents an oxygen atom or a sulphur atom;

$R^3$ and $R^4$ each represent a hydrogen atom or a loweralkyl, phenyl or phenylalkyl group wherein phenyl may be substituted by a halogen atom or a loweralkyl or lower alkoxy group, and $R^3$ and $R^4$ may be the same or different or $R^3$ and $R^4$ taken together with the adjacent nitrogen may form a heterocyclic residue;

alk$^1$ and alk$^2$ each represent a loweralkylene or loweralkylene-loweralkyl group and may be the same or different;

and the pharmaceutically acceptable addition salts and hydrates thereof.

In the further definition of symbols in the formulas hereof and where they appear elsewhere throughout this specification and claims, the terms have the following significance.

The term "loweralkyl" as used herein means straight and branched chain radicals of up to eight carbons inclusive and is exemplified by such groups as methyl, ethyl, propyl, isopropyl, butyl, sec. butyl, tert-butyl, amyl, isoamyl, hexyl, heptyl, and octyl radicals. The term "loweralkoxy" has the formula —O-loweralkyl.

The term "cycloalkyl" as used herein means primarily cyclic alkyl radicals containing 3 to 9 carbon atoms inclusive and includes such groups as cyclopropyl, cyclobutyl, cyclopentyl, cyclohexyl, methylcyclohexyl, cycloheptyl and the like.

The term "halogen" when referred to herein means fluorine, chlorine, bromine and iodine, preferably fluorine, chlorine and bromine.

The term "loweralkylene" as used herein means connecting hydrocarbon groups exemplified by methylene ($—CH_2—$), ethylene ($—CH_2—CH_2—$), and propylene ($—CH_2CH_2CH_2—$). The term "loweralkylene-loweralkyl" is represented by hydrocarbon groups such as ethylidene

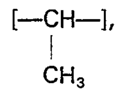

1,2-propylene 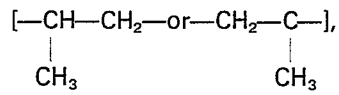

isopropylidene 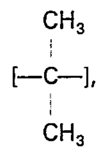, or

1,3-butylene 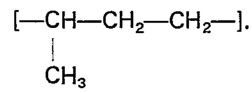

The term "heterocyclic residue" as used herein means pyrrolidine, piperidine, piperazine, 4-loweralkyl-piperazine or morpholino radicals.

"Pharmaceutically acceptable acid addition salts" are those salts formed by the N - (aryloxyalkyl) - N' - (aminoalkyl) ureas of this invention with any acid which is physiologically compatible in warm blooded animals, such salts being formed by either strong or weak acids. Representative of strong acids are hydrochloric, sulphuric and phosphoric acids.

Representative of weak acids are fumaric, maleic, succinic, tartaric, oxalic, citric, and cyclohexamic acid.

The compounds of the present invention exhibit anti-arrhythmic activity in dogs, several arrhythmia models in which arrhythmia is induced by one or more of the following is described more fully hereinbelow under pharmacology: 1) Ouabain, (2) Ligation, (3) Injury, and (4) acetylcholine.

It is therefore an object of the present invention to provide compositions and compounds for use in treating cardiac arrhythmias in living animal bodies the said compounds being certain N - (aryloxyalkyl) - N' - (aminoalkyl) ureas (and aminoalkyl thioureas).

Another object of the invention is to provide certain novel N - (aryloxyalkyl) - N' - (aminoalkyl) ureas (and thioureas), salts and hydrates thereof, which have a high degree of cardiological activity and methods for making such compounds.

## Detailed description of the invention

The invention provides N - (aryloxyalkyl) - N' - (aminoalkyl)ureas and thioureas and derivates thereof as set forth hereinabove in Formula I and definitions therewith for use in treating arrhythmias in living animals and pharmaceutical compositions containing such compounds which are administered to a living animal body for cardiac arrhythmic effect in an amount effective to control arrhythmia.

## Description of best method

The invention may be put into practice in various ways and a number of specific embodiments will be described to illustrate the invention. A general discussion of preparative methods will be given first followed by reference to preparations 1 to 42 which are of starting materials and Examples 1 to 45, and 47 to 65 which are of compounds in accordance with the present invention, Example 46 which is of an intermediate and Examples 66 to 70 which are examples of compositions in accordance with the invention.

The compounds of Formula I wherein X=oxygen may be prepared by one of 4 methods, A, B, C or D. Compounds wherein X=sulphur may be prepared by method A or D starting with thiophosgene.

### Method A

This method is represented by the following equation:

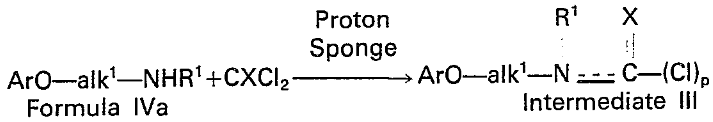


Formula IVa                                        Intermediate III

Intermediate III
+

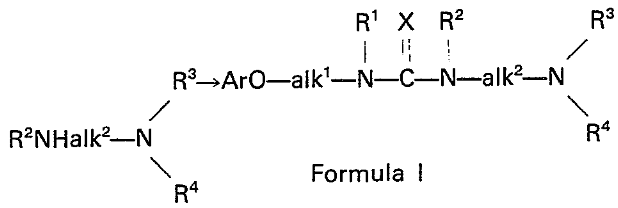

Formula IIa                                        Formula I

wherein;

Ar, $R^1$, $R^2$, $R^3$, $R^4$ and alk$^1$ and alk$^2$ are as defined hereinabove and when $R^1$ represents a hydrogen atom, p is zero and the dotted line is a double bond forming an isocyanate; otherwise p is 1 and the dotted line has no significance; with the proviso that when $R^2$ does not represent a hydrogen atom, $R^3$ and $R^4$ must be other than a hydrogen atom, or $R^2$ is the same as $R^3$, and $R^4$ represents a hydrogen atom.

Generally, in Method A the aryloxyalkyl amine is reacted with phosgene (or thiophosgene) in a suitable organic solvent plus Proton Sponge (Registered Trade Mark), (which is 1,8 - bis - (dimethylamino)-naphthalene) followed by extraction (washing) with dilute sulphuric acid and the oragnic layer is dried and evaporated to an oil (Intermediate, Formula III) which may, if desired, be isolated as in Example 46 and later used as in Example 3A described below. The oil is dissolved in tetrahydrofuran and reacted with an amine of Formula IIa. The reaction mixture is stripped to dryness and the residue partitioned between water and a solvent such as methylene chloride. Evaporation of the solvent yields an oil which may or may not crystallize. Pharmaceutically acceptable salts may be prepared by reacting with an appropriate acid. The method is illustrated more specifically in Examples 1, 2, 3 and other examples wherein aryloxyamines are reacted with phosgene or thio phosgene.

Method B

This method is represented by the following equation:

$$H_2N{-}alk^2NR^3R^4 + CDI + ArO{-}alk^1{-}NHR^1 \rightarrow$$

Formula IIb                    Formula IVa

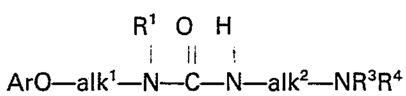

Formula Ib

where CDI is 1,1'-carbonyldiimidazole wherein Ar, $R^1$, $R^3$, $R^4$, alk$^1$ and alk$^2$ are as defined hereinabove. Formula Ib is encompassed by Formula I and $R^2$ always represents a hydrogen atom in this method.

Generally, in Method B an alkyldiamine is reacted first with 1,1'-carbonyldiimidazole in a suitable solvent (e.g., tetrahydrofuran) followed by reaction with a solution of the aryloxy-alkylamine. The reaction mixture is quenched in water and extracted with a suitable solvent (e.g., methylene chloride) or the reaction mixture is evaporated to dryness and the residue partitioned between water and a suitable organic solvent. The organic layer in either case is dried and evaporated to yield an oil, the free base. Pharmaceutically acceptable acid addition salts may then be provided with a suitable acid. The method is illustrated more fully in Example 18.

Method C

This method is represented by the following equation:

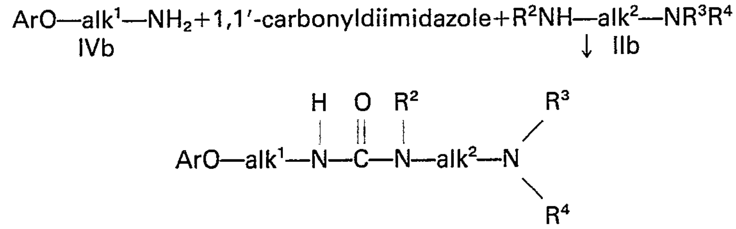

IVb IIb

Formula Ic

wherein Ar, $R^2$, $R^3$, $R^4$, $alk^1$ and $alk^2$ are as defined hereinabove and $R^1$ always represents a hydrogen atom in this method. Formula Ic is encompassed by Formula I.

Generally, in Method C an aryloxyalkyl amine is reacted first with 1,1′-carbonyldiimidazole in a suitable solvent (e.g., tetrahydrofuran) followed by reaction with an alkyldiamine having one free hydrogen. The solvent is removed by evaporation and the residue partitioned between a suitable solvent (e.g., chloroform) and water. The free base is obtained by evaporation and may be converted to a pharmaceutically acceptable salt with a suitable acid. The method is illustrated more fully in Example 33.

Method D

This method is represented by the following equation:

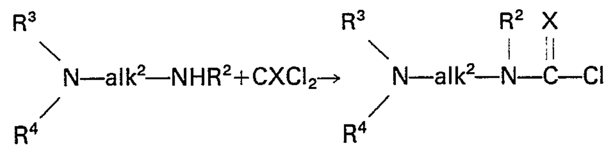

Formula IIa Intermediate V

Intermediate V
+
triethylamine
+
$Ar—O—alk^1—NHR^1$
Formula IVa

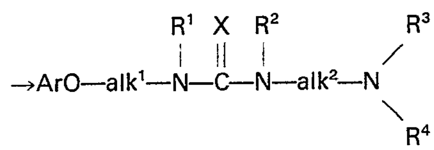

Formula Id

wherein;

Ar, $alk^1$, $alk^2$, $R^1$ and $R^2$ have the values assigned above except $R^2$, $R^3$ and $R^4$ never represent a hydrogen atom. Formula Id is encompassed by Formula I. See Example 52 for a demonstration of Method D.

Compounds wherein aryl is phenyl substituted by amino may be obtained by catalytic hydrogenation of the corresponding nitro derivative; for example, over palladium-on-charcoal.

Starting compounds of Formula IVa may be prepared by reacting aryloxyalkyl halides and the appropriate amine. Starting compounds of Formula IVb may be prepared by reacting the aryloxyalkyl halide with potassium phthalimide followed by reaction with hydrazine hydrate. Starting compounds of Formula IIa and IIb are commercially available or may be prepared from the appropriate aminoalkyl chloride and potassium phthalimide followed by reaction with hydrazine hydrate.

Preparations 1 to 42 are now given to more fully illustrate the preparation of starting compounds.

Preparation 1

2-(3,4-Dichlorophenoxy)-N-(1-methylethyl)ethanamine, hydrochloride

A solution of 36.18 g (0.135 mole) of 2-bromoethyl-3,4-dichlorophenyl ether and 31.8 g (0.54 mole) of isopropyl amine in 200 ml of chloroform was heated at reflux for 72 hours. Solvent was removed in a rotary evaporator and the residue partitioned between water and chloroform. The chloroform layer was extracted with 1N sulphuric acid. The aqueous phase, which contained a dispersed white solid, was made alkaline with 10% aqueous sodium hydroxide solution and extracted with chloroform. The chloroform layer was concentrated under vacuum to an oil, the free base. A portion of the oil was dissolved in methanol and treated with ethereal hydrogen chloride. The precipitate was recrystallized from methanol-diethyl ether. Overall yield of white crystalline product was 62.4% of theory based on the proportion taken. The melting point was 186—187°C.

Analysis:

| | |
|---|---|
| Calculated for $C_{11}H_{15}NOCl_3$: | C, 46.42; H, 5.67; N, 4.92 |
| Found: | C, 46.53; H, 5.69; N, 5.00 |

Preparation 2

2-(3,5-Dichlorophenoxy)-N-(1-methylethyl)ethanamine, hydrochloride

Following the procedure of Preparation 1, 2-bromoethyl-3,5-dichlorophenyl ether and isopropyl amine were reacted in refluxing chloroform overnight to yield an oil, the free base of the title compound. Thereafter, a portion of the oil was reacted with ethereal hydrogen chloride to form the hydrochloride salt in 43.4% yield, m.p. 195—197°C.

Analysis:

| | |
|---|---|
| Calculated for $C_{11}H_{16}NOCl_3$: | C, 46.42; H, 5.67; N, 4.92 |
| Found: | C, 46.38; H, 5.64; N, 4.95 |

Preparation 3

3-(3,5-Dichlorophenoxy)-N-(1-methylethyl)-1-propanamine, hydrochloride

Following the procedure of Preparation 1, a mixture of 3 - [3,5 - dichlorophenoxy] - 1 - chloropropane and 3 - [3,5 - dichlorophenoxy] - 1 - bromopropane and isopropyl amine were reacted in refluxing chloroform overnight and the reaction mixture processed to yield an oil, the free base of the title compound. A portion of the oil was reacted with ethereal hydrogen chloride to form the hydrochloride salt; m.p. 157—159°C.

Analysis:

| | |
|---|---|
| Calculated for $C_{12}H_{18}NOCl_3$: | C, 48.26; H, 6.08; N, 4.69 |
| Found: | C, 48.29; H, 6.03; N, 4.76 |

Preparation 4

N-(1-Methylethyl)-2-phenoxyethanamine, hydrochloride

Following the procedure of Preparation 1, 2-bromoethyl phenyl ether and isopropyl amine were reacted (in refluxing chloroform) overnight and the reaction mixture processed to yield an oil, the free base of the title compound. A portion of the oil was reacted with ethereal hydrogen chloride to form the hydrochloride salt in 68.3% yield, m.p. 152—154.5°C.

Analysis:

| | |
|---|---|
| Calculated for $C_{11}H_{18}NOCl$: | C, 61.25; H, 8.41; N, 6.49 |
| Found: | C, 61.50; H, 8.50; N, 6.53 |

Preparation 5

2-[2-[[N,N-bis(1-Methylethyl)]amino]ethyl]-2H-isoindole-1,3-dione, hydrochloride

A solution of 40.0 g (0.2 mole) of 2-diisopropylaminoethyl chloride hydrochloride and 74.0 g (0.4 mole) of potassium phthalimide in 500 ml of dimethylformamide were stirred overnight at 85°C. The reaction mixture was stripped to dryness on a rotary evaporator under reduced pressure, and the residue was partitioned between chloroform and water. The chloroform layer was extracted with 10% aqueous sodium hydroxide, dried over sodium sulphate and stripped to yield a brown oil, the free base of the title compound which slowly crystallized. A portion of the oil was reacted with ethereal hydrogen chloride to form the hydrochloride salt, which was recrystallized from methanol-diethyl ether, in 62.3% yield, m.p. 209—211°C.

Analysis:

| | |
|---|---|
| Calculated for $C_{16}H_{23}N_2O_2Cl$: | C, 61.83; H, 7.46; N, 9.01 |
| Found: | C, 61.52; H, 7.42; N, 8.93 |

Preparation 6

N,N-bis(1-Methylethyl)-1,2-ethanediamine, dihydrochloride

A solution of 40.54 g (0.148 mole) of 2 - [2 - [[N,N - bis(1 - methylethyl)]amino]ethyl] - 2H - isoindole - 1,3 - dione (oil in Preparation 5 and 11.8 g (0.2 mole) of 85% hydrazine hydrate in 400 ml of 95% ethyl alcohol was heated at reflux for 5 hr. The reaction mixture was allowed to cool to room temperature while standing overnight during which time a white solid precipitated. The reaction mixture was concentrated nearly to dryness in a rotary evaporator. The residue was dissolved in chloroform and extracted with 10% aqueous sodium hydroxide solution. Evaporation of the chloroform layer gave a brown oil, the free base of the title compound. A portion of the oil was reacted with ethereal hydrogen chloride to form the hydrochloride salt which was recrystallized from methanol-diethyl ether. Yield was 20% of theory, m.p. 178—182°C. (d).

Analysis:

| | |
|---|---|
| Calculated for $C_8H_{22}N_2Cl_2$: | C, 44.24; H, 10.21; N, 12.90 |
| Found: | C, 43.93; H, 10.15; N, 12.83 |

Preparation 7

N-[2-(3,4-Dimethoxyphenoxy)ethyl]-1-methylethanamine, hydrochloride

A solution of 17.06 g (0.068 mole) of 2-bromoethyl 3,4-dimethoxyphenyl ether in 500 ml of isopropyl amine was stirred overnight at room temperature. The reaction mixture was stripped to dryness and the residue partitioned between water and chloroform. The chloroform layer was then extracted with 10% sodium hydroxide. The organic layer was then extracted with 1N sulphuric acid. The acidic layer was then made alkaline and extracted with chloroform. Removal of chloroform gave a brown oil, the free base of the title compound. Three grams of the oil was converted to the hydrochloride salt and recrystallized from methanol-diethylether. 2.67 g (58.5%) of white crystalline product, m.p. 139—141°C was obtained after drying overnight *in vacuo* at 100°C.

Analysis:

| | |
|---|---|
| Calculated for $C_{13}H_{22}NO_3Cl$: | C, 56.62; H, 8.04; N, 5.08 |
| Found: | C, 56.53; H, 8.06; N, 5.10 |

Preparation 8

N-[2-(2,4-Dichlorophenoxy)ethyl]-1-methylethanamine, maleate [1:1].

1-Bromo-2-(2,4-dichlorophenoxy)ethane, 72.22 g (0.27 mole), and isopropyl amine in excess as solvent were reacted at room temperature with stirring for 18 hr and the reaction mixture processed to yield a solid, the free base of the title compound. A portion of the solid was reacted with maleic acid and recrystallized from methanol-methylene chloride-diethyl ether to yield a white crystalline solid, m.p. 143—145°C.

Analysis:

| | |
|---|---|
| Calculated for $C_{15}H_{19}NO_5Cl_2$: | C, 49.47; H, 5.26; N, 3.85 |
| Found: | C, 49.37; H, 5.22; N, 3.88 |

Preparation 9

N-[2-(3,5-Dichlorophenoxy)ethyl]benzeneamine, hydrochloride

A solution of 16.08 g (0.06 mole) 3,5-dichlorophenyl-2-bromoethyl ether in 100 ml aniline (excess) was stirred overnight at room temperature. The unreacted aniline was removed in a rotary evaporator with vacuum distillation. The residue was triturated with isopropyl ether and chilled in a methanol-dry ice bath. A purple solid aniline hydrobromide was filtered from the mixture and discarded. The filtrate was evaporated to dryness yielding 15.25 g of oil, the free base of the title compound. A portion of the oil was reacted with ethereal hydrogen chloride to form the hydrochloride salt which was recrystallized from methanoldiethyl ether in 40.0% overall yield, m.p. 193—196°C.

Analysis:

| | |
|---|---|
| Calculated for $C_{14}H_{14}NOCl_3$: | C, 52.77; H, 4.43; N, 4.40 |
| Found: | C, 52.79; H, 4.39; N, 4.59 |

Preparation 10

1-(2-Bromoethoxy)naphthalene.

A solution of 144.0 g (1 mole) α-naphthol and 56.1 g (1 mole) potassium hydroxide in 600 ml of 95% ethanol was stirred for 1.5 hr at room temperature. To this solution of potassium naphthalate was added 930.0 g (5.0 mole) of 1,2-dibromoethane, and the solution was heated at reflux overnight. The reaction mixture was filtered, and the filtrate stripped to dryness under reduced pressure in a rotary evaporator. The resulting oil was dissolved in chloroform and the solution extracted with 10% aqueous sodium hydroxide. The chloroform layer was dried and stripped of chloroform to yield 126 g (50.3%) of dark brown oil.

Preparation 11

1-Methyl-N-[2-(1-naphthalenyloxy)ethyl]ethanamine hydrochloride

A solution of 25.0 g (0.1 mole) of 2-bromoethyl-α-naphthyl ether and 100 ml of isopropyl amine (excess) was stirred at room temperature overnight. The reaction mixture was stripped to dryness and partitioned between chloroform and water. Evaporation of the chloroform layer gave an oil, the free base of the title compound. A portion of the oil was reacted with ethereal hydrogen chloride and the hydrochloride salt recrystallized from methanol-diethyl ether. Overall yield was 89.3%, m.p. 198—200°C.

Analysis:

| | |
|---|---|
| Calculated for $C_{15}H_{20}NOCl$: | C, 67.79; H, 7.56; N, 5.27 |
| Found: | C, 67.75; H, 7.61; N, 5.24 |

Preparation 12

N-[2-(3-Chlorophenoxy)ethyl]-1-methylethanamine, hydrochloride

Following the procedure of Preparation 11, 2-bromoethyl-3-chlorophenyl ether and isopropyl amine in excess were reacted and the reaction mixture processed to yield an oil, the free base of the title compound. A portion of the oil was converted to the hydrochloride salt (77.8%), m.p. 153.5—155.5°C.

Analysis:

| | |
|---|---|
| Calculated for $C_{11}H_{17}NOCl_2$: | C, 52.81; H, 6.85; N, 5.60 |
| Found: | C, 52.83; H, 6.89; N, 5.69 |

Preparation 13

N-[2-(2,6-Dichlorophenoxy)ethyl]-1-methylethanamine, maleate

Following the procedure of Preparation 11, 30.0 g (0.112 mole) of 2-bromoethyl-2,6-dichlorophenyl ether and isopropyl amine in excess were reacted and the reaction mixture processed to yield an oil, the free base of the title compound. A portion of the oil was converted to the maleate salt, m.p. 143—144°C.

Analysis:

| | |
|---|---|
| Calculated for $C_{15}H_{19}NO_5Cl_2$: | C, 49.46; H, 5.26; N, 3.84 |
| Found: | C, 49.55; H, 5.30; N, 3.84 |

Preparation 14

2-[2-(3,5-Dichlorophenoxy)ethyl]-1H-isoindole-1,3(2H)-dione

A mixture of 101.88 g (0.38 mole) of 2-bromoethyl-3,5-dichlorophenyl ether and 70.3 g (0.38 mole) of potassium phthalimide in 800 ml of dimethylformamide was stirred overnight at 85°C. The reaction mixture was filtered and dimethylformamide removed from the filtrate in a rotary evaporator to yield a white solid. The solid was triturated with diethyl ether and the mixture filtered. The solid was dried *in vacuo*. Recrystallized from methylene chloridehexane, the product melted at 133—136°C. Yield overall was 41.7%.

Analysis:

| | |
|---|---|
| Calculated for $C_{16}H_{11}NO_3Cl_2$: | C, 57.17; H, 3.30; N, 4.17 |
| Found: | C, 57.04; H, 3.29; N, 4.15 |

Preparation 15

2-(3,5-Dichlorophenoxy)ethanamine, hydrochloride

A mixture of 105.5 g (0.31 mole) of 2 - [2 - (3,5 - dichlorophenoxy)ethyl] - 1H - isoindole - 1,3 - (2H) - dione and 24.5 g (0.41 mole) 85% hydrazine hydrate in 1 litre of 95% ethanol was heated at reflux for 4 hr. A white solid was filtered off and discarded. The filtrate was stripped to dryness and the residue partitioned between water and chloroform. The chloroform layer was washed with aqueous 10% sodium hydroxide solution and then extracted with 1N sulphuric acid. The aqueous acidic layer was made alkaline and extracted with chloroform. The chloroform layer was dried over magnesium sulphate and evaporated to obtain an oil, the free base of the title compound. A portion of the oil was reacted with ethereal hydrogen chloride to give a white crystalline product in 43.2% yield, m.p. 229—231°C.

Analysis:

| | |
|---|---|
| Calculated for $C_8H_{10}NOCl_3$: | C, 39.62; H, 4.16; N, 5.78 |
| Found: | C, 39.22; H, 4.11; N, 5.86 |

Preparation 16

N-(1-Methylethyl)-2-(3,5-dimethylphenoxy)ethanamine, maleate

A solution of 36.2 g (0.159 mole) of 1-bromo-2-(3,5-dimethylphenoxy)ethane in 400 ml of isopropyl amine (excess) was stirred at room temperature for 20 hr. The reaction mixture was quenched in a large excess of dilute aqueous sodium hydroxide solution and the mixture extracted with methylene chloride. The methylene chloride extract was washed several times with dilute aqueous sodium hydroxide solution and dried over magnesium sulphate. The methylene chloride was removed *in vacuo* to give an oil, the free base of the title compound, in an amount of 28.3 g. A small portion of the oil was reacted with maleic acid and recrystallized from methanol-diethyl ether to give a white crystalline solid, m.p. 158—159.5°C.

Analysis:

| | |
|---|---|
| Calculated for $C_{17}H_{25}NO_5$: | C, 63.14; H, 7.79; N, 4.33 |
| Found: | C, 63.14; H, 7.80; N, 4.32 |

Preparation 17

N-(1-Methylethyl)-2-(3,5-dimethoxyphenoxy)ythanamine, maleate

Following the procedure of Preparation 16, 1-bromo-2-(3,5-dimethoxyphenoxy)ethane and excess isopropyl amine were reacted and the reaction mixture processed to give an oil, the free base of the title compound. A portion of the oil was reacted with maleic acid. The white crystalline solid melted at 128—129.5°C.

Analysis:

| | |
|---|---|
| Calculated for $C_{17}H_{25}NO_7$: | C, 57.45; H, 7.09; N, 3.94 |
| Found: | C, 57.35; H, 7.14; N, 3.97 |

Preparation 18

N-[2-(3,4-Dimethylphenoxy)ethyl]-1-methylethanamine, hydrochloride

Following the procedure of Preparation 16, 1-bromo-2-(3,4-dimethylphenoxy)ethane and excess isopropyl amine were reacted and the reaction mixture processed to give an oil, the free base of the title compound. A portion of the oil was reacted with ethereal hydrogen chloride. The white crystalline solid melted at 143—149.5°C.

Analysis:

| | |
|---|---|
| Calculated for $C_{13}H_{22}NOCl$: | C, 64.05; H, 9.10; N, 5.75 |
| Found: | C, 64.13; H, 9.18; N, 5.86 |

Preparation 19

N-[2-(4-Chlorophenoxy)ethyl]-1-methylethanamine, hydrochloride

Following the procedure of Preparation 11, 2-bromoethyl-4-chlorophenyl ether and isopropyl amine (excess) were reacted and the reaction mixture processed to give an oil, the free base of the title compound. A portion of the oil was reacted with ethereal hydrogen chloride to give the hydrochloride salt, m.p. 162—164°C. in 69.3% yield.

Analysis:

| | |
|---|---|
| Calculated for $C_{11}H_{17}NOCl_2$: | C, 52.81; H, 6.85; N, 5.60 |
| Found: | C, 52.72; H, 6.88; N, 5.71 |

Preparation 20

N-[2-(2,5-Dichlorophenoxy)ethyl]-1-methylethanamine hydrochloride

Following the procedure of Preparation 11, 2-bromoethyl-2,5-dichlorophenyl ether and isopropyl amine (excess) were reacted and the reaction mixture processed to give an oil, the free base of the title compound. A portion of the oil was reacted with ethereal hydrogen chloride to give the hydrochloride salt, m.p. 152—155°C in 63.3% yield.

Analysis:

| | |
|---|---|
| Calculated for $C_{11}H_{16}NOCl_3$: | C, 46.42; H, 5.67; N, 4.92 |
| Found: | C, 46.39; H, 5.67; N, 4.94 |

Preparation 21

1-Methyl-N-[2-(3-trifluoromethylphenoxy)ethyl]ethanamine maleate

Following the procedure of Preparation 16, 1-bromo-2-(3-trifluoromethylphenoxy)ethane and isopropylamine were reacted and the reaction mixture processed to give an oil, the free base of the title compound. A portion of the oil was reacted with maleic acid to give the maleate salt, m.p. 125.5—126.5°C.

Analysis:

| | |
|---|---|
| Calculated for $C_{16}H_{20}NO_5F$: | C, 52.89; H, 5.55; N, 3.86 |
| Found: | C, 52.79; H, 5.57; N, 3.83 |

Preparation 22

2-(3,5-Dichlorophenoxy)-N-methylethanamine, hydrochloride

A solution of 31.56 g (0.118 mole) 2-bromoethyl-3,5-dichlorophenyl ether in excess methylamine was agitated overnight in a bomb at room temperature. The methylamine was evaporated under nitrogen and the residue partitioned between water and methylene chloride. The methylene chloride was evaporated to give an oil, the free base of the title compound. A portion of the oil was reacted with ethereal hydrogen chloride to give the hydrochloride salt which was recrystallized from methanol-diethyl ether to give a white crystalline product in 35.7% overall yield, m.p. 173—175°C.

Analysis:

| | |
|---|---|
| Calculated for $C_9H_{12}NOCl_3$: | C, 42.13; H, 4.72; N, 5.46 |
| Found: | C, 42.01; H, 4.69; N, 5.59 |

Preparation 23

N-[2-(3-Fluorophenoxy)ethyl]-1-methylethanamine, maleate

A solution of 20.94 g (0.096 mole) of 1-bromo-2-(3-fluorophenoxy)ethane in 300 ml of isopropylamine was stirred at room temperature overnight. The excess amine was removed *in vacuo* to give an oil (15.29 g), the free base of the title compound. A portion of the oil was reacted with maleic acid to give the maleate salt which was recrystallized from methanol-diethyl ether, m.p. 141—142°C.

Analysis:

| | |
|---|---|
| Calculated for $C_{15}H_{20}NO_5F$: | C, 57.50; H, 6.43; N, 4.47 |
| Found: | C, 57.51; H, 6.48; H, 4.44 |

Preparation 24

N-[2-(2,3-Dichlorophenoxy)ethyl]-1-methylethanamine, hydrochloride

Following the procedure of Example 11, 2-bromoethyl-2,3-dichlorophenyl ether was reacted with isopropyl amine in excess and the reaction mixture processed to give an oil, the free base of the title compound. A portion of the oil was reacted with ethereal hydrogen chloride to give the salt, m.p. 161—163.5°C in 62.7% yield.

Analysis:

| | |
|---|---|
| Calculated for $C_{11}H_{16}NOCl_3$: | C, 46.42; H, 5.67; N, 4.92 |
| Found: | C, 46.23; H, 5.69; N, 5.02 |

Preparation 25

N-[2-(2-Chlorophenoxy)ethyl]-1-methylethanamine hydrochloride

Following the procedure of Example 11, 2-bromoethyl-2-chlorophenyl ether was reacted with isopropyl amine in excess and the reaction mixture processed to give an oil, the free base of the title

compound. A portion of the oil was reacted with ethereal hydrogen chloride and the hydrochloride salt recrystallized from methanol-diethyl ether, m.p. 118—119.5°C (75%).

Analysis:

| | |
|---|---|
| Calculated for $C_{11}H_{17}NOCl_2$: | C, 52.81; H, 6.85; N, 5.60 |
| Found: | C, 52.77; H, 6.80; N, 5.64 |

Preparation 26

2-(2-Bromoethoxy)naphthalene

Following the procedure of Preparation 10 and substituting β-naphthol for α-naphthol, the title compound was obtained as brown solid which, when triturated with diethyl ether, gave a light brown solid, m.p. 91.5—93.0°C in 28% yield.

Analysis:

| | |
|---|---|
| Calculated for $C_{12}H_{11}OBr$: | C, 57.40; H, 4.42; |
| Found: | C, 57.69; H, 4.40 |

Preparation 27

1-Methyl-N-[2-(2-naphthalenyloxy)ethyl]ethanamine, hydrochloride

Following the procedure of Preparation 11, 2-(2-bromoethoxy)naphthalene was reacted with isopropyl amine in excess and the reaction mixture processed to give an oil, the free base of the title compound. A portion of the oil was converted to the hydrochloride salt (48%), m.p. 179—180.5°C.

Analysis:

| | |
|---|---|
| Calculated for $C_{15}H_{20}NOCl$: | C, 67.79; H, 7.59; N, 5.27 |
| Found: | C, 67.92; H, 7.62; N, 5.34 |

Preparation 28

N-[2-[(2,3-Dihydro-1H-inden-5-yl)oxy]ethyl]-1-methylethanamine, hydrochloride

The potassium salt of 5-indanol was prepared from 97.0 g (0.723 mole) of 5-indanol and 40.5 g (0.723 mole) potassium hydroxide in 1 litre of 95% ethanol with stirring for 1 hr at room temperature. To this solution was added 679.2 g (3.615 mole) of 1,2-dibromoethane. The resulting solution was heated at reflux overnight. The reaction mixture was filtered and stripped to dryness. The residue was dissolved in chloroform and washed with 10% aqueous sodium hydroxide solution. The chloroform layer was dried and evaporated to dryness to obtain a dark brown oil. A portion of the oil, 46.04 g, was added to 200 ml of isopropylamine and the mixture stirred overnight at room temperature. The excess amine was stripped off to dryness and the residue partitioned between water and chloroform. Removal of chloroform from the chloroform layer produced a dark brown oil, the free base of the title compound. A portion of this oil was converted to the hydrochloride salt with ethereal hydrogen chloride which, on recrystallization from methanol-diethyl ether (24%), melted at 154—155.5°C.

Analysis:

| | |
|---|---|
| Calculated for $C_{14}H_{22}NOCl$: | C, 65.74; H, 8.67; N, 5.48 |
| Found: | C, 65.22; H, 8.65; N, 5.43 |

Preparation 29

N-[2-[(2,3-Dihydro-1H-inden-4-yl)oxy]ethyl]-1-methylethanamine, hydrochloride

A solution of 24.0 g (0.1 mole) of 4-indanyl-2-bromoethyl ether in 100 ml of isopropylamine was stirred overnight at room temperature. The solution was stripped of excess isopropylamine and the residue partitioned between methylene chloride and water. The methylene chloride layer was evaporated to give a dark brown oil. The oil was reacted with ethereal hydrogen chloride and the mixture filtered. The solid was recrystallized from methanol-diethyl ether to give 16.95 g (66.3%) of white crystals, m.p. 160—162°C.

Analysis:

| | |
|---|---|
| Calculated for $C_{14}H_{22}NOCl$: | C, 65.74: H, 8.67; N, 5.48 |
| Found: | C, 65.75; H, 8.68; N, 5.46 |

Preparation 30

2-[2-[(2,3-Dihydro-1H-inden-4-yl)oxy]ethyl]-1-H-isoindole-1,3-(2H)-dione

A solution of 54.01 g (0.226 mole) of 4-(2-bromoethoxy) indan and 41.81 g (0.226 mole) of potassium phthalimide in 1200 ml of dimethylformamide was stirred overnight at 85°C. The reaction was filtered and dimethylformamide removed *in vacuo* in a rotary evaporator to give a white solid. The solid was dissolved in methylene chloride and extracted with water. Methylene chloride was evaporated to give a light brown solid. The solid was triturated with diethyl ether to give 59.3 g light brown solid. A portion of the solid was recrystallized from methylene chloride-ethanol-diethyl ether to give a white crystalline solid, m.p. 128.5—132.0°C.

Analysis:

| | |
|---|---|
| Calculated for $C_{19}H_{17}NO_3$: | C, 74.26; H, 5.58; N, 4.56 |
| Found: | C, 73.97; H, 5.46; N, 4.82 |

Preparation 31

N-[2-[(2,3-Dihydro-1H-inden-4-yl)oxy]ethyl]-ethanamine maleate [1:1].

A solution of 55.28 g (0.18 mole) of 2 - [2 - [(2,3 - dihydro - 1H - inden - 4 - yl)oxy]ethyl] - 1H - isoindole - 1,3 - (2H) dione and 13.0 g (0.22 mole) hydrazine hydrate in 500 ml of 95% ethyl alcohol was heated at reflux for 5 hr. On filtration, a white solid was obtained. The solid was partitioned between chloroform and 10% sodium hydroxide. The ethyl alcohol was removed from the filtrate in a rotary evaporator and the residue was partitioned between chloroform and 10% sodium hydroxide. The chloroform extracts were combined, filtered and evaporated to give an oil (27.20 g) (free base). A portion of the oil was reacted with maleic acid and the salt recrystallized from methanol-diethyl ether to give 3.94 g (57.3%) of white crystalline product; m.p. 150.5—152°C.

Analysis:

| | |
|---|---|
| Calculated for $C_{15}H_{19}NO_5$: | C, 61.42; H, 6.53; N, 4.78 |
| Found: | C, 61.35; H, 6.53; N, 4.75 |

Preparation 32

N-[2-(3,5-Dichlorophenoxy)ethyl]cyclohexaneamine hydrochloride

A solution of 27.2 g (0.1 mole) of 2-bromoethyl-3,5-dichlorophenyl ether in 300 ml of cyclohexylamine (excess) was stirred overnight at room temperature. The reaction mixture was filtered and the filtrate stripped to dryness on a rotary evaporator. The residue was partitioned between chloroform and water and between chloroform and 10% sodium hydroxide. The chloroform layer was evaporated to dryness leaving an oil (free base). The oil was dissolved in methanol and reacted with ethereal hydrogen chloride to give 25.8 g (94.2%) solid, m.p. 216.5—220°C.

Analysis:

| | |
|---|---|
| Calculated for $C_{14}H_{20}NOCl_3$: | C, 51.79; H, 6.21; N, 4.31 |
| Found: | C, 51.92; H, 6.22; N, 4.34 |

Preparation 33

N-Methyl-2-phenoxyethanamine hydrochloride

A solution of 71.42 g (0.307 mole) of 2-bromoethyl phenylether in excess methylamine was agitated at room temperature overnight in a steel bomb. Unreacted methylamine was allowed to evaporate by opening the bomb. The residue was dissolved in chloroform and washed with water by extraction. Evaporation of chloroform left a light yellow oil (free base) which was dissolved in methanol and reacted with ethereal hydrogen chloride. Recrystallization of the solid from methanol-diethyl ether gave 25.19 g (43.9%) of white crystals, mp. 173.5—175.0°C.

Analysis:

| | |
|---|---|
| Calculated for $C_9H_{14}NOCl$: | C, 57.60; H, 7.52; N, 7.46 |
| Found: | C, 57.38; H, 7.53; N, 7.42 |

Preparation 34

N-[-2-(3,5-Dichlorophenoxy)ethyl]butanamine hydrochloride

A solution of 33.76 g (0.126 mole) of 2-(3,5-dichlorophenoxy)-1-bromoethane in 200 ml of n-butylamine was stirred at 25°C for 20 hr. The excess n-butylamine was evaporated *in vacuo*, and the residue was suspended in a mixture of dilute sodium hydroxide and shaken with methylene chloride. The methylene chloride layer was extracted in sequence with two additional portions of dilute sodium hydroxide, one of dilute sulphuric acid and once more with dilute sodium hydroxide. The volume of the methylene chloride layer was reduced to about 150 ml and excess ethereal hydrogen chloride was added. Addition of anhydrous ether gave a white precipitate which was collected and dried under high vacuum (78°C) to give 19.35 g (51.5%) of white crystals, m.p. 195—196°C.

Analysis:

| | |
|---|---|
| Calculated for $C_{12}H_{18}NOCl_3$: | C, 48.26; H, 6.08; N, 4.69 |
| Found: | C, 48.17; H, 6.07; N, 4.73 |

Preparation 35

N-[-2-(3,5-Dichlorophenoxy)ethyl]propanamine hydrochloride

The title compound was prepared by the method of Preparation 34 from 2 - (3,5 - dichlorophenoxy) - 1 - bromoethane, n-propylamine and hydrogen chloride. The free base was obtained as an oil. The hydrochloride salt melted at 185—186°C.

Analysis:

| | |
|---|---|
| Calculated for $C_{11}H_{16}NOCl_3$: | C, 46.42; H, 5.67; H, 4.92 |
| Found: | C, 46.45; H, 5.70; N, 4.92 |

Preparation 36

N-[-2-(3,5-Dichlorophenoxy)ethyl]-1,1-dimethylethanamine maleate

The title compound was prepared by the method of Preparation 34 from 2 - (3,5 - dichlorophenoxy) - 1 - bromoethane, and t-butylamine with stirring for 13 days. The free base was obtained as a solid (69%).

The maleate salt melted at 202—203°C.

Analysis:

| | |
|---|---|
| Calculated for $C_{16}H_{21}NO_5$: | C, 50.81; H, 5.60; N, 3.70 |
| Found: | C, 50.75; H, 5.64; N, 3.68 |

Preparation 37

N-[2-(3,5-Dichlorophenoxy)ethyl]-2-methylpropanamine hydrochloride

The title compound was prepared by the method of Preparation 34 from 2 - (3,5 - dichlorophenoxy) - 1 - bromoethane and isobutylamine with stirring for 72 hrs and hydrogen chloride. The free base was obtained as an oil. The hydrochloride salt melted at 190—191.5°C.

Analysis:

| | |
|---|---|
| Calculated for $C_{12}H_{18}NOCl_3$: | C, 48.26; H, 6.08; N, 4.69 |
| Found: | C, 48.38; H, 6.12; N, 4.74 |

Preparation 38

N-(1-Methylethyl)-2-[4-(methylthio)phenoxy]ethanamine

4-(Methylthio)phenol was reacted with a large excess of 1,2-dibromethane in the presence of an alkali metal base such as sodium or potassium hydroxide. The product of that reaction, 2-bromoethyl-4-methylthiophenyl ether, was then reacted with isopropylamine as in Preparation 1 to give the title compound.

Preparation 39

N-(1-Methylethyl)-2-[4-(methylsulphinyl)phenoxy]ethanamine

N-(1-Methylethyl)-2-[4-(methylthio)phenoxy]ethanamine (from Preparation 38) was converted to the title compound by reacting with sodium perborate hydrate in 2M sulphuric acid at room temperature. The product was isolated by making basic with sodium hydroxide, extracting with chloroform and evaporating the chloroform layer.

Preparation 40

N-(1-Methylethyl)-2-[4-(methylsulphonyl)phenoxy]ethanamine

N-(1-Methylethyl)-2-[4-(methylthio)phenoxy]ethanamine (from Preparation 38) was converted to the title compound by reacting with sodium perborate hydrate in 2M sulphuric acid at reflux. The product was isolated by making basic with sodium hydroxide, extracting with chloroform and evaporating the chloroform layer.

Preparation 41

N-(1-Methylethyl)-2-(4-nitrophenoxy)ethanamine hydrochloride

p-Nitrophenol was reacted with 1,2-dibromoethane in the presence of an alkali metal base such as sodium or potassium hydroxide. The product of that reaction, 2-bromoethyl-4-nitrophenyl ether, was then reacted with isopropylamine as in Preparation 1 to give the title compound, m.p. 204—205°C.

Preparation 42

2-(4-Acetylphenoxy)-N-(1-methylethyl)ethanamine

4-Hydroxyphenylmethyl ketone was reacted with 1,2-dibromoethane in the presence of a base. The product of that reaction, 2-bromoethyl-4-acetylphenyl ether, was then reacted with isopropylamine as in Preparation 1 to give the title compound.

The following examples 1—45 and 47—65 serve to illustrate the preparation of compounds in accordance with the present invention for use in treating arrhythmias. The scope of the invention is, however, not limited thereto. Structures are given in Table 1 and analytical data in Table 2. Example 46 is representative of novel intermediates useful in the preparation of active compounds in accordance with the present invention.

Examples 1A to 1G

N-[2-(3,4-Dichlorophenoxy)ethyl]-N'-[2-(dimethylamino)ethyl]-N-(1-methylethyl)urea

To a solution of 12.25 g (0.05 mole) of 2 - (3,4 - di - chlorophenoxy) - N - (1 - methylethyl)ethanamine (oil obtained in Preparation 1) and 1.07 g (0.05 mole) of 1,8-bis(dimethylamino)naphthylene (Proton Sponge) in 200 ml of methylene chloride was added dropwise over a 30 minute period, a solution prepared by adding 9.3 g (0.095 mole) of phosgene in 50 ml of benzene to 100 ml of methylene chloride. The reaction solution was stirred for 3 hr at room temperature, after which it was extracted with 1N sulphuric acid. The solvent layer was dried over sodium sulphate-sodium bicarbonate. Solvent was evaporated off to give an oil. The oil was dissolved in 250 ml of tetrahydrofuran, and 8.8 g (0.1 mole) of N,N-dimethyl-1,2-ethanediamine in 150 ml of tetrahydrofuran was added dropwise over a 15 min period. The reaction mixture was stirred overnight at room temperature, then stripped to dryness and the residue partitioned between water and methylene chloride. Evaporation of solvent gave a light brown oil which slowly crystallized. Recrystallization from isopropyl ether gave 9.02 g (50%) of white crystalline product, m.p. 53—56°C. Various salts, such as maleate (1B), oxalate (1C), citrate (1D), fumarate (1E), tartrate (1F) and hydrochloride

(1G), were prepared therefrom by reaction with maleic, oxalic, citric, fumaric, and tartaric acid and hydrogen chloride, respectively, using an appropriate solvent.

Example 2
N-[2-(3,4-Dichlorophenoxy)ethyl]-N'-[2-(dimethylamino)ethyl]-N-(1-methylethyl)urea, monohydrochloride, monohydrate

This salt was prepared essentially as in Example 1, except the last oil was reacted with ethereal hydrogen chloride followed by recrystallization from methanol-diethyl ether, m.p. 108—111°C.

Example 3
N-[2-(3,5-Dichlorophenoxy)ethyl]-N'-[2-(dimethylamino)ethyl]-N-(1-methylethyl)urea, maleate [1:1]

To a solution of phosgene in benzene (23.0 ml of 1.9 M (0.0436 mole)phosgene) and 4.28 g (0.02 mole) of Proton Sponge in 100 ml of methylene chloride was added dropwise with stirring a solution of 4.92 g (0.02 mole) of 2 - (3,5 - dichlorophenoxy - N - (1 - methylethyl)ethanamine (oil in Preparation 2) in 100 ml of methylene chloride. After the solution has been stirred for 3/4 hr at room temperature, it was extracted with several portions of 1N sulphuric acid. The methylene chloride layer was dried over sodium sulphate and sodium bicarbonate and then filtered. The solvent was removed under reduced pressure to give an oil. The oil was dissolved in 300 ml of tetrahydrofuran. To the tetrahydrofuran solution was added dropwise 3.52 g (0.04 mole) of N,N-dimethyl-1,2-ethanediamine in 50 ml of tetrahydrofuran with stirring over a 5 minute period. The reaction mixture was stirred over the week-end at room temperature and filtered. Tetrahydrofuran was removed and the residue dissolved in chloroform. The chloroform solution was extracted with water, dried, filtered and evaporated to give an oil, the free base of the title compound. The oil was dissolved in methanol and reacted with maleic acid. Recrystallization from methanol-diethyl ether gave 4.70 g (49.1%) of white crystalline product, m.p. 103—105°C.

Example 3A
N-[2-(3,5-Dichlorophenoxy)ethyl]-N'-[2-(dimethylamino)ethyl]-N-(1-methylethyl)urea, maleate [1:1]

The title compound was prepared, utilizing the latter part of the procedure of Example 3, reacting:
N-[2-(3,5-dichlorophenoxy)ethyl]-N-(1-methylethyl) carbamic chloride (obtained in Example 46), N,N-dimethyl-1,2-ethanediamine, and maleic acid; m.p. 103—105°C.

Example 4
N-[2-(3,4-Dichlorophenoxy)ethyl]-N'-[3-(dimethylamino)propyl]-N-(1-methylethyl)urea oxalate [1:1]

The title compound was prepared by Method A by reacting in sequence:
phosgene, Proton Sponge,
2-(3,4-dichlorophenoxy)-N-(1-methylethyl)ethanamine (oil in Preparation 1) and N,N-dimethyl-1,3-propanediamine to give an oil (the free base of the title compound) which was then reacted with oxalic acid to give the title compound (39%); m.p. 106—110°C.

Example 5
N-[3-(3,5-Dichlorophenoxy)propyl]-N'-[2-(dimethylamino)ethyl]-N-(1-methylethyl)urea, oxalate [1:1]

The title compound was prepared by Method A by reacting in sequence:
phosgene, Proton Sponge,
3-(3,5-dichlorophenoxy)-N-(1-methylethyl)propanamine (oil in Preparation 3) and N,N-dimethyl-1,2-ethanediamine to give an oil (the free base of the title compound) which was then reacted with oxalic acid to give the title compound (49%); m.p. 139—141°C.

Example 6
N-[2-(Dimethylamino)ethyl]-N'-(1-methylethyl)-N'-(2-phenoxyethyl)urea; fumarate [1:1]

The title compound was prepared by Method A by reacting in sequence:
phosgene, proton Sponge,
N-(1-xethylethyl)-2-phenoxyethanamine (oil in Preparation 4) and N,N-dimethyl-1,2-ethanediamine to give an oil, (the free base of the title compound) which was then reacted with fumaric acid to give the title compound (27%); m.p. 90—93.5°C.

Example 7
N-[3-(3,5-Dichlorophenoxy)propyl]-N'-[3-(dimethylamino)propyl]-N-(1-methylethyl)urea citrate [1:1]

The title compound was prepared by Method A, reacting in sequence:
phosgene, Proton Sponge,
3-(3,5-dichlorophenoxy)-N-(1-methylethyl)propanamine (oil in Preparation 3) and N,N-dimethyl-1,3-propanediamine to give an oil (the free base of the title compound) which was then reacted with citric acid to give the title compound (69%); m.p. 109—112°C.

Example 8
N-[2-(3,5-Dichlorophenoxy)ethyl]-N'-[dimethylamino)propyl]-N-(1-methylethyl)urea citrate [1:1]

The title compound was prepared by Method A by reacting in sequence:
phosgene, Proton Sponge,
2-(3,5-dichlorophenoxy)-N-(1-methylethyl)ethanamine (oil in Preparation 2) and N,N-dimethyl-1,3-propandiamine to give an oil (the free base of the title compound) which was then reacted with citric acid to give the title compound (85%); m.p. 126—128°C.

Example 9
N-[2-(3,5-Dichlorophenoxy)ethyl]-N-(1-methylethyl)-N′-[2-[bis-(1-methylethyl)amino]ethyl]urea hydrochloride [1:1]

The title compound was prepared by Method A by reacting in sequence:
phosgene, Proton Sponge,
2-(3,5-dichlorophenoxy)-N-(1-methylethyl)ethanamine (oil in Preparation 2) and N,N - bis - (1 - methylethyl) - 1,2 - ethanediamine (from Preparation 6) and triethylamine to give an oil (the free base of the title compound) which was then reacted with ethereal hydrogen chloride to give the title compound (41%); m.p. 187—189°C.

Example 10
N′-[2-(Dimethylamino)ethyl]-N-[(3,4-dimethoxyphenoxy)ethyl]-N-(methylethyl)urea, fumarate [1:1.5]

The title compound was prepared by Method A by reacting in sequence:
phosgene, Proton Sponge,
N-[2-(3,4-dimethoxyphenoxy)ethyl]-1-methylethanamine (oil in Preparation 7) and N,N - dimethyl - 1,2 - ethanediamine to give an oil (the free base of the title compound) which was then reacted with fumaric acid to give the title compound (39.6%); m.p. 128—130°C.

Example 11
N-[2-(2,4-Dichlorophenoxy)ethyl]-N′-[2-(dimethylamino)ethyl]-N-(1-methylethyl)urea, maleate [1:1]

The title compound was prepared by Method A by reacting in sequence:
phosgene, Proton Sponge,
N-[2-(2,4-dichlorophenoxy)ethyl]-1-methylethanamine (solid in Prepartion 8 prior to conversion to maleate salt) and N,N-dimethyl-1,2-ethanediamine to give an oil (the free base of the title compound which was then reacted with maleic acid to give the title compound (40.5%); m.p. 131—132.5°C.

Example 12
N-[2-(3,5-Dichlorophenoxy)ethyl]-N′-[(dimethylamino)ethyl]-N-phenylurea, monohydrochloride

The title compound was prepared by Method A by reacting in sequence:
phosgene, Proton Sponge,
N-[2-(3,5-dichlorophenoxy)ethyl]benzenamine (obtained by neutralizing the hydrochloride salt of Preparation 9) N,N-dimethyl-1,2-ethanediamine to give an oil (the free base of the title compound) which was then reacted with ethereal hydrogen chloride to give the title compound (40%); m.p. 159—162.5°C.

Example 13
N′-[2-(Dimethylamino)ethyl]-N-(1-methylethyl)-N-[2-(1-naphthyleneoxy)ethyl]urea maleate [1:1]

The title compound was prepared by Method A by reacting in sequence:
phosgene, Proton Sponge,
1-methyl-N-[2-(1-naphthyleneoxy)ethyl]ethanamine (oil in Preparation 11) and N,N-dimethyl-1,2-ethanediamine to give an oil (the free base of the title compound) which was then reacted with maleic acid to give the title compound (75.9%); m.p. 131—133°C.

Example 14
N-[2-(3-Chlorophenoxy)ethyl]-N′-[dimethylamino)ethyl]-N-(1-methylethyl)urea maleate [1:1]

The title compound was prepared by Method A by reacting in sequence:
phosgene, Proton Sponge,
N-[2-(3-chlorophenoxy)ethyl]-1-methylethanamine (oil in Preparation 12) and N,N-dimethyl-1,2-ethanediamine to give an oil (the free base of the title compound) which was then reacted with maleic acid to give the title compound (56.3%); m.p. 70.5—73.0°C.

Example 15
N-[2-(2,6-Dichlorophenoxy)ethyl]-N′-[2-(dimethylamino)ethyl]-N-(1-methylethyl)urea

The title compound was prepared by Method A by reacting in sequence:
phosgene, Proton Sponge,
N-[2-(2,6-dichlorophenoxy)ethyl]-1-methylethanamine (oil in Preparation 13) and N,N-dimethyl-1,2-ethanediamine to give an oil (60.6%).

Example 16

N-[2-(3,5-Dichlorophenoxy)ethyl]-N'-[2-(dimethylamino)ethyl]-N'-methyl-N-(1-methylethyl)urea citrate [1:1]

The title compound was prepared by Method A by reacting in sequence:
phosgene, Proton Sponge,
2-(3,5-dichlorophenoxy)-N-(1-methylethyl)ethanamine (oil in Preparation 2) and N,N,N'-trimethyl-1,2-ethanediamine to give an oil (the free base of the title compound) which was then reacted with citric acid to give the title compound (71.9%); m.p. 122—125°C.

Example 17

N-[2-(3,5-Dichlorophenoxy)ethyl]-N'-[2-(dimethylamino)ethyl]urea, fumarate [1:1]

The title compound was prepared by Method A by reacting in sequence:
phosgene, proton Sponge,
2-(3,5-dichlorophenoxy)ethanamine (oil in Preparation 15) and N,N-dimethyl-1,2-ethanediamine to give an oil (the free base of the title compound) which was then reacted with fumaric acid to give the title compound (46.4%); m.p. 87—91°C.

Example 18

N'-[2-(Dimethylamino)ethyl]-N-[2-(3,5-dimethylphenoxy)ethyl]-N-(1-methylethyl)urea fumarate [1:1]

Method B demonstration

To a stirred solution of 8.04 g (0.0496 mole) 1,1'-carbonyldiimidazole in 200 ml of tetrahydrofuran was added dropwise, a solution of 4.3 g (0.049 mole) of N,N-dimethyl-1,2-ethanediamine in 50 ml of tetrahydrofuran. The solution was stirred for 1 hr at room temperature. A solution of 9.76 g (0.047 mole) of N - isopropyl - N - [2 - (3,5 - dimethylphenoxy)ethyl]amine (oil in Preparation 16) in 100 ml of tetrahydrofuran was added and the solution stirred at room temperature overnight. The reaction mixture was refluxed for 5 hr, quenched in water and extracted with methylene chloride. The organic layer was dried over magnesium sulphate. Removal of solvent under reduced pressure gave the free base as an oil. The free base was dissolved in methanol and fumaric acid added. The fumarate salt was separated and recrystallized from methanol-diethyl ether to give 15.44 g (71.4%) of white crystalline solid; m.p. 119—120°C.

Example 19

N-[2-(3,5-Dimethoxyphenoxy)ethyl]-N'-[2-(dimethylamino)ethyl]-N-(1-methylethyl)urea, fumarate [1:1]

The title compound was prepared by Method B by reacting in sequence:
N,N-dimethyl-1,2-ethanediamine,
1,1'-carbonyldiimidazole and
N-(1-methylethyl)-2-(3,5-dimethoxyphenoxy)ethanamine (oil in Preparation 17) to give an oil (the free base of the title compound) which was then reacted with fumaric acid to give the title compound (49%); m.p. 120—122°C.

Example 20

N'-[2-(Dimethylamino)ethyl]-N-[2-(3,4-dimethylphenoxy)ethyl]-N-(1-methylethyl)urea, fumarate [1:1].

The title compound was prepared by Method B by reacting in sequence:
N,N-dimethyl-1,2-ethanediamine,
1,1'-carbonyldiimidazole and
N-[2-(3,4-dimethylphenoxy)ethyl]-1-methylethanamine (oil in Preparation 18) to give an oil (the free base of the title compound) which was then reacted with fumaric acid to give the title compound (75.1%); m.p. 94—96°C.

Example 21

N-[2-(4-Chlorophenoxy)ethyl]-N'-[2-(dimethylamino)ethyl]-N-(1-methylethyl)urea maleate [1:1]

The title compound was prepared by Method B by reacting in sequence:
N,N-dimethyl-1,2-ethanediamine,
1,1'-carbonyldiimidazole and
N-[2-(4-chlorophenoxy)ethyl]-1-methylethanamine (oil in Preparation 19) to give an oil (the free base of the title compound) which was then reacted with maleic acid to give the title compound (60.2%); m.p. 97—100°C.

Example 22

N-[2-(2,5-Dichlorophenoxy)ethyl]-N'-[2-(dimethylamino)ethyl]-N-(1-methylethyl)urea, maleate [1:1]

The title compound was prepared by Method B, reacting in sequence:
N,N-dimethyl-1,2-ethanediamine,
1,1'-carbonyldiimidazole, and
N-[2-(2,5-dichlorophenoxy)ethyl]-1-methylethanamine (oil in Preparation 20), to give an oil (the free base of the title compound) which was then reacted with maleic acid to give the title compound (73.1%); m.p. 133.5—135°C.

Example 23
N-[2-(3,5-Dichlorophenoxy)ethyl]-N-(1-methylethyl)-N'-[2-(4-morpholinyl)ethyl]urea fumarate [1:1]

The title compound was prepared by Method B, reacting in sequence:
N-(2-aminoethyl)morpholine,
1,1'-carbonyldiimidazole, and
2-(3,5-dichlorophenoxy)-N-(1-methylethyl)ethanamine (oil in Preparation 2), to give an oil (the free base of the title compound) which was then reacted with fumaric acid to give the title compound (49%); m.p. 105—107°C.

Example 24
N'-[2-(Dimethylamino)ethyl]-N-(1-methylethyl)-N-[2-(3-trifluoromethylphenoxy)ethyl]urea fumarate [2:1]

The title compound was prepared by Method B, reacting in sequence:
N,N-dimethyl-1,2-ethanediamine,
1,1'-carbonyldiimidazole, and
1-methyl-N-[2-(3-trifluoromethylphenoxy)ethyl]ethanamine (oil in Preparation 21), to give a solid (the free base of the title compound) which was then reacted with fumaric acid to give the title compound (49.8%); m.p. 114—115°C.

Example 25
N-[2-(3,5-Dichlorophenoxy)ethyl]-N'-[2-(dimethylamino)ethyl]-N-methyl urea maleate [1:1]

The title compound was prepared by Method B, reacting in sequence:
N,N-dimethyl-1,2-ethanediamine,
1,1'-carbonyldiimidazole, and
2-(3,5-dichlorophenoxy)-N-methylethanamine (oil in Preparation 22) to give an oil (the free base of the title compound) was then reacted with maleic acid to give the title compound (48.7%); m.p. 105—107°C.

Example 26
N'-[2-(Dimethylamino)ethyl]-N-[2-(3-fluorophenoxy)ethyl]-N-(1-methylethyl)urea fumarate [1:1]

The title compound was prepared by Method B, reacting in sequence:
N,N-dimethyl-1,2-ethanediamine,
1,1'-carbonyldiimidazole, and
N-[2-(3-fluorophenoxy)ethyl]-1-methylethanamine (oil in Preparation 23), to give an oil (the free base of the title compound) which was then reacted with fumaric acid to give the title compound (66.4%); m.p. 106—108°C.

Example 27
N-[2-(2,3-Dichlorophenoxy)ethyl]-N'-[2-(dimethylamino)ethyl]-N-(1-methylethyl)urea maleate [1:1]

The title compound was prepared by Method B, reacting in sequence:
N,N-dimethyl-1,2-ethanediamine,
1,1'-carbonyldiimidazole, and
N-[2-(2,3-dichlorophenoxy)ethyl]-1-methylethanamine (oil in Preparation 24), to give an oil (the free base of the title compound) which was then reacted with maleic acid to give the title compound (69.7%); m.p. 128.5—130°C.

Example 28
N-[2-(2-Chlorophenoxy)ethyl]-N'-[2-(dimethylamino)ethyl]-N-(1-methylethyl)urea maleate [1:1]

The title compound was prepared by Method B, reacting in sequence:
N,N-dimethyl-1,2-ethanediamine,
1,1'-carbonyldiimidazole, and
N-[2-(2-chlorophenoxy)ethyl]-1-methylethanamine (oil in Preparation 25), to give an oil (the free base of the title compound) which was then reacted with maleic acid to give the title compound (73%); m.p. 107.5—109°C.

Example 29
N'-[2-(Dimethylamino)ethyl]-N-(1-methylethyl)-N-[2-(2-naphthalenyloxy)ethyl]urea hydrochloride

The title compound was prepared by Method B, reacting in sequence:
N,N-dimethyl-1,2-ethanediamine,
1,1'-carbonyldiimidazole, and
1-methyl-N-[2-(2-naphthalenyloxy)ethyl]ethanamine (oil of Preparation 27) to give an oil (the free base of the title compound) which was then reacted with ethereal hydrogen chloride to give the title compound (29%); m.p. 169—171°C.

Example 30
N'-[2-(Dimethylamino)ethyl]-N-[2-[(2,3-dihydro-1H-inden-5-yl)oxy]ethyl]-N-(1-methylethyl)urea, maleate [1:1]

The title compound was prepared by Method B, reacting in sequence:

N,N-dimethyl-1,2-ethanediamine,

1,1′-carbonyldiimidazole, and

N-[2-[(2,3-Dihydro-1H-inden-5-yl)oxy]ethyl]methylethanamine (second oil in Preparation 28), to give an oil (the free base of the title compound) which was then reacted with maleic acid to give the title compound (40.9%); m.p. 125.5—127.0°C.

Example 31

N′-[2-(dimethylamino)ethyl]-N-[2-[(2,3-dihydro-1H-inden-4-yl)oxy]ethyl]-N-(1-methylethyl)urea maleate [1:1]

The title compound was prepared by Method B, reacting in sequence:

N,N-dimethyl-1,2-ethanediamine,

1,1′-carbonyldiimidazole, and

N-[2-[(2,3-dihydro-1H-inden-4-yl)oxy]ethyl]-1-methylethanamine (oil obtained in Preparation 29), to give an oil (the free base of the title compound) which was then reacted with maleic acid to give the title compound (76.8%); m.p. 136.5—138.0°C.

Example 32

N-[2-(3,5-Dichlorophenoxy)ethyl]-N′-methyl-N′-[2-(methylamino)ethyl]-N-(1-methylethyl)urea maleate [1:1]

The title compound was prepared by Method A, reacting in sequence: phosgene, Proton Sponge, 2-(3,5-dichlorophenoxy)-N-(1-methylethyl)ethanamine (oil in Preparation 2) and symmetrical dimethylethylenediamine to give an oil (the free base of the title compound) which was then reacted with maleic acid to give the title compound (50.1%); m.p. 119.5—122°C.

Example 33

N′-[2-[(2,3-Dihydro-1H-inden-4-yl)oxy]ethyl]-N-[2-(methylamino)ethyl]-N-(1-methyl)urea fumarate [1:1]

Method C demonstration

A solution of 3.24 g (0.02 mole) of 1,1′-carbonyldiimidazole and 3.54 g (0.02 mole) of 2 - [(2,3 - dihydro - 1H - inden - 4 - yl)oxy]ethanamine (free base as oil obtained in Preparation 31) in 100 ml tetrahydrofuran was stirred at room temperature for 5 hr. Symdimethylethylenediamine, 3.06 g (0.03 mole) was added and the solution was heated for 24 hr at reflux. The tetrahydrofuran was removed in a rotary evaporator and the resultant oil partitioned between chloroform and water. The chloroform layer was evaporated to give an oil, the free base of the title compound which was then reacted with fumaric acid. Recrystallization from methanol-diethyl ether gave 4.60 g (56.1%) of white crystalline product after drying at 80°C. overnight *in vacuo*; m.p. 62.5—65°C.

Example 34

N-Butyl-N-[2-(3,5-dichlorophenoxy)ethyl]-N′-[2-(dimethylamino)ethyl]urea maleate [1:1]

The title compound was prepared by Method B, reacting in sequence:

N,N-dimethylaminoethyl amine,

1,1′-carbonyldiimidazole, and

N-[2-(3,5-dichlorophenoxy)ethyl]butamine (neutralized product of Preparation 34), to give an oil (the free base of the title compound) which was then reacted with maleic acid to give the title compound (71.1%); m.p. 128—129°C.

Example 35

N-[2-(3,5-Dichlorophenoxy)ethyl]-N′-[2-(dimethylamino)ethyl]-N-propylurea maleate [1:1]

The title compound was prepared by Method B, reacting in sequence:

N,N-dimethylaminoethyl amine,

1,1′-carbonyldiimidazole, and

N-[2-(3,5-dichlorophenoxy)ethyl]propanamine (from Preparation 35), to give an oil (the free base of the title compound) which was then reacted with maleic acid to give the title compound; m.p. 119—121°C.

Example 36

N-Cyclohexyl-N-[2-(3,5-dichlorophenoxy)ethyl]-N′-[2-(dimethylamine)ethyl]urea maleate [1:1]

The title compound was prepared by Method A from:

phosgene, Proton Sponge,

N-[2-(3,5-dichlorophenoxy)ethyl]cyclohexanamine, and

N-N-dimethyl-1,2-ethandiamine, to give an oil (the free base of the title compound) which was then reacted with fumaric acid to give the title compound (60.1%); m.p. 125.5—126.5°C.

Example 37

N-[2-(3,5-Dichlorophenoxy)ethyl]-N′-[2-(dimethylamino)ethyl]-N-(2-methylpropyl)urea maleate [1:1]

The title compound was prepared by Method B by reacting in sequence:

N,N-dimethylaminoethylamine,

1,1'-carbonyldiimidazole,

N-[2-(3,5-dichlorophenoxy)ethyl]-2-methyl-1-propanamine (oil in Preparation 37) to give an oil, (the free base of the title compound) which was then reacted with maleic acid to give the title compound (69.9%); m.p. 117.5—119°C.

Example 38

N-[2-(3,5-Dichlorophenoxy)ethyl]-N'-[2-(dimethylamino)ethyl]-N-(1,1-dimethylethyl)urea maleate

The title compound was prepared by Method B by reacting in sequence:

N,N-dimethylaminoethylamine,

1,1'-carbonyldiimidazole, and

N-[2-(3,5-dichlorophenoxy)ethyl]-1,1-dimethylethanamine (free base from Preparation 36) to give the free base of the title compound which was then reacted with maleic acid.

Example 39

N'-[2-(3,5-Dichlorophenoxy)ethyl]-N-[2-(dimethylamino)ethyl]-N-methylurea maleate [1:1]

The title compound was prepared by Method C, reacting in sequence:

2-(3,5-dichlorophenoxy)ethanamine (oil in Preparation 15)

1,1'-carbonyldiimidazole, and

N,N,N'-trimethylethylenediamine, to give an oil (the free base of the title compound) which was then reacted with maleic acid to give the title compound (94.4%); m.p. 86—89°C.

Example 40

N-[2-(3,5-Dichlorophenoxy)ethyl]-N-(1-methylethyl)-N'-(2-aminoethyl)urea maleate [1:1]

The title compound was prepared by Method A, reacting in sequence:

phosgene, Proton Sponge,

2-(3,5-dichlorophenoxy)-N-(1-methylethyl)ethanamine (oil in Preparation 2), and

ethylenediamine to give an oil (the free base of the title compound) which was then reacted with maleic acid to give the title compound (44.3%); m.p. 153—154°C.

Examples 43A and 43B

N-Methyl-N-(2-phenoxyethyl)-N'-[2-(1-pyrrolidinyl)ethyl]urea oxalate [1:1]

The title compound was prepared by Method A, reacting in sequence:

phosgene,

N-(1-methyl)-2-phenoxyethanamine (oil in Preparation 33) and

N-(2-aminoethyl)pyrrolidine, to give an oil (the free base of the title compound) which was then reacted with oxalic acid to give the title compund; m.p. 117.5—118.5°C.

Example 43B

The title compound was also prepared by Method B, reacting in sequence:

N-(2-aminoethyl)pyrrolidine,

1,1'-carbonyldiimidazole, and

N-(1-methyl)-2-phenoxyethanamine (oil in Preparation 33), to give an oil (the free base of the title compound) which was then reacted with oxalic acid to give the title compound; m.p. 117.5—118.5°C.

Example 44

N-[2-(3,5-Dichlorophenoxy)ethyl]-N'-[2-(diethylamino)ethyl]-N-(1-methylethyl)urea tartrate [1:1]

The title compound was prepared by Method B, reacting in sequence:

diethylaminoethylamine,

1,1'-carbonyldiimidazole, and

2-(3,5-dichlorophenoxy)-N-(1-methylethyl)ethanamine to give an oil (the free base of the title compound) which was then reacted with tartaric acid to give the title compound (55%); m.p. 119—120°C.

Example 45

N-[2-(3,5-Dichlorophenoxy)ethyl]-N'-[2-(dimethylamino)ethyl]-N-(1-methylethyl)urea hydrate [2:1]

A solution of N - [2 - (3,5 - dichlorophenoxy)ethyl] - N' - [2 - (dimethylamino)ethyl] - N - (1 - methylethyl)urea Maleate [1:1], from Example 3A, was made basic with 2M sodium hydroxide solution and extracted with methylene chloride. The methylene chloride layer was dried over magnesium sulphate and evaporated under vacuum to give an oil. Nuclear magnetic resonance measurements showed the product had 1/2 equivalent of water present.

Example 46

N-[2-(3,5-Dichlorophenoxy)ethyl]-N-(1-methylethyl)carbamic chloride.

This is an intermediate.

A solution of 4.53 g (0.018 mole) of 2 - (3,5 - dichlorophenoxy) - N - (1 - methylethyl)ethanamine (oil obtained in Preparation 1), 3.92 g (0.040 moles) phosgene in 21 ml solution of benzene and 3.92 g (0.018

mole) of 1,8-bis-(dimethylamino)naphthalene (Proton Sponge) in 200 ml of methylene chloride was stirred overnight at room temperature. The reaction mixture was extracted with 1N sulphuric acid and the methylene chloride layer thereafter dried over sodium sulphate-sodium carbonate. Evaporation of solvent gave an oil which, when triturated with hexane, gave 1.91 g (34.2%) g of solid. The solid formed a gum on exposure to air which prevented measurement of melting point by conventional means.

Analysis:

| | |
|---|---|
| Calculated for $C_{12}H_{14}NO_2Cl_3$: | C, 46.40; H, 4.54; N, 4.51 |
| Found: | C, 46.51; H, 4.50; N, 4.50 |

Example 47

N-[2-(3,5-Dichlorophenoxy)ethyl]-N'-[2-(dimethylamino)ethyl]-N-(1-methylethyl)thiourea, hemifumarate, hemihydrate

The title compound was prepared by Method A as follows: To a solution of 6.42 g (0.03 mole) of Proton Sponge (1,8-bis(dimethylamino)naphthalene) and 4.90 g (0.043 mole) of thiophosgene in 300 ml of methylene chloride was added dropwise a solution of 7.30 g (0.03 mole) of 2 - (3,5 - dichlorophenoxy) - N - (1 - methylethyl)ethanamine (oil in Preparation 2) in methylene chloride. The solution was stirred for 2 hours additionally at room temperature and then extracted several times with 1N aqueous sulphuric acid solution. The methylene chloride layer was dried over anhydrous potassium carbonate, filtered and rotary-evaporated to give a dark brown oil. The oil was dissolved in tetrahydrofuran, 5.28 g (0.06 mole) of unsym. N,N-dimethylethylenediamine added and the solution stirred overnight at room temperature. The reaction mixture was stripped to dryness and partitioned several times between chloroform and 10% aqueous sodium hydroxide. Removal of chloroform gave an oil, the free base of the title compound. The fumarate salt was prepared and recrystallized several times from methanol-diethyl ether. After drying overnight at 80°C, 3.14 g (23.5%) of a white crystalline solid was obtained, m.p. 153°C.

Example 48

N-[2-(Diethylamino)ethyl]-N'-(1-methylethyl)-N'-(2-phenoxyethyl)thiourea oxalate [1:1]

The title compound was prepared by Method A and the procedure of Example 47 by reacting in sequence:

thiophosgene, Proton Sponge,

N-(1-methylethyl)-2-phenoxyethanamine (free base oil in Preparation 4), and

N,N-diethylethylenediamine to give an oil, (the free base of the title compound) which was then reacted with oxalic acid to give the oxalate, (16.4%), m.p. 117—117.5°C.

Example 49

N'-[2-(Dimethylamino)ethyl]-N-(1-methylethyl)-N-[2-(1-naphthalenyloxy)ethyl]thiourea maleate [1:1]

The title compound was prepared by Method A and the procedure of Example 47 by reacting in sequence:

thiophosgene, Proton Sponge,

1-methyl-N-[2-(1-naphthenyloxy)ethyl]ethanamine (oil in Preparation 11), and

N,N-dimethylethylenediamine to give an oil (the free base of the title compound) which was then converted to the maleate salt (27.0%), m.p. 130—131°C.

Example 50

N'-[2-(Diethylamino)ethyl]-N-methyl-N-(2-phenoxyethyl)thiourea, citrate hemihydrate

The title compound was prepared by Method A and the procedure of Example 47 by reacting in sequence:

thiophosgene, Proton Sponge,

N-methyl-2-phenoxyethanamine (oil in Preparation 33) and

N,N-diethylethylenediamine to give an oil (the free base of the title compound) which was then converted to the citrate hemihydrate salt, (25%) a pink salt, m.p. 106—110°C.

Example 51

N-[2-(3,5-Dichlorophenoxy)ethyl]-N'-[2-(dimethylamino)ethyl]-N'-(1-methylethyl)urea maleate.

The title compound was prepared by Method A, reacting in sequence:

phosgene, Proton Sponge,

2-(3,5-dichlorophenoxy)ethanamine (oil in Preparation 15),

N'-isopropyl-N,N-dimethylethylenediamine and triethylamine to give an oil (the free base of the title compound) which was then reacted with maleic acid to give the title compound (24.4%), m.p. 115—116°C.

Example 52

N-[2-[(2,3-Dihydro-1H-inden-4-yl)oxy]ethyl]-N'-[2-(dimethylamino)ethyl]-N'-(1-methylethyl)urea maleate [1:1]

(Method D demonstration)

To a solution of 39.2 g (0.05 mole) of phosgene (40 ml of 12% phosgene in benzene) in methylene

chloride was added 6.5 g (0.05 mole) of N'-isopropyl-N,N-dimethylethylenediamine also in methylene chloride over a 30 minute period. The solution was stirred for one hour at room temperature. To the reaction mixture were added dropwise with stirring 8.85 g (0.05 mole) of N - [2 - [(2,3 - dihydro - 1H - inden - 4 - yl)oxy]ethyl]ethanamine (oil in Preparation 31) and 10.1 g (0.1 mole) of triethylamine over a 30 min period. The reaction mixture was stirred overnight at room temperature and thereafter extracted with aqueous 10% sodium hydroxide solution. The methylene chloride layer was then extracted with 1N sulphuric acid. The acid layer was made alkaline and extracted with chloroform. The chloroform layer was evaporated to give an oil, the free base of the title compound. The free base was converted to the maleate salt which was recrystallized from methanol-diethyl ether to give 2.41 g (10.7%) of white crystalline product, m.p. 118—120°C.

Example 53
N-[2-(3,4-Dichlorophenoxy)ethyl]-N'-[2-(dimethylamino)ethyl]-N-(1-methylethyl)thiourea

When in the procedure of Example 3, equal molar amounts of thiophosgene were substituted for phosgene, the title compound was obtained.

Examples 54A to 54O

When in the procedure of Example 3, equal molar substitutions of thiophosgene for phosgene and the following for 2 - (3,4 - dichlorophenoxy) - N - (1 - methylethyl)ethanamine were made:

3-(3,5-dichlorophenoxy)-N-(1-methylethyl)propanamine (oil in Preparation 3),

N-[2-(3,4-dimethoxyphenoxy)ethyl]-1-methylethanamine (oil in Preparation 7),

N-[2-(2,4-dichlorophenoxy)ethyl]-1-methylethanamine (solid in Preparation 8 prior to conversion to maleate salt),

N-[2-(3,5-dichlorophenoxy)ethyl]benzeneamine (neutralized Preparation 9),

N-[2-(3-chlorophenoxy)ethyl]-1-methylethanamine (oil in Preparation 12),

N-[2-(2,6-dichlorophenoxy)ethyl]-1-methylethanamine (oil in Preparation 13),

2-(3,5-dichlorophenoxy)ethanamine (oil in Preparation 15),

N-[2-(3,5-dichlorophenoxy)ethyl]cyclohexanamine (oil in Preparation 32),

there were obtained:

N-[3-(3,5-dichlorophenoxy)propyl]-N'-[2-(dimethylamino)ethyl]-N-(1-methylethyl)thiourea and the oxalate salt prepared therefrom (Examples 54A and B),

N'-[2-(dimethylamino)ethyl]-N-(3,4-dimethoxyphenoxy)ethyl]-N-(methylethyl)thiourea and the fumarate salt thereof (Examples 54C and D),

N-[2-(2,4-dichlorophenoxy)ethyl]-N'-[2-(dimethylamino)ethyl]-N-(1-methylethyl)thiourea and the maleate salt thereof (Examples 54E and F),

N-[2-(3,5-dichlorophenoxy)ethyl]-N'-[2-(dimethylamino)ethyl]-N-phenylthiourea and the hydrochloride salt thereof (Examples 54G and H),

N-[2-(3-chlorophenoxy)ethyl]-N'-[2-(dimethylamino)ethyl]-N-(1-methylethyl)thiourea and the maleate salt thereof (Examples 54I and J),

N-[2-(2,6-dichlorophenoxy)ethyl]-N'-[2-(dimethylamino)ethyl]-N-(1-methylethyl)thiourea (Example 54K)

N-[2-(3,5-dichlorophenoxy)ethyl]-N'-[2-(dimethylamino)ethyl]thiourea and the fumarate salt thereof (Examples 54L and M),

N-cyclohexyl-N-[2-(3,5-dichlorophenoxy)ethyl]-N'-[2-(dimethylamino)ethyl]thiourea and the maleate salt thereof (Examples 54N and O).

Examples 55A to 55F

When in the procedure of Example 3, equal molar substitutions of thiophosgene for phosgene, N,N-dimethyl-1,3-propanamine for N,N-dimethyl-1,2-ethanediamine and the following for 2 - (3,4 - dichlorophenoxy) - N - (1 - methylethyl) ethanamine were made:

2-(3,4-dichlorophenoxy)-N-(1-methylethyl)ethanamine (oil in Preparation 1),

3-(3,5-dichlorophenoxy)-N-(1-methylethyl)propanamine (oil in Preparation 3),

2-(3,5-dichlorophenoxy)-N-(1-methylethyl)ethanamine (oil in Preparation 2),

there were obtained:

N-[2-(3,4-dichlorophenoxy)ethyl]-N'-[3-(dimethylamino)propyl]-N-(1-methylethyl)thiourea and the oxalate salt thereof, (Examples 55A and B),

N-[3-(3,5-dichlorophenoxy)propyl]-N'-[3-(dimethylamino)propyl]-N-(1-methylethyl)thiourea and the citrate salt, (Examples 55C and D),

N-[2-(3,5-dichlorophenoxy)ethyl]-N'-[3-(dimethylamino)propyl]-N-(1-methylethyl)thiourea and the citrate salt thereof, (Examples 55E and F).

Example 56
N-[2-(3,5-Dichlorophenoxy)ethyl]-N'-[2-(dimethylamino)ethyl]-N'-methyl-N-(1-methylethyl)thiourea citrate

The title compound was prepared by Method A by reacting in sequence:

thiophosgene, Proton Sponge,

2-(3,5-dichlorophenoxy)-N-(1-methylethyl)ethanamine (oil in Preparation 2), and

N,N,N'-trimethyl-1,2-ethanediamine to give the free base which was then reacted with citric acid to give the title compound.

Example 57

N-[2-(3,5-Dichlorophenoxy)ethyl]-N'-methyl-N'-[2-(methylamino)ethyl]-N-(1-methylethyl)thoiurea maleate

The title compound was prepared by Method A by reacting in sequence:

thiophosgene, Proton Sponge,

2-(3,5-dichlorophenoxy)-N-(1-methylethyl)ethanamine (oil in Preparation 2) and symmetrical-dimethylethyleneamine to give the free base which was then reacted with maleic acid to give the title compound.

Example 58

N-[2-(3,5-Dichlorophenoxy)ethyl]-N-(1-methylethyl)-N'-(2-aminoethyl)thiourea

The title compound was prepared by Method A, reacting in sequence:

thiophosgene, Proton Sponge,

2-(3,5-dichlorophenoxy)-N-(1-methylethyl)ethanamine (oil in Preparation 2) and ethylenediamine.

Example 59

N-Methyl-N-(2-phenoxyethyl)-N'-[2-(1-pyrrolidinyl)ethyl]thiourea

The title compound was prepared by Method A, reacting in sequence:

thiophosgene, Proton Sponge,

N-(1-methyl)-2-phenoxyethanamine (oil in Preparation 33) and, N-(2-aminoethyl)pyrrolidine.

Example 60

N'-[2-(Dimethylamino)ethyl]-N-(1-methylethyl)-N-[2-[4-(methylthio)phenoxy]ethyl]urea maleate

The title compound was prepared by Method B, reacting in sequence:

N,N-dimethyl-1,2-ethanediamine,

1,1'-carbonyldiimidazole, and

N-(1-methylethyl)-2-(4-methylthiophenoxy)ethanamine (from Preparation 38) to give the free base of the title compound which was then reacted with maleic acid.

Example 61

N'-[2-(Diethylamino)ethyl]-N-(1-methylethyl)-N-[2-[4-(methylsulphinyl)phenoxy]ethyl]urea maleate

The title compound was prepared by Method B, reacting in sequence:

N,N-diethyl-1,2-ethanediamine,

1,1'-carbonyldiimidazole, and

N-(1-methylethyl)-2-(4-methylsulphinylphenoxy) ethanamine (from Preparation 39) to give the title compound which was then reacted with maleic acid.

Example 62

N'-[2-(Diethylamino)ethyl]-N-(1-methylethyl)-N-[2-[4-(methylsulphonyl)phenoxy]ethyl]urea maleate

The title compound was prepared by Method B, reacting in sequence:

N,N-diethyl-1,2-ethanediamine,

1,1-carbonyldiimidazole, and

N-(1-methylethyl)-2-(4-methylsulphonylphenoxy) ethanamine (from Preparation 40) to give the title compound which is then reacted with maleic acid.

Example 63

N'-[2-(Dimethylamino)ethyl]-N-(1-methylethyl)-N-[2-(4-nitrophenoxy)ethyl]urea maleate

The title compound was prepared by Method B, reacting in sequence:

N,N-dimethyl-1,2-ethanediamine,

1,1'-carbonyldiimidazole, and

N-(1-methylethyl)-2-(4-nitrophenoxy)ethanamine (from Preparation 41) to give the free base of the title compound which was then reacted with maleic acid.

Example 64

N'-[2-(Dimethylamino)ethyl]-N-(1-methylethyl)-N-[2-(4-aminophenoxy)ethyl]urea

N'-[2-(dimethylamino)ethyl]-N-(1-methylethyl)-N-[2-(4-nitrophenoxy)ethyl]urea (the free base in Example 62) was hydrogenated over palladium-on-charcoal catalyst to give the title compound.

Example 65

N-[2-(4-Acetylphenoxy)ethyl]-N'-[2-(dimethylamino)ethyl]-n-(1-methylethyl)urea, maleate

The title compound was prepared by Method B, reacting in sequence:

N,N-dimethyl-1,2-ethanediamine,
1,1'-carbonyldiimidazole, and
2-(4-acetylphenoxy)-N-(1-methylethyl)ethanamine (from Preparation 42) to give the free base of the title compound which was then reacted with maleic acid.

TABLE 1A

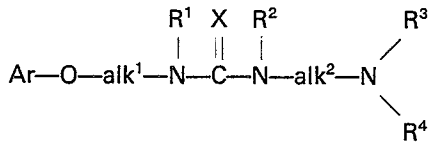

| Ex. No. | Ar | $alk^1$ | $R^1$ | X | $R^2$ |
|---|---|---|---|---|---|
| 1 | $3,4-Cl_2-C_6H_3-$ | $-(CH_2)_2-$ | $-CH(CH_3)_2$ | O | H |
| 2 | $3,4-Cl_2-C_6H_3-$ | $-(CH_2)_2-$ | $-CH(CH_3)_2$ | O | H |
| 3 | $3,5-Cl_2-C_6H_3-$ | $-(CH_2)_2-$ | $-CH(CH_3)_2$ | O | H |
| 4 | $3,4-Cl_2-C_6H_3-$ | $-(CH_2)_2-$ | $-CH(CH_3)_2$ | O | H |
| 5 | $3,5-Cl_2-C_6H_3-$ | $-(CH_2)_3-$ | $-CH(CH_3)_2$ | O | H |
| 6 | $-C_6H_5$ | $-(CH_2)_2-$ | $-CH(CH_3)_2$ | O | H |
| 7 | $3,5-Cl_2-C_6H_3-$ | $-(CH_2)_3-$ | $-CH(CH_3)_2$ | O | H |
| 8 | $3,5-Cl_2-C_6H_3-$ | $-(CH_2)_2-$ | $-CH(CH_3)_2$ | O | H |
| 9 | $3,5-Cl_2-C_6H_3-$ | $-(CH_2)_2-$ | $-CH(CH_3)_2$ | O | H |
| 10 | $3,4-(OCH_3)_2C_6H_3-$ | $-(CH_2)_2-$ | $-CH(CH_3)_2$ | O | H |
| 11 | $2,4-Cl_2-C_6H_3-$ | $-(CH_2)_2-$ | $-CH(CH_3)_2$ | O | H |
| 12 | $3,5-Cl_2-C_6H_3-$ | $-(CH_2)_2-$ | $-C_6H_5$ | O | H |
| 13 | $1-C_{10}H_7-$ | $-(CH_2)_2-$ | $-CH(CH_3)_2$ | O | H |
| 14 | $3-Cl-C_6H_4-$ | $-(CH_2)_2-$ | $-CH(CH_3)_2$ | O | H |
| 15 | $2,6-Cl_2-C_6H_3-$ | $-(CH_2)_2-$ | $-CH(CH_3)_2$ | O | H |
| 16 | $3,5-Cl_2-C_6H_3-$ | $-(CH_2)_2-$ | $-CH(CH_3)_2$ | O | $CH_3$ |
| 17 | $3,5-Cl_2-C_6H_3-$ | $-(CH_2)_2-$ | H | O | H |
| 18 | $3,5-(CH_3)_2-C_6H_3-$ | $-(CH_2)_2-$ | $-CH(CH_3)_2$ | O | H |
| 19 | $3,5-(OCH_3)_2-C_6H_3-$ | $-(CH_2)_2-$ | $-CH(CH_3)_2$ | O | H |
| 20 | $3,4-(CH_3)_2-C_6H_3-$ | $-(CH_2)_2-$ | $-CH(CH_3)_2$ | O | H |
| 21 | $4-Cl-C_6H_4-$ | $-(CH_2)_2-$ | $-CH(CH_3)_2$ | O | H |
| 22 | $2,5-Cl_2-C_6H_3-$ | $-(CH_2)_2-$ | $-CH(CH_3)_2$ | O | H |
| 23 | $3,5-Cl_2-C_6H_3-$ | $-(CH_2)_2-$ | $-CH(CH_3)_2$ | O | H |
| 24 | $3CF_3-C_6H_4-$ | $-(CH_2)_2-$ | $-CH(CH_3)_2$ | O | H |
| 25 | $3,5-Cl_2-C_6H_3-$ | $-(CH_2)_2-$ | $-CH_3$ | O | H |

TABLE 1A (continued)

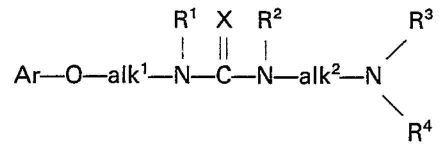

| Ex. No. | Ar | alk¹ | R¹ | X | R² |
|---|---|---|---|---|---|
| 26 | $3F—C_6H_4—$ | $—(CH_2)_2—$ | $—CH(CH_3)_2$ | O | H |
| 27 | $2,3—Cl_2—C_6H_3—$ | $—(CH_2)_2—$ | $—CH(CH_3)_2$ | O | H |
| 28 | $2\ CL—C_6H_4—$ | $—(CH_2)_2—$ | $—CH(CH_3)_2$ | O | H |
| 29 | $2—C_{10}H_7—$ | $—(CH_2)_2—$ | $—CH(CH_3)_2$ | O | H |
| 30 | $5—C_9H_9{}^{(a)}—$ | $—(CH_2)_2—$ | $—CH(CH_3)_2$ | O | H |
| 31 | $4—C_9H_9{}^{(b)}$ | $—(CH_2)_2—$ | $—CH(CH_3)_2$ | O | H |
| 32 | $3,5—Cl_2—C_6H_3—$ | $—(CH_2)_2—$ | $—CH(CH_3)_2$ | O | $CH_3$ |
| 33 | $4—C_9H_9{}^{(b)}$ | $—(CH_2)_2—$ | H | O | $CH_3$ |
| 34 | $3,5—Cl_2—C_6H_3—$ | $(—CH_2)_2—$ | $—C_4H_9$ | O | H |
| 35 | $3,5—Cl_2—C_6H_3—$ | $—(CH_2)_2—$ | $—C_3H_7$ | O | H |
| 36 | $3,5—Cl_2—C_6H_3—$ | $—(CH_2)_2—$ | $—C_6H_{11}$ | O | H |
| 37 | $3,5—Cl_2—C_6H_3—$ | $—(CH_2)_2—$ | $—CH_2CH(CH_3)_2$ | O | H |
| 38 | $3,5—Cl_2—C_6H_3—$ | $—(CH_2)_2—$ | $—C(CH_3)_3$ | O | H |
| 39 | $3,5—Cl_2—C_6H_3—$ | $—(CH_2)_2—$ | H | O | $CH_3$ |
| 40 | $3,5—Cl_2—C_6H_3—$ | $—(CH_2)_2—$ | $—CH(CH_3)_2$ | O | H |
| 41 | $C_6H_5$ | $—(CH_2)_2—$ | $—CH_3$ | O | H |
| 42 | $C_6H_5$ | $—(CH_2)_2—$ | $—CH_3$ | O | H |
| 43 | $C_6H_5$ | $—(CH_2)_2—$ | $—CH_3$ | O | H |
| 44 | $3,5—Cl_2—C_6H_3—$ | $—(CH_2)_2—$ | $—CH(CH_3)_2$ | O | H |
| 45 | $3,5—Cl_2—C_6H_3—$ | $—(CH_2)_2—$ | $—CH(CH_3)_2$ | O | H |
| 47 | $3,5—Cl_2—C_6H_3—$ | $—(CH_2)_2—$ | $—CH(CH_3)_2$ | S | H |
| 48 | $C_6H_5$ | $—(CH_2)_2—$ | $—CH(CH_3)_2$ | S | H |
| 49 | $1—C_{10}H_7$ | $—(CH_2)_2—$ | $—CH(CH_3)_2$ | S | H |
| 50 | $C_6H_5$ | $—(CH_2)_2—$ | $—CH_3$ | S | H |
| 51 | $3,5—Cl_2—C_6H_3—$ | $—(CH_2)_2$ | H | O | $—CH(CH_3)_2$ |
| 52 | $4—C_9H_9{}^b$ | $—(CH_2)_2$ | H | O | $—CH(CH_3)_2$ |

[a] 2,3-dihydro-1H-inden-5-yl
[b] 2,3-dihydro-1H-inden-4-yl

TABLE 1B

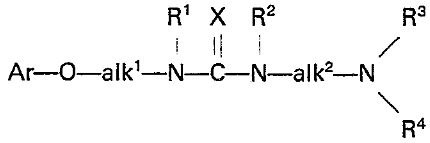

| Ex. No. | alk² | —N—$R^3$,$R^4$ | Salt | M.P.°C. |
|---|---|---|---|---|
| 1 | $-(CH_2)_2-$ | N—$CH_3$,$CH_3$ | — | 53—56 |
| 2 | $-(CH_2)_2-$ | N—$CH_3$,$CH_3$ | HCl · 1/2 $H_2O$ | 108—111 |
| 3 | $-(CH_2)_2-$ | N—$CH_3$,$CH_3$ | maleate | 103—105 |
| 4 | $-(CH_2)_3-$ | N—$CH_3$,$CH_3$ | oxalate | 106—110 |
| 5 | $-(CH_2)_2-$ | N—$CH_3$,$CH_3$ | oxalate | 139—141 |
| 6 | $-(CH_3)_2-$ | N—$CH_3$,$CH_3$ | fumarate | 90—93.5 |
| 7 | $-(CH_2)_3-$ | N—$CH_3$,$CH_3$ | citrate | 109—112 |
| 8 | $-(CH_2)_3-$ | N—$CH_3$,$CH_3$ | citrate | 126—128 |
| 9 | $-(CH_2)_2-$ | N—[—$CH(CH_3)_2]_2$ | HCl | 187—189 |
| 10 | $-(CH_2)_2-$ | N—$CH_3$,$CH_3$ | 1.5 fumarate | 128—130 |
| 11 | $-(CH_2)_2-$ | N—$CH_3$,$CH_3$ | maleate | 131—132.5 |
| 12 | $-(CH_2)_2-$ | N—$CH_3$,$CH_3$ | HCl | 159—162.5 |
| 13 | $-(CH_2)_2-$ | N—$CH_3$,$CH_3$ | maleate | 131—133 |
| 14 | $-(CH_2)_2-$ | N—$CH_3$,$CH_3$ | maleate | 70.5—73.0 |
| 15 | $-(CH_2)_2-$ | N—$CH_3$,$CH_3$ | — | — |
| 16 | $-(CH_2)_2-$ | N—$CH_3$,$CH_3$ | citrate | 122—125 |
| 17 | $-(CH_2)_2-$ | N—$CH_3$,$CH_3$ | fumarate | 87—91 |
| 18 | $-(CH_2)_2-$ | N—$CH_3$,$CH_3$ | fumarate | 119—120 |
| 19 | $-(CH_2)_2-$ | N—$CH_3$,$CH_3$ | fumarate | 120—122 |
| 20 | $-(CH_2)_2-$ | N—$CH_3$,$CH_3$ | fumarate | 94—96 |
| 21 | $-(CH_2)_2-$ | N—$CH_3$,$CH_3$ | maleate | 97—100 |
| 22 | $-(CH_2)_2-$ | N—$CH_3$,$CH_3$ | maleate | 133.5—135 |
| 23 | $-(CH_2)_2-$ | 4-morpholino | fumarate | 105—107 |
| 24 | $-(CH_2)_2-$ | N—$CH_3$,$CH_3$ | 0.5 fumarate | 114—115 |
| 25 | $-(CH_2)_2-$ | N—$CH_3$,$CH_3$ | maleate | 105—107 |
| 26 | $-(CH_3)_2-$ | N—$CH_3$,$CH_3$ | fumarate | 106—108 |
| 27 | $-(CH_2)_2-$ | N—$CH_3$,$CH_3$ | maleate | 128.5—130 |

TABLE 1B (continued)

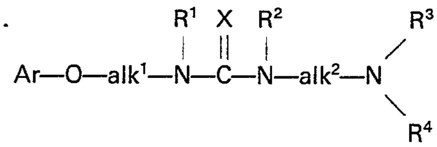

| Ex. No. | alk² | —N—R³,R⁴ | Salt | M.P.°C. |
|---|---|---|---|---|
| 28 | $-(CH_2)_2-$ | $N-CH_3,CH_3$ | maleate | 107.5—109 |
| 29 | $-(CH_2)_2-$ | $N-CH_3,CH_3$ | HCl | 169—171 |
| 30 | $-(CH_2)_2-$ | $N-CH_3,CH_3$ | maleate | 125.5—127.0 |
| 31 | $-(CH_2)_2-$ | $N-CH_3,CH_3$ | maleate | 136.5—138.0 |
| 32 | $-(CH_2)_2-$ | $N-H,CH_3$ | maleate | 119.5—122 |
| 33 | $-(CH_2)_2-$ | $N-H,CH_3$ | fumarate | 62.5—65 |
| 34 | $-(CH_2)_2-$ | $N-CH_3,CH_3$ | maleate | 128—129 |
| 35 | $-(CH_2)_2-$ | $N-CH_3,CH_3$ | maleate | 119—121 |
| 36 | $-(CH_2)_2-$ | $N-CH_3,CH_3$ | maleate | 125.5—126.5 |
| 37 | $-(CH_2)_2-$ | $N-CH_3,CH_3$ | maleate | 117.5—119 |
| 38 | $-(CH_2)_2-$ | $N-CH_3,CH_3$ | maleate | — |
| 39 | $-(CH_2)_2-$ | $N-CH_3,CH_3$ | maleate | 86—89 |
| 40 | $-(CH_2)_2-$ | $N-H,H$ | maleate | 153—154 |
| 41 | $-(CH_2)_2-$ | $N-C_2H_5,C_2H_5$ | 1.5 oxalate | 107—108 |
| 42 | $-(CH_2)_2-$ | $N-C_2H_5,C_2H_5$ | 0.5 $H_2O$ | — |
| 43 | $-(CH_2)_2-$ | 1-pyrrolidinyl | oxalate | 117.5—118.5 |
| 44 | $-(CH_2)_2-$ | $N-C_2H_5,C_2H_5$ | tartrate | 119—120 |
| 45 | $-(CH_2)_2-$ | $N-CH_3,CH_3$ | 1/2 $H_2O$ | — |
| 47 | $-(CH_2)_2-$ | $N-CH_3,CH_3$ | 1/2 fumarate. 1/2 $H_2O$ | 153 |
| 48 | $-(CH_2)_2-$ | $N-C_2H_5,C_2H_5$ | oxalate | 117—117.5 |
| 49 | $-(CH_2)_2-$ | $N-CH_3,CH_3$ | maleate | 130—131 |
| 50 | $-(CH_2)_2-$ | $N-C_2H_5,C_2H_5$ | citrate. 1/2 $H_2O$ | 106—110 |
| 51 | $-(CH_2)_2-$ | $N-CH_3,CH_3$ | maleate | 115—116 |
| 52 | $-(CH_2)_2-$ | $N-CH_3,CH_3$ | maleate | 118—120 |

TABLE 2.

| Ex. No. | Empirical formula | Analytical data: Calculated C | Calculated H | Calculated N | Found C | Found H | Found N |
|---|---|---|---|---|---|---|---|
| 1 | $C_{16}H_{25}N_3O_2Cl_2$ | 53.04 | 6.96 | 14.60 | 52.94 | 6.97 | 11.63 |
| 2 | $C_{16}H_{27}N_3O_{2.5}Cl_3$ | 47.12 | 6.67 | 10.31 | 47.02 | 6.55 | 10.41 |
| 3 | $C_{20}H_{29}N_3O_6Cl_2$ | 50.22 | 6.11 | 8.78 | 50.20 | 6.09 | 8.80 |
| 4 | $C_{19}H_{29}N_3O_6Cl_2$ | 48.93 | 6.27 | 9.01 | 48.87 | 6.25 | 9.03 |
| 5 | $C_{19}H_{29}N_3O_6Cl_2$ | 48.93 | 6.27 | 9.01 | 48.59 | 6.26 | 6.89 |
| 6 | $C_{20}H_{31}N_3O_6$ | 58.66 | 7.63 | 10.26 | 58.45 | 7.62 | 10.25 |
| 7 | $C_{24}H_{37}N_3O_9Cl_2$ | 49.49 | 6.40 | 7.21 | 49.19 | 6.40 | 7.14 |
| 8 | $C_{23}H_{35}N_3O_9Cl_2$ | 48.60 | 6.21 | 7.39 | 48.54 | 6.19 | 7.37 |
| 9 | $C_{20}H_{34}N_3O_2Cl_3$ | 52.81 | 7.53 | 9.24 | 52.45 | 7.51 | 9.08 |
| 10 | $C_{24}H_{37}N_3O_{10}$ | 54.64 | 7.07 | 7.97 | 54.89 | 7.23 | 8.12 |
| 11 | $C_{20}H_{29}N_3O_6Cl_2$ | 50.22 | 6.11 | 8.78 | 50.08 | 6.13 | 8.84 |
| 12 | $C_{19}H_{24}N_3O_2Cl_3$ | 52.73 | 5.59 | 9.71 | 52.51 | 5.61 | 9.79 |
| 13 | $C_{24}H_{33}N_3O_6$ | 62.73 | 7.24 | 9.14 | 62.75 | 7.26 | 9.12 |
| 14 | $C_{20}H_{30}N_3O_6Cl$ | 54.11 | 6.81 | 9.46 | 54.03 | 6.84 | 9.36 |
| 15 | $C_{16}H_{25}N_3O_2Cl_2$ | 53.04 | 6.96 | 11.60 | 52.54 | 6.95 | 11.79 |
| 16 | $C_{23}H_{35}N_3O_9Cl_2$ | 48.60 | 6.21 | 7.39 | 48.61 | 6.17 | 7.35 |
| 17 | $C_{17}H_{23}N_3O_6Cl_2$ | 46.80 | 5.31 | 9.63 | 46.56 | 5.26 | 9.67 |
| 18 | $C_{22}H_{33}N_3O_6$ | 60.39 | 8.06 | 9.60 | 60.38 | 8.11 | 9.58 |
| 19 | $C_{22}H_{35}N_3O_8$ | 56.28 | 7.51 | 8.95 | 56.24 | 7.49 | 8.94 |
| 20 | $C_{22}H_{35}N_3O_6$ | 60.39 | 8.06 | 9.60 | 60.45 | 8.18 | 9.79 |
| 21 | $C_{20}H_{30}N_3O_6Cl$ | 54.11 | 6.81 | 9.46 | 53.73 | 6.69 | 9.64 |
| 22 | $C_{20}H_{29}N_3O_6Cl_2$ | 50.22 | 6.11 | 8.78 | 50.13 | 6.16 | 8.98 |
| 23 | $C_{22}H_{31}N_3O_7Cl_2$ | 50.78 | 6.00 | 8.07 | 50.45 | 6.00 | 7.56 |
| 24 | $C_{19}H_{28}N_3O_4F_3$ | 54.41 | 6.73 | 10.02 | 54.41 | 6.68 | 9.82 |
| 25 | $C_{18}H_{25}N_3O_6Cl_2$ | 48.01 | 5.60 | 9.33 | 47.91 | 5.61 | 9.38 |
| 26 | $C_{20}H_{30}N_3O_6F$ | 56.20 | 7.07 | 9.83 | 56.38 | 7.12 | 9.85 |
| 27 | $C_{20}H_{29}N_3O_6Cl_2$ | 50.22 | 6.11 | 8.78 | 50.34 | 6.08 | 8.82 |
| 28 | $C_{20}H_{30}N_3O_6Cl$ | 54.11 | 6.81 | 9.46 | 53.95 | 6.81 | 9.47 |
| 29 | $C_{20}H_{30}N_3O_2Cl$ | 63.22 | 7.95 | 11.06 | 63.19 | 7.96 | 11.07 |

TABLE 2 (continued)

| Ex. No. | Empirical formula | Analytical data Calculated C | H | N | Found C | H | N |
|---|---|---|---|---|---|---|---|
| 30 | $C_{23}H_{35}N_3O_6$ | 61.45 | 7.85 | 9.35 | 61.52 | 7.90 | 9.32 |
| 31 | $C_{23}H_{35}N_3O_6$ | 61.45 | 7.85 | 9.35 | 61.21 | 7.88 | 9.44 |
| 32 | $C_{20}H_{29}N_3O_6Cl_2$ | 50.22 | 6.11 | 8.78 | 50.16 | 6.12 | 8.84 |
| 33 | $C_{20}H_{29}N_3O_6$ | 58.96 | 7.17 | 10.31 | 58.51 | 7.17 | 10.22 |
| 34 | $C_{21}H_{31}N_3O_6Cl_2$ | 51.23 | 6.35 | 8.53 | 51.30 | 6.40 | 8.65 |
| 35 | $C_{20}H_{29}N_3O_6Cl_2$ | 50.22 | 6.11 | 8.78 | 49.91 | 6.03 | 9.09 |
| 36 | $C_{23}H_{33}N_3O_6Cl_2$ | 53.29 | 6.42 | 8.11 | 53.15 | 6.41 | 8.12 |
| 37 | $C_{21}H_{31}N_3O_6Cl_2$ | 51.23 | 6.35 | 8.53 | 51.35 | 6.38 | 8.62 |
| 38 | $C_{21}H_{31}N_3O_6Cl_2$ | — | — | — | — | — | — |
| 39 | $C_{18}H_{25}N_3O_6Cl_2$ | 48.01 | 5.60 | 9.33 | 48.10 | 5.64 | 9.35 |
| 40 | $C_{18}H_{25}N_3O_6Cl_2$ | 48.01 | 5.60 | 9.33 | 47.92 | 5.59 | 9.34 |
| 41 | $C_{19}H_{30}N_3O_8$ | 53.26 | 7.06 | 9.81 | 53.11 | 7.07 | 9.87 |
| 42 | $C_{16}H_{28}N_3O_{2.5}$ | 63.55 | 9.33 | 13.90 | 63.92 | 9.25 | 13.76 |
| 43 | $C_{18}H_{27}N_3O_6$ | 56.68 | 7.14 | 11.02 | 56.41 | 7.11 | 11.00 |
| 44 | $C_{22}H_{35}N_3O_8$ | 48.89 | 6.53 | 7.78 | 48.83 | 6.60 | 7.71 |
| 45 | $C_{16}H_{26}N_3O_{2.5}Cl_2$ | 51.76 | 7.06 | 11.32 | 51.78 | 6.82 | 11.37 |
| 47 | $C_{18}H_{28}N_3O_{3.5}SCl_2$ | 48.54 | 6.34 | 9.43 | 48.96 | 6.13 | 9.57 |
| 48 | $C_{20}H_{33}N_3O_5S$ | 56.18 | 7.78 | 4.83 | 56.14 | 7.80 | 4.85 |
| 49 | $C_{24}H_{33}N_3O_5S$ | 60.61 | 6.69 | 8.84 | 60.64 | 7.01 | 8.83 |
| 50 | $C_{22}H_{36}N_3O_{8.5}S$ | 51.75 | 7.11 | 8.23 | 51.88 | 7.05 | 8.46 |
| 51 | $C_{20}H_{29}N_3O_6Cl_2$ | 50.22 | 6.11 | 8.78 | 50.02 | 6.14 | 8.89 |
| 52 | $C_{23}H_{35}N_3O_6$ | 61.45 | 7.85 | 9.35 | 61.06 | 7.83 | 9.17 |

Pharmacology

The action of compounds of this invention in correcting cardiac arrhythmias or preventing cardiac arrhythmias is demonstrated by the following procedures:

Ouabain induced arrhythmias

Correction of existing cardiac arrhythmias of ventricular origin is carried out on (1) adult mongrel dogs which are under barbiturate anesthesia during the test. A Grass Model 7 polygraph was used for recording femoral arterial blood pressure (Statham P23AC transducer) and the electrocardiogram (Grass 7P4 preamplifier). Ouabain was given intravenously in an initial dose of 40 µg/kg and in a second dose of 20 µg/kg 30 minutes after the first dose and in subsequent doses of 10 µg/kg which were repeated at 15 min intervals as required for producing cardiac arrhythmias that persisted for at least 15 minutes. When the arrhythmias were established, the test compounds were administered by infusion (Harvard Model 942 Infusion Pump) into a femoral vein at a rate of 1 mg/kg/min. Concentration of compound was adjusted

according to the weight of the dog to allow a volume infusion of 1 ml/min. The compound was considered to be active as antiarrhythmic agent if reversion to sinus rhythm occurred which was maintained for at least 30 min.

Coronary artery ligation induced arrhythmias

Adult mongrel dogs which are in the conscious state were used for the test and cardiac arrhythmias were induced by prior (22—24 hr) surgical preparation in which blood flow through a coronary artery was occluded by use of a constrictor device as reported by Smith et al, 1973. A Grass Model 79 polygraph was used for recording the electrocardiogram (Grass 7P4 preamplifier).

The test compound was administered by infusion (Howard Model 942 Infusion Pump) into a sapheneous vein to one group of dogs at a rate of 0.5 mg/kg/min. Concentration of compound was adjusted according to the weight of the dog to allow a volume of infusion of 0.5 ml/min. The test compound was administered orally by gavage to another group of dogs at dose levels of 10 to 40 mg/kg. The test compound was prepared in distilled water to give a total volume of 20 ml. Following the administration of the test compound, the heart rate number of ectopic cardiac beats per min and the percent ectopic beats (Ectopic beats/HR×100) were recorded at 15 min intervals. The compound was considered active if it abolished the ectopic ventricular frequency and caused a return to normal sinus rhythm within 2 hours of administration.

Injury-stimulus induced arrhythmias

Correction of existing arrhythmias of atrial origin is carried out on adult mongrel dogs which are under barbiturate anesthesia and mechanical respiration (Harvard Respiration Pump Model 6B). During the test a Grass Model 7 polygraph was used for recording femoral arterial blood pressure (Statham P23AC Transducer) and the electrocardiogram (Grass 7p4 preamplifier). The heart was exposed by an incision at the fourth intercostal space of the right thorax and the right atrium was exposed. A band of right atrial tissue lying between the superior and inferior vena cava was crushed using hemostatic forceps. Atrial arrhythmias were initiated by applying an electrical stimulus of 1 m sec, 20-100Hz and 3—5 V to the crushed area (Method of Rosenbluth & Garcia-Ramos). When the arrhythmias were established and persisted for at least 15 min, the test compound was administered by infusion (Harvard Model 940 Infusion Pump) into a femoral vein at a rate of 1 mg/kg/min. Concentration of the test compound was adjusted according to the weight of the dog to allow a volume infusion of 1 ml/min. The compound was considered active as an anti-arrhythmic agent if the induced arrhythmia (atrial flutter or atrial fibrilation) was reverted to a normal sinus rhythm and the atrial frequency is diminished in order that a 1:1 relationship of atrial and ventricular was established.

Acetylcholine induced arrhythmias

Protection activity against the induction of cardiac arrhythmias of atrial origin were carried out in adult mongrel dogs which were under barbiturate anesthesia and mechanical respiration (Harvard Respiration Pump Model 613). A Grass Model 7 Polygraph was used for recording femoral arterial blood pressure (P23Ac Transducer) and the electrocardiogram. The heart was exposed by an incision at the fourth intercostal space of the right thorax and the right atrium was exposed. An atrial arrhythmia was produced by placing 2 drops of a 10% aqueous solution of acetylcholine directly on the right atrium and stroking the area of application with a cotton applicator (Method of Scherf & Chick). The time period of the arrhythmia was determined by noting the spontaneous return of the sinus rhythm of the electrocardiogram. This procedure was repeated at 15 min. intervals until at least 2 consecutive periods of arrhythmia of comparable duration were obtained.

The test compound was administered intravenously as an aqueous solution at an initial dose of 1 mg active compound per kilogram of body weight. After drug administration, attempts were made up to 60 min to reproduce the arrhythmia. Higher dosages of the test compound were administered if lower levels failed to prevent the occurrence of the arrhythmia during the 60 min trial period.

Data obtained for one preferred compound; namely, N - [2 - (3,5 - dichlorophenoxy)ethyl] - N' - [2 - (dimethylamino)ethyl] - N - (1 - methylethyl)urea as represented by its maleate salt of Example 3 are shown in Table 3. This compound exhibited minimal CNS side effects as compared to quinidine and lidocaine. The other compounds of this invention show qualitatively by similar effects in one or more types of arrhythmias as represented by the foregoing tests and generally exhibit less side effects than quinidine or lidocaine.

TABLE 3
Effect of compound of Example 3: N-[2-(3,5-Dichlorophenoxy)ethyl]-N'-[2-(dimethylamino)ethyl]-N-(1-methylethyl) urea on cardiac arrhythmias in dogs

| Arrhythmia model | Arrhythmia type[1] | Correcting dose range mg/kg I.V. | Correcting dose range mg/kg P.O. | Protecting dose range (mg/kg, I.V.) |
|---|---|---|---|---|
| Ouabain-induced[2] | V | 3—15 | | |
| Coronary artery Ligation-induced[3] | V | 3—15 | 10—40 | |
| Injury-stimulus Induced[4] | A | 5—17 | | |
| Acetylcholine-Induced[5] | A | | | 1—8 |

[1] V=Cardiac arrhythmia of ventricular origin.
A=Cardiac arrhythmia of atrial origin.
[2] Cardiac arrhythmias produced by Method of Lucchessi and Hardman, 1961, U. Pharmacol. Exp. Ther., *132*, 372—381.
[3] Cardiac arrhythmias produced by modification of Method of Harris, 1950, Circulation *1*, 1318, as reported by Smith et al, 1973, Pharmacologist *15*, 192.
[4] Cardiac arrhythmias produced by Method of Rosenblueth and Garcia-Ramos 1947, Am. Heart. J. *33*, 677.
[5] Cardiac arrhythmias produced by Method of Scherf and Chick 1951, Circulation *3*, 764.

Pharmaceutical compositions

The invention further provides pharmaceutical compositions for administration to a living animal body comprising, as active ingredients, at least one of the compounds according to the invention in association with a pharmaceutical carrier or excipient. The compounds are thus presented in a therapeutic composition suitable for oral, rectal, parenteral or intracardial administration. Thus, for example, compositions for oral administration are preferably solids and can take the form of capsules, tablets or coated tablets containing carriers conveniently used in the pharmaceutical art. Suitable tableting excipients include lactose, potato and maize starches, talc, gelatin and stearic and silicic acids, magnesium stearate and polyvinyl pyrrolidone.

For parenteral administration, the carrier or excipient can be a sterile, parenterally acceptable liquid; e.g., water, or a parenterally acceptable oil; e.g., arachis oil, contained in ampoules.

In compositions for rectal administration the carrier can comprise a suppository base; e.g., cocoa butter, or a glyceride.

Advantageously, the compositions are formulated as dosage units, each unit being adapted to supply a fixed dose of active ingredients. Tablets, coated tablets, capsules, ampoules and suppositories are examples of preferred dosage forms according to the invention. It is only necessary that the active ingredient constitute an effective amount; i.e., such that a suitable effective dosage will be obtained consistent with the dosage form employed. The exact individual dosages, as well as daily dosages, will, of course, be determined according to standard medical principles under the direction of a physician or veterinarian. Generally, the pharmacology on animals suggests that the oral dosage effective to correct arrhythmias will be about 3 times that of the intravenous dosage. The animal data also suggest dosage requirements will be about half that of quinidine for the more active compounds.

Based on the animal data, allowing for variation in species and severity of cardiac arrhythmia unit dosages containing an amount of compound equivalent to about 1 to about 100 mg/kg of body weight, are contemplated. Based on all of the above considerations, a choice in a range of unit oral dosages for humans of about 10 to about 1000 mg is contemplated, preferably about 10 to 600 mg for a more active compound such as Example 3. Daily dosages of about 30 to 2400 mg are contemplated for humans and obviously several unit dosage forms may be administered at about the same time. However, the scope of the invention is not to be limited by these contemplations due to the uncertainty in transpositions discussed above.

Examples of unit dosage compositions are as follows:

Example 66

Capsules may be prepared as follows:

| Ingredients | Per capsule |
|---|---|
| 1. Active ingredient | 10.0 mg |
| 2. Lactose | 146.0 mg |
| 3. Magnesium stearate | 4.0 mg |

Ingredients 1, 2 and 3 were blended. This blend was then milled and blended again. This milled blend was then filled into #1 hard gelatin capsules.

Examples 67A and 67B

Tablets may be prepared as follows:

| Ingredients | Mg/Tablet |
|---|---|
| 1. Active ingredient | 10.0 mg |
| 2. Corn starch | 20.0 mg |
| 3. Kelacid | 20.0 mg |
| 4. Keltose | 20.0 mg |
| 5. Magnesium stearate | 1.3 mg |

This is Example 67A.

Example 67B

| Ingredients | Mg/Tablet |
|---|---|
| 1. Active ingredient | 50.0 mg |
| 2. Milo starch | 20.0 mg |
| 3. Corn starch | 38.0 mg |
| 4. Lactose | 90.0 mg |
| 5. Calcium stearate | 2.0 mg |
| | 200.0 mg |

The procedure for both Examples 67A and 67B was to blend ingredients 1, 2, 3 and 4 and then add sufficient water portionwise to the blend with careful stirring after each addition. Such additions of water and stirring were continued until the mass was of a consistency to permit its conversion to wet granules. The wet mass was converted to granules by passing it through an oscillating granulator, using 8-mesh screen (U.S. Sieve series which has openings of mms). The wet granules were then dried in an oven at 140°F ( °C). The dried granules were then passed through an oscillating granulator, using a 10-mesh screen (U.S. Sieve series which has openings of mms). The dry granules were then lubricated with 0.5% magnesium stearate. The lubricated granules were compressed on a suitable tablet press.

Example 68

Intravenous injections may be prepared as follows:

| Ingredients | | Per ml |
|---|---|---|
| 1. Active ingredient | | 1.0 mg |
| 2. pH 4.0 Buffer solution | q.s. to | 1.0 ml |

The active ingredient was dissolved in the buffer solution and this solution aseptically filtered. The sterile solution was now aseptically filled into ampoules, which were sealed under aseptic conditions.

Example 69

Intramuscular injections were prepared as follows:

| Ingredients | Per ml |
|---|---|
| 1. Active ingredients | 5.0 mg |
| 2. Isotonic buffer solution 4.0 | q.s. to 1.0 ml |

The active ingredient was dissolved in the buffer solution and this solution aseptically filtered. The sterile solution was now aseptically filled into sterile ampoules which were sealed under aseptic conditions.

Example 70

Suppositories were prepared as follows:

| Ingredients | Per suppository |
|---|---|
| 1. Active ingredient | 10.0 mg |
| 2. Polyethylene glycol 1000 | 1350.0 mg |
| 3. Polyethylene glycol 4000 | 450.0 mg |

Ingredients 2 and 3 were milled together and the mixture stirred until uniform. The active ingredient was dissolved in this molten mass and the mixture stirred until uniform. This molten mass was then poured into suppository moulds and chilled. The suppositories were then removed from the moulds and wrapped.

Therapeutic compositions having cardiac arrhythmia inhibiting activity in dosage unit form, comprising a pharmaceutical carrier and a cardiac arrhythmia inhibiting amount of a compound of Formula I or a pharmaceutically acceptable acid addition salt thereof are therefore an embodiment of this invention.

**Claims for the Contracting States: BE, CH, DE, FR, GB, IT, LI, LU, NL, SE**

1. The use of a compound having the formula:

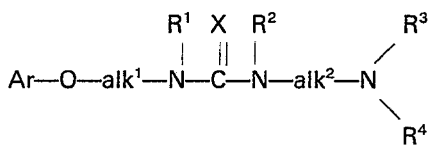

wherein;

Ar represents a 1- or 2-naphthyl, 2,3-dihydro-1-H-inden-4 (or 5)yl, phenyl or substituted phenyl group, the said substituted phenyl group being substituted by 1—3 radicals which may be the same or different selected from halogen atoms or loweralkyl, loweralkoxy, trifluoromethyl, amino, nitro, loweralkylthio, loweralkylsulphinyl, loweralkylsulphonyl, loweralkanoyl, aroylamino or acylamino groups;

$R^1$ and $R^2$ each represent a hydrogen atom or a loweralkyl, cycloalkyl, phenyl or phenyl-loweralkyl group;

X represents an oxygen or a sulphur atom;

$R^3$ and $R^4$ each represent a hydrogen atom or a loweralkyl, phenyl or phenylalkyl group wherein phenyl may be substituted by a halogen atom or a lower alkyl or lower alkoxy group, and $R^3$ and $R^4$ may be the same or different or $R^3$ and $R^4$ taken together with the adjacent nitrogen may form a heterocyclic residue;

$alk^1$ and $alk^2$ each represent a loweralkylene or loweralkylene-loweralkyl group and may be the same or different or a salt or hydrate thereof in the manufacture of a medicament for treating arrhythmias.

2. Compounds having the formula:

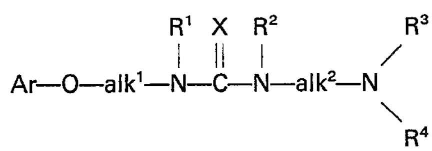

wherein;

Ar represents a 1- or 2-naphthyl, 2,3-dihydro-1-H-inden-4 (or 5)yl, phenyl or substituted phenyl group, the said substituted phenyl group being substituted by 1—3 radicals which may be the same or different selected from halogen atoms or loweralkyl, loweralkoxy, trifluoromethyl, amino, nitro, loweralkylthio, loweralkylsulphinyl, loweralkylsulphonyl, loweralkanoyl, aroylamino or acylamino groups;

$R^1$ and $R^2$ each represent a hydrogen atom or a loweralkyl, cycloalkyl, phenyl or phenyl-loweralkyl group;

X represents an oxygen or a sulphur atom;

$R^3$ and $R^4$ each represent a hydrogen atom or a loweralkyl, phenyl or phenylalkyl group wherein phenyl may be substituted by a halogen atom or a lower alkyl or lower alkoxy group, and $R^3$ and $R^4$ may be the same or different or $R^3$ and $R^4$ taken together with the adjacent nitrogen may form a heterocyclic residue;

$alk^1$ and $alk^2$ each represent a loweralkylene or loweralkylene-loweralkyl group and may be the same or different other than

N'-[2-(diethylamino)ethyl]-N-methyl-N-[2-(phenoxy)ethyl]urea and

N-methyl-N-[2-(phenoxy)ethyl]-N'-[2-(pyrrolidinyl)ethyl]urea,

and the pharmaceutically acceptable addition salts and hydrates thereof.

3. A compound as claimed in Claim 1 or Claim 2 in which Ar represents a substituted phenyl group.

4. A compound as claimed in Claim 3 in which Ar represents a dichloro phenyl group.

5. A compound as claimed in Claim 4 in which Ar represents a $3,4—Cl_2—C_6H_3—$ or a $3,5,—Cl_2C_6H_3—$ group.

6. A compound as claimed in any one of Claims 1 to 4 in which $R^1$ represents a $—CH(CH_3)_2$ group.

7. A compound as claimed in any one of Claims 1 to 6 in which X represents a sulphur atom.

8. A compound as claimed in Claim 3 in which Ar represents a 3,5-dichlorophenyl group, $alk^1$ and $alk^2$ both represent a $—(CH_2)_2—$ group and $R^3$ and $R^4$ both represent a methyl group.

9. A compound as claimed in Claim 1 or Claim 2 which is

N-[2-(3,4-dichlorophenoxy)ethyl]-N'-[2-(dimethylmino)ethyl]-N-(1-methylethyl)urea, or

N-[2-(3,4-dichlorophenoxy)ethyl]-N'-[2-(dimethylamino)ethyl]-N-(1-methylethyl)urea, monohydrochloride monohydrate,

N-[2-(3,4-dichlorophenoxy)ethyl]-N'-[3-(dimethylamino)propyl]-N-(1-methylethyl)urea or

N-[2-(3,4-dichlorophenoxy)ethyl]-N'-[3-(dimethylamino)propyl]-N-(1-methylethyl)urea oxalate [1:1].

10. A compound as claimed in Claim 1 or Claim 2 which is

N-[2-(3,5-dichlorophenoxy)ethyl]-N'-[2-(dimethylamino)ethyl]-N-(1-methylethyl)urea,

N-[2-(3,5-dichlorophenoxy)ethyl]-N'-[2-(dimethylamino)ethyl]-N-(1-methylethyl)urea maleate [1:1],

N-[3-(3,5-dichlorophenoxy)propyl]-N'-[2-(dimethylamino)ethyl]-N-(1-methylethyl)urea,

N-[3-(3,5-dichlorophenoxy)propyl]-N'-[2-(dimethylamino)ethyl]-N-(1-methylethyl)urea oxalate [1:1],

N-[3-(3,5-dichlorophenoxy)propyl]-N'-[3-(dimethylamino)propyl]-N-(1-methylethyl)urea,

N-[3-(3,5-dichlorophenoxy)propyl]-N'-[3-(dimethylamino)propyl]-N-(1-methylethyl)urea citrate [1:1],

N-[2-(3,5-dichlorophenoxy)ethyl]-N'-[3-(dimethylamino)propyl]-N-(1-methylethyl)urea,

N-[2-(3,5-dichlorophenoxy)ethyl]-N'-[3-(dimethylamino)propyl]-N-(1-methylethyl)urea citrate [1:1],

N-[2-(3,5-dichlorophenoxy)ethyl]-N-(1-methylethyl)-N'-[2-[bis(1-methylethyl)amino]ethyl]urea,

N-[2-(3,5-dichlorophenoxy)ethyl]-N-(1-methylethyl)-N'-[2-[bis(1-methylethyl)amino]ethyl]urea hydrochloride [1:1],

N-[2-(3,5-dichlorophenoxy)ethyl]-N'-[2-(dimethylamino)ethyl]-N-phenylurea,

N-[2-(3,5-dichlorophenoxy)ethyl]-N'-[2-(dimethylamino)ethyl]-N-phenylurea monohydrochloride,

N-[2-(3,5-dichlorophenoxy)ethyl]-N'-[2-(dimethylamino)ethyl]-N'-methyl-N-(1-methylethyl)urea,

N-[2-(3,5-dichlorophenoxy)ethyl]-N'-[2-(dimethylamino)ethyl]-N'-methyl-N-(1-methylethyl)urea citrate [1:1],

N-[2-(3,5-dichlorophenoxy)ethyl]-N'-[2-(dimethylamino)ethyl] urea,

N-[2-(3,5-dichlorophenoxy)ethyl]-N'-[2-(dimethylamino)ethyl] urea fumarate [1:1],

N-[2-(3,5-dichlorophenoxy)ethyl]-N-(1-methylethyl)-N'-[2-(4-morpholinyl)ethyl]urea,

N-[2-(3,5-dichlorophenoxy)ethyl]-N-(1-methylethyl)-N'-[2-(4-morpholinyl)ethyl]urea fumarate [1:1],

N-[2-(3,5-dichlorophenoxy)ethyl]-N'-[2-(dimethylamino)ethyl]-N-methyl urea,

N-[2-(3,5-dichlorophenoxy)ethyl]-N'-[2-(dimethylamino)ethyl]-N-methyl urea maleate [1:1],

N-[2-(3,5-dichlorophenoxy)ethyl]-N'-methyl-N'-[2-(methylamino)ethyl]-N-(1-methylethyl)urea,

N-[2-(3,5-dichlorophenoxy)ethyl]-N'-methyl-N'-[2-(methylamino)ethyl]-N-(1-methylethyl)urea maleate [1:1],

N-butyl-N-[2-(3,5-dichlorophenoxy)ethyl]-N'-[2-(dimethylamino)ethyl]urea,

N-butyl-N-[2-(3,5-dichlorophenoxy)ethyl]-N'-[2-(dimethylamino)ethyl]urea maleate [1:1],

N-[2-(3,5-dichlorophenoxy)ethyl]-N'-[2-(dimethylamino)ethyl]-N-propylurea,

N-[2-(3,5-dichlorophenoxy)ethyl]-N'-[2-(dimethylamino)ethyl]-N-propylurea maleate [1:1],

N-cyclohexyl-N-[2-(3,5-dichlorophenoxy)ethyl]-N'-[2-(dimethylamino)ethyl]urea,

N-cyclohexyl-N-[2-(3,5-dichlorophenoxy)ethyl]-N'-[2-(dimethylamino)ethyl]urea maleate [1:1],

N-[2-(3,5-dichlorophenoxy)ethyl]-N'-[2-(dimethylamino)ethyl]-N-(2-methylpropyl)urea,

N-[2-(3,5-dichlorophenoxy)ethyl]-N'-[2-dimethylamino)ethyl]-N-(2-methylpropyl)urea maleate [1:1],

N'-[2-(3,5-dichlorophenoxy)ethyl]-N-[2-(dimethylamino)ethyl]-N-methylurea,

N'-[2-(3,5-dichlorophenoxy)ethyl]-N-[2-(dimethylamino)ethyl]-N-methylurea maleate [1:1],
N-[2-(3,5-dichlorophenoxy)ethyl]-N-(1-methylethyl)-N'-(2-aminoethyl)urea,
N-[2-(3,5-dichlorophenoxy)ethyl]-N-(1-methylethyl)-N'-[2-aminoethyl)urea maleate [1:1],
N-[2-(3,5-dichlorophenoxy)ethyl]-N'-[2-(diethylamino)ethy]-N-(1-methylethyl)urea,
N-[2-(3,5-dichlorophenoxy)ethyl]-N'-[2-(diethylamino)ethyl]-N-(1-methylethyl)urea tartrate [1:1],
N-[2-(3,5-dichlorophenoxy)ethyl]-N'-[2-(dimethylamino)ethyl]-N-(1-methylethyl)urea hydrate [2:1],
N-[2-[(3,5-dichlorophenyl)oxy]ethyl]-N'-[2-(dimethylamino)ethyl]-N'-(1-methylethyl)urea, or
N-[2-[(3,5-dichlorophenyl)oxy]ethyl]-N'-[2-(dimethylamino)ethyl]-N'-(1-methylethyl)urea maleate.

11. A compound as claimed in Claim 1 or Claim 2 which is
N-[2-(2,4-dichlorophenoxy)ethyl]-N'-[2-(dimethylamino)ethyl]-N-(1-methylethyl)urea or
N-[2-(2,4-dichlorophenoxy)ethyl]-N'-[2-(dimethylamino)ethyl]-N-(1-methylethyl)urea maleate [1:1].

12. A compound as claimed in Claim 1 or Claim 2 which is
N-[2-(2,6-dichlorophenoxy)ethyl]-N'-[2-(dimethylamino)ethyl]-N-(1-methylethyl)urea.

13. A compound as claimed in Claim 1 or Claim 2 which is
N-[2-(2,5-dichlorophenoxyethyl]-N'-[2-(dimethylamino)ethyl]-N-(1-methylethyl)urea or
N-[2-(2,5-dichlorophenoxyethyl]-N'-[2-(dimethylamino)ethyl]-N-(1-methylethyl)urea maleate [1:1].

14. A compound as claimed in Claim 1 or Claim 2 which is
N-[2-(2,3-dichlorophenoxy)ethyl]-N'-[2-(dimethylamino)ethyl]-N-(1-methylethyl)urea or
N-[2-(2,3-dichlorophenoxy)ethyl]-N'-[2-(dimethylamino)ethyl]-N-(1-methylethyl)urea maleate [1:1].

15. A compound as claimed in Claim 1 or Claim 2 which is
N'-[2-(dimethylamino)ethyl]-N-[2-(3,4-dimethylphenoxy)ethyl]-N-(1-methylethyl)urea,
N'-[2-(dimethylamino)ethyl]-N-[2-(3,4-dimethylphenoxy)ethyl]-N-(1-methylethyl)urea fumarate [1:1.5],
N'-[2-(dimethylamino)ethyl]-N-[2-(3,4-dimethylphenoxy)ethyl]-N-(1-methylethyl)urea or
N'-[2-(dimethylamino)ethyl]-N-[2-(3,4-dimethylphenoxy)ethyl]-N-(1-methylethyl)urea fumarate [1:1].

16. A compound as claimed in Claim 1 or Claim 2 which is
N'-[2-(dimethylamino)ethyl]-N-[2-(3,5-dimethylphenoxy)ethyl]-N-(1-methylethyl)urea or
N'-[2-(dimethylamino)ethyl]-N-[2-(3,5-dimethylphenoxy)ethyl]-N-(1-methylethyl)urea fumarate [1:1].

17. A compound as claimed in Claim 1 or Claim 2 which is
N-[2-(3,5-dimethoxyphenoxy)ethyl]-N'-[2-(dimethylamino)ethyl]-N-(1-methylethyl)urea, or
N-[2-(3,5-dimethoxyphenoxy)ethyl]-N'-[2-(dimethylamino)ethyl]-N-(1-methylethyl)urea fumarate [1:1].

18. A compound as claimed in Claim 1 or Claim 2 which is
N-[2-(3-chlorophenoxy)ethyl]-N'-[2-(dimethylamino)ethyl]-N-(1-methylethyl)urea,
N-[2-(3-chlorophenoxy)ethyl]-N'-[2-(dimethylamino)ethyl]-N-1-methylethyl)urea maleate [1:1],
N-[2-(3-chlorophenoxy)ethyl]-N'-[2-(dimethylamino)ethyl]-N-(1-methylethyl)urea,
N-[2-(3-chlorophenoxy)ethyl]-N'-[2-dimethylamino)ethyl]-N-(1-methylethyl)urea maleate [1:1],
N-[2-(2-chlorophenoxy)ethyl]-N'-[2-(dimethylamino)ethyl]-N-(1-methylethyl)urea or
N-[2-(2-chlorophenoxy)ethyl]-N'-[2-(dimethylamino)ethyl]-N-(1-methylethyl)urea maleate [1:1].

19. A compound as claimed in Claim 1 or Claim 2 which is
N'-[2-(dimethylamino)ethyl]-N-[2-(3-fluorophenoxy)ethyl]-N-(1-methylethyl)urea or
N'-[2-(dimethylamino)ethyl]-N-[2-(3-fluorophenoxy)ethyl]-N-(1-methylethyl)urea fumarate [1:1].

20. A compound as claimed in Claim 1 or Claim 2 which is
N'-[2-(dimethylamino)ethyl]-N-(1-methylethyl)-N-[2-(3-trifluoromethylphenoxy)ethyl]urea, or
N'-[2-(dimethylamino)ethyl]-N-(1-methylethyl)-N-[2-(3-trifluoromethylphenoxy)ethyl]urea fumarate [1:1].

21. A compound as claimed in Claim 1 or Claim 2 which is
N'-[2-(dimethylamino)ethyl]-N-(1-methylethyl)-N-[2-(1-naphthalenyloxy)ethyl]urea,
N'-[2-(dimethylamino)ethyl]-N-(1-methylethyl)-N-[2-(1-naphthalenyloxy)ethyl]urea maleate [1:1],
N'-[2-(dimethylamino)ethyl]-N-(1-methylethyl)-N-[2-(2-naphthalenyloxy)ethyl]urea, or
N'-[2-(dimethylamino)ethyl]-N-(1-methylethyl)-N-[2-(2-naphthalenyloxy)ethyl]urea hydrochloride.

22. A compound as claimed in Claim 1 or Claim 2 which is
N'-[2-(dimethylamino)ethyl]-N-[2-[(2,3-dihydro-1H-inden-5-yl)oxy]ethyl]-N-(1-methylethyl)urea,
N'-[2-(dimethylamino)ethyl]-N-[2-[(2,3-dihydro-1H-inden-5-yl)oxy]ethyl]-N-(1-methylethylurea) maleate [1:1],
N'-[2-(dimethylamino)ethyl]-N-[2-[(2,3-dihydro-1H-inden-4-yl)oxy]ethyl]-N-(1-methylethyl)urea,
N'-[2-(dimethylamino)ethyl]-N-[2-[(2,3-dihydro-1H-inden-4-yl)oxy]ethyl]-N-(1-methylethyl)urea maleate [1:1],
N'-[2-[(2,3-dihydro-1H-inden-4-yl)oxy]ethyl]-N-[2-(methylamino)ethyl]-N-methylurea,
N'-[2-[(2,3-dihydro-1H-inden-4-yl)oxy]ethyl]-N-[2-(methylamino)ethyl]-N-methylurea fumarate [1:1],
N-[2-[(2,3-dihydro-1H-inden-4-yl)oxy]ethyl]-N'-[2-(dimethylamino)ethyl]-N'-(1-methylethyl)urea, or
N-[2-[(2,3-dihydro-1H-inden-4-yl)oxy]ethyl]-N'-[2-(dimethylamino)ethyl]-N'-(1-methylethyl)urea maleate [1:1].

23. A compound as claimed in Claim 1 or Claim 2 which is
N-[2-(dimethylamino)ethyl]-N'-(1-methylethyl)-N'-(2-phenoxyethyl)urea, or
N-[2-(dimethylamino)ethyl]-N'-(1-methylethyl)-N'-(2-phenoxyethyl)urea fumarate [1:1].

24. A compound as claimed in Claim 1 or Claim 2 which is

N-[2-(3,5-dichlorophenoxy)ethyl]-N'-[2-(dimethylamino)ethyl]-N-(1-methylethyl)thiourea,

N-[2-(3,5-dichlorophenoxy)ethyl]-N'-[2-(dimethylamino)ethyl]-N-(1-methylethyl)thiourea, fumarate hydrate [2:1],

N-[2-(diethylamino)ethyl]-N'-(1-methylethyl)-N'-(2-phenoxyethyl)thiourea,

N-[2-(diethylamino)ethyl]-N'-(1-methylethyl)-N'-(2-phenoxyethyl)thiourea oxalate [1:1],

N-[2-(dimethylamino)ethyl]-N-(1-methylethyl)-N-[2-(1-naphthalenyloxy)ethyl]thiourea,

N'-[2-(dimethylamino)ethyl]-N-(1-methylethyl)-N-[2-(1-naphthalenyloxy)ethyl]thiourea maleate [1:1],

N'-[2-(dimethylamino)ethyl]-N-methyl-N-(2-phenoxyethyl)thiourea, or

N'-[2-(diethylamino)ethyl]-N-methyl-N-(2-phenoxyethyl)thiourea, citrate hemihydrate.

25. A therapeutic composition having cardiac arrhythmic inhibiting activity comprising (a) an effective amount of a compound as claimed in any one of Claims 1 to 24 (b) a pharmaceutical carrier therefor.

26. Capsules, tablets, ampoules or suppositories containing

(a) compounds having the formula:

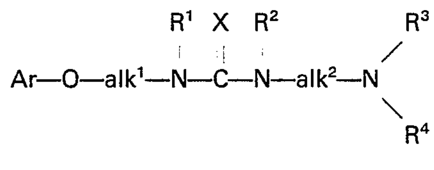

wherein;

Ar represents a 1- or 2-naphthyl, 2,3-dihydrol-1-H-inden-4 (or 5)yl, phenyl or substituted phenyl group, the said substituted phenyl group being substituted by 1—3 radicals which may be the same or different selected from halogen atoms or loweralkyl, loweralkoxy, trifluoromethyl, amino, nitro, loweralkylthio, loweralkylsulphinyl, loweralkylsulphonyl, loweralkanoyl, aroylamino or acylamino groups;

$R^1$ and $R^2$ each represent a hydrogen atom or a loweralkyl, cycloalkyl, phenyl or phenyl-loweralkyl group;

X represents an oxygen or a sulphur atom;

$R^3$ and $R^4$ each represent a hydrogen atom or a loweralkyl, phenyl or phenylalkyl group wherein phenyl may be substituted by a halogen atom or a lower alkyl or lower alkoxy group, and $R^3$ and $R^4$ may be the same or different or $R^3$ and $R^4$ taken together with the adjacent nitrogen may form a heterocyclic residue;

$alk^1$ and $alk^2$ each represent a loweralkylene or loweralkylene-loweralkyl group and may be the same or different or a pharmaceutically acceptable addition salt or hydrates thereof and

(b) a pharmaceutical carrier therefor.

27. Compounds having the formula:

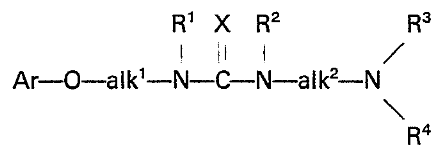

wherein;

Ar represents a 1- or 2-naphthyl, 2,3-dihydro-1-H-inden-4 (or 5)yl, phenyl or substituted phenyl group, the said substituted phenyl group being substituted by 1—3 radicals which may be the same or different selected from halogen atoms or loweralkyl, loweralkoxy, trifluoromethyl, amino, nitro, loweralkylthio, loweralkylsulphinyl, loweralkylsulphonyl, loweralkanoyl, aroylamino or acylamino groups;

$R^1$ and $R^2$ each represent a hydrogen atom or a loweralkyl, cycloalkyl, phenyl or phenylloweralkyl group;

X reprsents an oxygen or a sulphur atom;

$R^3$ and $R^4$ each represent a hydrogen atom or a loweralkyl, phenyl or phenylalkyl group wherein phenyl may be substituted by a halogen atom or a lower alkyl or lower alkoxy group, and $R^3$ and $R^4$ may be the same or different or $R^3$ and $R^4$ taken together with the adjacent nitrogen may form a heterocyclic residue;

$alk^1$ and $alk^2$ each represent a loweralkylene or loweralkylene-loweralkyl group and may be the same or different, other than

N'-[2-(diethylamino)ethyl]-N-methyl-N-[2-(phenoxy)ethyl]urea and

N-methyl-N-[2-(phenoxy)ethyl]-N$^1$-[2-(pyrrolidinyl)ethyl]urea,

and the pharmaceutically acceptable addition salts and hydrates thereof for use in treating arrhythmias.

**Claims for the Contracting State: AT**

1. A process for the preparation of a compound of general formula I

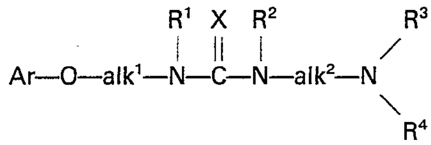

(I)

wherein;

Ar represents a 1- or 2-naphthyl, 2,3-dihydro-1-H-inden-4 (or 5)yl, phenyl or substituted phenyl group, the said substituted phenyl group being substituted by 1—3 radicals which may be the same or different selected from halogen atoms of loweralkyl, loweralkoxy, trifluoromethyl, amino, nitro, loweralkylthio, loweralkylsulphinyl, loweralkylsulphonyl, loweralkanoyl, aroylamino or acylamino groups;

$R^1$ and $R^2$ each represent a hydrogen atom or a loweralkyl, cycloalkyl, phenyl or phenyl-loweralkyl group;

X represents an oxygen or a sulphur atom;

$R^3$ and $R^4$ each represent a hydrogen atom or a loweralkyl, phenyl or phenylalkyl group wherein phenyl may be substituted by a halogen atom or a lower alkyl or lower alkoxy group, and $R^3$ and $R^4$ may be the same or different or $R^3$ and $R^4$ taken together with the adjacent nitrogen may form a heterocyclic residue;

$alk^1$ and $alk^2$ each represent a loweralkylene or loweralkylene-loweralkyl group and may be the same or different other than

N'-[2-(diethylamino)ethyl]-N-methyl-N-[2-(phenoxy)ethyl]urea and

N-methyl-N-[-(phenoxy)ethyl]-N'-[2-(pyrrolidinyl)ethyl]urea,

or a pharmaceutically acceptable addition salt or hydrate thereof, the process comprising:

(A) reacting a compound of general Formula IIa

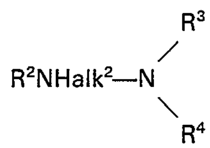

(IIa)

wherein $R^2$, $alk^2$, $R^3$ and $R^4$ are as defined for formula I with the proviso that when $R^2$ does not represent a hydrogen atom, $R^3$ and $R^4$ must be other than a hydrogen atom, or $R^2$ is the same as $R^3$, and $R^4$ represents a hydrogen atom, with a compound of general formula III:

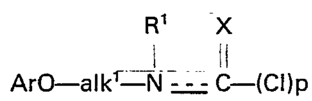

(III)

wherein Ar, $alk^1$, $R^1$ and X are as defined for general formula I and when $R^1$ represents a hydrogen atom, p is zero and the dotted line is a double bond forming an isocyanate; otherwise p is one and the dotted line has no significance;

(B) when X represents an oxygen atom and $R^2$ represents a hydrogen atom, reacting a compound of general formula IIb

$$H_2N—alk^2NR^3R^4 \qquad (IIb)$$

wherein $alk^2$, $R^3$ and $R^4$ are as defined for general formula I, and a compound of general formula IVa

$$ArO—alk^1—NHR^1 \qquad (IVa)$$

wherein Ar, $alk^1$ and $R^1$ are as defined for general formula I, with 1,1'-carbonyldiimidazole;

(C) when X represents an oxygen atom and $R^1$ represents a hydrogen atom, reacting a compound of general formula IIb'

$$R^2NH—alk^2—NR^3R^4 \qquad (IIb')$$

wherein $R^2$, $alk^2$, $R^3$ and $R^4$ are as defined for general formula I, and a compound of general formula IVb

$$Ar—O—alk^1—NH_2 \qquad (IVb)$$

wherein Ar and $alk^1$ are as defined for general formula I, with 1,1'-carbonyldiimidazole; or

(D) reacting a compound of general formula V

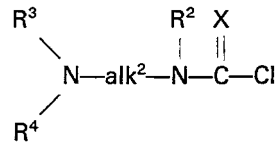

(V)

wherein, X, $R^2$, $alk^2$, $R^3$ and $R^4$ are as defined for general formula I except that $R^2$, $R^3$ and $R^4$ never represent a hydrogen atom, and a compound of general formula IVa

$$Ar—O—alk^1—NHR^1 \quad \text{(IVa)}$$

wherein Ar, $alk^1$ and $R^1$ are as defined for general formula I above, with triethylamine, and subsequently if desired forming a pharmaceutically acceptable addition salt or hydrate of a compound so formed.

2. A process as claimed in Claim 1 in which Ar represents a substituted phenyl group.

3. A process as claimed in Claim 2 in which Ar represents a dichloro phenyl group.

4. A process as claimed in Claim 3 in which Ar represents a 3,4—$Cl_2$—$C_6H_3$— or a 3,5,—$Cl_2C_6H_3$— group.

5. A process as claimed in Claim 1, 2 or 3 in which $R^1$ represents a —$CH(CH_3)_2$ group.

6. A process as claimed in any one of Claims 1 to 5 in which X represents a sulphur atom.

7. A process as claimed in Claim 2 in which Ar represents a 3,5-dichlorophenyl group, $alk^1$ and $alk^2$ both represent a —$(CH_2)_2$— group and $R^3$ and $R^4$ both represent a methyl group.

8. A process as claimed in Claim 1 for preparing
N-[2-(3,4-dichlorophenoxy)ethyl]-N'-[2-(dimethylamino)ethyl]-N-(1-methylethyl)urea, or
N-[2-(3,4-dichlorophenoxy)ethyl]-N'-[2-(dimethylamino)ethyl]-N-(1-methylethyl)urea, mono-hydrochloride monohydrate,
N-[2-(3,4-dichlorophenoxy)ethyl]-N'-[3-(dimethylamino)propyl]-N-(1-methylethyl)urea or
N-[2-(3,4-dichlorophenoxy)ethyl]-N'-[3-(dimethylamino)propyl]-N-(1-methylethyl)urea oxalate [1:1].

9. A process as claimed in Claim 1 for preparing
N-[2-(3,5-dichlorophenoxy)ethyl]-N'-[2-(dimethylamino)ethyl]-N-(1-methylethyl)urea,
N-[2-(3,5-dichlorophenoxy)ethyl]-N'-[2-(dimethylamino)ethyl]-N-(1-methylethyl)urea maleate [1:1],
N-[3-(3,5-dichlorophenoxy)propyl]-N'-[2-(dimethylamino)ethyl]-N-(1-methylethyl)urea,
N-[3-(3,5-dichlorophenoxy)propyl]-N'-[2-(dimethylamino)ethyl]-N-(1-methylethyl)urea oxalate [1:1],
N-[3-(3,5-dichlorophenoxy)propyl]-N'-[3-(dimethylamino)propyl]-N-(1-methylethyl)urea,
N-[3-(3,5-dichlorophenoxy)propyl]-N'-[3-(dimethylamino)propyl]-N-(1-methylethyl)urea citrate [1:1],
N-[2-(3,5-dichlorophenoxy)ethyl]-N'-[3-(dimethylamino)propyl]-N-(1-methylethyl)urea,
N-[2-(3,5-dichlorophenoxy)ethyl]-N'-[3-(dimethylamino)propyl]-N-(1-methylethyl)urea citrate [1:1],
N-[2-(3,5-dichlorophenoxy)ethyl]-N-(1-methylethyl)-N'-[2-[bis(1-methylethyl)amino]ethyl]urea,
N-[2-(3,5-dichlorophenoxy)ethyl]-N-(1-methylethyl)-N'-[2-[bis(1-methylethyl)amino]ethyl]urea hydrochloride [1:1],
N-[2-(3,5-dichlorophenoxy)ethyl]-N'-[2-(dimethylamino)ethyl]-N-phenylurea,
N-[2-(3,5-dichlorophenoxy)ethyl]-N'-[2-(dimethylamino)ethyl]-N-phenylurea monohydrochloride,
N-[2-(3,5-dichlorophenoxy)ethyl]-N'-[2-(dimethylamino)ethyl]-N'-methyl-N-(1-methylethyl)urea,
N-[2-(3,5-dichlorophenoxy)ethyl]-N'-[2-(dimethylamino)ethyl]-N'-methyl-N-(1-methylethyl)urea citrate [1:1],
N-[2-(3,5-dichlorophenoxy)ethyl]-N'-[2-(dimethylamino)ethyl]urea,
N-[2-(3,5-dichlorophenoxy)ethyl]-N'-[2-(dimethylamino)ethyl]urea fumarate [1:1],
N-[2-(3,5-dichlorophenoxy)ethyl]-N-(1-methylethyl)-N'-[2-4-morpholinyl)ethyl]urea,
N-[2-(3,5-dichlorophenoxy)ethyl]-N-(1-methylethyl)-N'-[2-(4-morpholinyl)ethyl]urea fumarate [1:1],
N-[2-(3,5-dichlorophenoxy)ethyl]-N'-[2-(dimethylamino)ethyl]-N-methyl urea,
N-[2-(3,5-dichlorophenoxy)ethyl]-N'-[2-(dimethylamino)ethyl]-N-methyl urea maleate [1:1],
N-[2-(3,5-dichlorophenoxy)ethyl]-N'-methyl-N'-[2-(methylamino)ethyl]-N-(1-methylethyl)urea,
N-[2-(3,5-dichlorophenoxy)ethyl]-N'-methyl-N'-[2-(methylamino)ethyl]-N-(1-methylethyl)urea maleate [1:1],
N-butyl-N-[2-(3,5-dichlorophenoxy)ethyl]-N'-[2-(dimethylamino)ethyl]urea,
N-butyl-N-[2-(3,5-dichlorophenoxy)ethyl]-N'-[2-(dimethylamino)ethyl]urea maleate [1:1],
N-[2-(3,5-dichlorophenoxy)ethyl]-N'-[2-(dimethylamino)ethyl]-N-propylurea,
N-[2-(3,5-dichlorophenoxy)ethyl]-N'-[2-(dimethylamino)ethyl]-N-propylurea maleate [1:1],
N-cyclohexyl-N-[2-(3,5-dichlorophenoxy)ethyl]-N'-[2-(dimethylamino)ethyl]urea,
N-cyclohexyl-N-[2-(3,5-dichlorophenoxy)ethyl]-N'-[2-(dimethylamino)ethyl]urea maleate [1:1],
N-[2-(3,5-dichlorophenoxy)ethyl]-N'-[2-(dimethylamino)ethyl]-N-(2-methylpropyl)urea,
N-[2-(3,5-dichlorophenoxy)ethyl]-N'-[2-(dimethylamino)ethyl]-N-(2-methylpropyl)urea maleate [1:1],
N'-[2-(3,5-dichlorophenoxy)ethyl]-N-[2-(dimethylamino)ethyl]-N-methylurea,
N'-[2-(3,5-dichlorophenoxy)ethyl]-N-[2-(dimethylamino)ethyl]-N-methylurea maleate [1:1],
N-[2-(3,5-dichlorophenoxy)ethyl]-N-(1-methylethyl)-N'-(2-aminoethyl)urea,
N-[2-(3,5-dichlorophenoxy)ethyl]-N-(1-methylethyl)-N'-(2-aminoethyl)urea maleate [1:1],

N-[2-(3,5-dichlorophenoxy)ethyl]-N'-[2-(diethylamino)ethyl]-N-(1-methylethyl)urea,
N-[2-(3,5-dichlorophenoxy)ethyl]-N'-[2-(diethylamino)ethyl]-N-(1-methylethyl)urea tartrate [1:1],
N-[2-(3,5-dichlorophenoxy)ethyl]-N'-[2-(dimethylamino)ethyl]-N-(1-methylethyl)urea hydrate [2:1],
N-[2-[(3,5-dichlorophenyl)oxy]ethyl]-N'-[2-(dimethylamino)ethyl]-N'-(1-methylethyl)urea, or
N-[2-[(3,5-dichlorophenyl)oxy]ethyl]-N'-[2-(dimethylamino)ethyl]-N'-(1-methylethyl)urea maleate.

10. A process as claimed in Claim 1 for preparing
N-[2-(2,4-dichlorophenoxy)ethyl]-N'-[2-(dimethylamino)ethyl]-N-(1-methylethyl)urea or
N-[2-(2,4-dichlorophenoxy)ethyl]-N'-[2-(dimethylamino)ethyl]-N-(1-methylethyl)urea maleate [1:1].

11. A process as claimed in Claim 1 for preparing
N-[2-(2,6-dichlorophenoxy)ethyl]-N'-[2-(dimethylamino)ethyl]-N-(1-methylethyl)urea.

12. A process as claimed in Claim 1 for preparing
N-[2-(2,5-dichlorophenoxyethyl]-N'-[2-(dimethylamino)ethyl]-N-(1-methylethyl)urea or
N-[2-(2,5-dichlorophenoxyethyl]-N'-[2-(dimethylamino)ethyl]-N-(1-methylethyl)urea maleate [1:1].

13. A process as claimed in Claim 1 for preparing
N-[2-(2,3-dichlorophenoxy)ethyl]-N'-[2-(dimethylamino)ethyl]-N-(1-methylethyl)urea or
N-[2-(2,3-dichlorophenoxy)ethyl]-N'-[2-(dimethylamino)ethyl]-N-(1-methylethyl)urea maleate [1:1].

14. A process as claimed in Claim 1 for preparing
N'-[2-(dimethylamino)ethyl]-N-[2-(3,4-dimethylphenoxy)ethyl]-N-(1-methylethyl)urea,
N'-[2-(dimethylamino)ethyl]-N-[2-(3,4-dimethylphenoxy)ethyl]-N-(1-methylethyl)urea fumarate [1:1.5],
N'-[2-(dimethylamino)ethyl]-N-[2-(3,4-dimethylphenoxy)ethyl]-N-(1-methylethyl)urea or
N'-[2-(dimethylamino)ethyl]-N-[2-(3,4-dimethylphenoxy)ethyl]-N-(1-methylethyl)urea fumarate [1:1].

15. A process as claimed in Claim 1 for preparing
N'-[2-(dimethylamino)ethyl]-N-[2-(3,5-dimethylphenoxy)ethyl]-N-(1-methylethyl)urea or
N'-[2-(dimethylamino)ethyl]-N-[2-(3,5-dimethylphenoxy)ethyl]-N-(1-methylethyl)urea fumarate [1:1].

16. A process as claimed in Claim 1 for preparing
N-[2-(3,5-dimethoxyphenoxy)ethyl]-N'-[2-(dimethylamino)ethyl]-N-(1-methylethyl)urea, or
N-[2-(3,5-dimethoxyphenoxy)ethyl]-N'-[2-(dimethylamino)ethyl]-N-(1-methylethyl)urea fumarate [1:1].

17. A process as claimed in Claim 1 for preparing
N-[2-(3-chlorophenoxy)ethyl]-N'-[2-(dimethylamino)ethyl]-N-(1-methylethyl)urea,
N-[2-(3-chlorophenoxy)ethyl]-N'-[2-(dimethylamino)ethyl]-N-(1-methylethyl)urea maleate [1:1],
N-[2-(3-chlorophenoxy)ethyl]-N'-[2-(dimethylamino)ethyl]-N-(1-methylethyl)urea,
N-[2-(3-chlorophenoxy)ethyl]-N'-[2-dimethylamino)ethyl]-N-(1-methylethyl)urea maleate [1:1],
N-[2-(2-chlorophenoxy)ethyl]-N'-[2-(dimethylamino)ethyl]-N-(1-methylethyl)urea or
N-[2-(2-chlorophenoxy)ethyl]-N'-[2-(dimethylamino)ethyl]-N-(1-methylethyl)urea maleate [1:1].

18. A process as claimed in Claim 1 for preparing
N'-[2-(dimethylamino)ethyl]-N-[2-(3-fluorophenoxy)ethyl]-N-(1-methylethyl)urea or
N'-[2-(dimethylamino)ethyl]-N-[2-(3-fluorophenoxy)ethyl]-N-(1-methylethyl)urea fumarate [1:1].

19. A process as claimed in Claim 1 for preparing
N'-[2-(dimethylamino)ethyl]-N-(1-methylethyl)-N-[2-(3-trifluoromethylphenoxy)ethyl]urea, or
N'-[2-(dimethylamino)ethyl]-N-(1-methylethyl)-N-[2-(3-trifluoromethylphenoxy)ethyl]urea fumarate [1:1].

20. A process as claimed in Claim 1 for preparing
N'-[2-(dimethylamino)ethyl]-N-(1-methylethyl)-N-[2-(1-naphthalenyloxy)ethyl]urea,
N'-[2-(dimethylamino)ethyl]-N-(1-methylethyl)-N-[2-(1-naphthalenyloxy)ethyl]urea maleate [1:1],
N'-[2-(dimethylamino)ethyl]-N-(1-methylethyl)-N-[2-(2-naphthalenyloxy)ethyl]urea, or
N'-[2-(dimethylamino)ethyl]-N-(1-methylethyl)-N-[2-(2-naphthalenyloxy)ethyl]urea hydrochloride.

21. A process as claimed in Claim 1 for preparing
N'-[2-(dimethylamino)ethyl]-N-[2-[(2,3-dihydro-1H-inden-5-yl)oxy]ethyl]-N-(1-methylethyl)urea,
N'-[2-(dimethylamino)ethyl]-N-[2-[(2,3-dihydro-1H-inden-5-yl)oxy]ethyl]-N-(1-methylethylurea) maleate [1:1],
N'-[2-(dimethylamino)ethyl]-N-[2-[(2,3-dihydro-1H-inden-4-yl)oxy]ethyl]-N-(1-methylethyl)urea,
N'-[2-(dimethylamino)ethyl]-N-[2-[(2,3-dihydro-1H-inden-4-yl)oxy]ethyl]-N-(1-methylethyl)urea maleate [1:1],
N'-[2-[(2,3-dihydro-1H-inden-4-yl)oxy]ethyl]-N-[2-(methylamino)ethyl]-N-methylurea,
N'-[2-[(2,3-dihydro-1H-inden-4-yl)oxy]ethyl]-N-[2-(methylamino)ethyl]-N-methylurea fumarate [1:1],
N-[2-[(2,3-dihydro-1H-inden-4-yl)oxy]ethyl]-N'-[2-(dimethylamino)ethyl]-N'-(1-methylethyl)urea, or
N-[2-[(2,3-dihydro-1H-inden-4-yl)oxy]ethyl]-N'-[2-(dimethylamino)ethyl]-N'-(1-methylethyl)urea maleate [1:1].

22. A process as claimed in Claim 1 for preparing
N-[2-(dimethylamino)ethyl]-N'-(1-methylethyl)-N'-(2-phenoxyethyl)urea, or
N-[2-(dimethylamino)ethyl]-N'-(1-methylethyl)-N'-(2-phenoxyethyl)urea fumarate [1:1].

23. A process as claimed in Claim 1 for preparing
N-[2-(3,5-dichlorophenoxy)ethyl]-N'-[2-(dimethylamino)ethyl]-N-(1-methyl)thiourea,
N-[2-(3,5-dichlorophenoxy)ethyl]-N'-[2-(dimethylamino)ethyl]-N-(1-methylethyl)thiourea, fumarate hydrate [2:1],

N-[2-(diethylamino)ethyl]-N'-(1-methylethyl)-N'-(2-phenoxyethyl)thiourea,
N-[2-(diethylamino)ethyl]-N'-(1-methylethyl)-N'-(2-phenoxyethyl)thiourea oxalate [1:1],
N-[2-(dimethylamino)ethyl]-N-(1-methylethyl)-N-[2-(1-naphthalenyloxy)ethyl]thiourea,
N'-[2-(dimethylamino)ethyl]-N-(1-methylethyl)-N-[2-(1-naphthalenyloxy)ethyl]thiourea maleate [1:1],
N'-[2-(dimethylamino)ethyl]-N-methyl-N-(2-phenoxyethyl)thiourea, or
N'-[2-(diethylamino)ethyl]-N-methyl-N-(2-phenoxyethyl)thiourea, citrate hemihydrate.

24. A process for preparing a therapeutic composition having cardiac arrhythmic inhibiting activity comprising admixing (a) an effective amount of a compound having the formula:

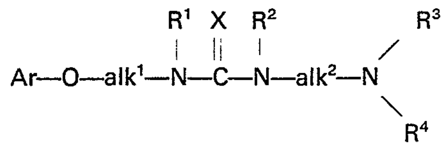

wherein;

Ar represents a 1- or 2-naphthyl, 2,3-dihydro-1-H-inden-4 (or 5)yl, phenyl or substituted phenyl group, the said substituted phenyl group being substituted by 1—3 radicals which may be the same or different selected from halogen atoms or loweralkyl, loweralkoxy, trifluoromethyl, amino, nitro, loweralkylthio, lower alkylsulphinyl, loweralkylsulphonyl, loweralkanoyl, aroylamino or acylamino groups;

$R^1$ and $R^2$ each represent a hydrogen atom or a loweralkyl, cycloalkyl, phenyl or phenyl-loweralkyl group;

X represents an oxygen or a sulphur atom;

$R^3$ and $R^4$ each represent a hydrogen atom or a loweralkyl, phenyl or phenylalkyl group wherein phenyl may be substituted by a halogen atom or a lower alkyl or lower alkoxy group, and $R^3$ and $R^4$ may be the same or different or $R^3$ and $R^4$ taken together with the adjacent nitrogen may form a heterocyclic residue;

$alk^1$ and $alk^2$ each represent a loweralkylene or loweralkylene-loweralkyl group and may be the same or different or a salt or hydrate thereof, and (b) a pharmaceutically acceptable carrier therefor.

25. A process for preparing a therapeutic composition having cardiac arrhythmic inhibiting activity comprising admixing a compound prepared by a process as claimed in any one of Claims 1 to 24 with a pharmaceutically acceptable carrier therefor.

26. A process for the preparation of capsules, tablets, ampoules or suppositories having cardiac arrhythmia inhibiting activity, the process comprising admixing (a) compounds having the formula:

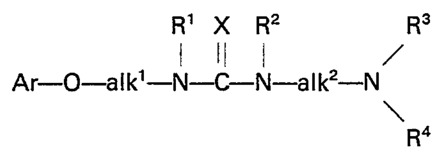

wherein;

Ar represents a 1- or 2-naphthyl, 2,3-dihydrol-1-H-inden-4 (or 5)yl, phenyl or substituted phenyl group, the said substituted phenyl group being substituted by 1—3 radicals which may be the same or different selected from halogen atoms or loweralkyl, loweralkoxy, trifluoromethyl, amino, nitro, loweralkylthio, loweralkylsulphinyl, loweralkylsulphonyl, loweralkanoyl, aroylamino or acylamino groups;

$R^1$ and $R^2$ each represent a hydrogen atom or a loweralkyl, cycloalkyl, phenyl or phenyl-loweralkyl group;

X represents an oxygen or a sulphur atom;

$R^3$ and $R^4$ each represent a hydrogen atom or a loweralkyl, phenyl or phenylalkyl group wherein phenyl may be substituted by a halogen atom or a lower alkyl or lower alkoxy group, and $R^3$ and $R^4$ may be the same or different or $R^3$ and $R^4$ taken together with the adjacent nitrogen may form a heterocyclic residue;

$alk^1$ and $alk^2$ each represent a loweralkylene or loweralkylene-loweralkyl group and may be the same or different or a pharmaceutically acceptable addition salt or hydrates thereof and

(b) a pharmaceutical carrier therefor, and subsequently preparing capsules, tablets, ampoules or suppositories from the mixture.

**Patentansprüche für die Vertragsstaaten: BE, CH, DE, FR, GB, IT, LI, LU, NL, SE**

1. Verwendung einer Verbindung der Formel

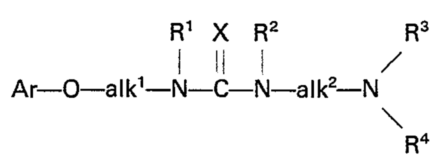

worin:

Ar eine 1-oder 2-Naphthyl-, 2,3-Dihydro-1-H-inden-4-(oder 5)-yl oder Phenylgruppe darstellt oder eine substituierte Phenylgruppe darstellt, wobei die substituierte Phenylgruppe durch 1 bis 3 Gruppen substituiert ist, die gleich oder verschieden ausgewählt sein können aus Halogenatomen oder Niederalkyl-, Niederalkoxy-, Trifluormethyl-, Amino-, Nitro-, Niederalkylthio-, Niederalkylsulfinyl-, Niederalkylsulfonyl-, Niederalkanoyl-, Aroylamino- oder Acylaminogruppen;

$R^1$ und $R^2$ je ein Wasserstoffatom oder eine Niederalkyl-, Cycloalkyl-, Phenyl- oder Phenylniederalkylgruppe darstellen;

X ein Sauerstoff- oder ein Schwefelatom darstellt;

$R^3$ und $R^4$ je ein Wasserstoffatom oder eine Niederalkyl-, Phenyl- oder Phenylalkylgruppe, worin Phenyl durch ein Halogenatom oder eine Niederalkyl- oder Niederalkoxygruppe substituiert sein kann, darstellen, und $R^3$ und $R^4$ gleich oder verschieden sein können oder $R^3$ und $R^4$ zusammen mit dem anliegenden Stickstoff eine heterocyclische Gruppe bilden können;

$alk^1$ und $alk^2$ je eine Niederalkylen- oder Niederalkylenniederalkylgruppe darstellen und gleich oder verschieden sein können, oder eines Salzes oder Hydrates davon für die Herstellung eines Heilmittels für die Behandlung von Arrhythmien.

2. Verbindung der Formel

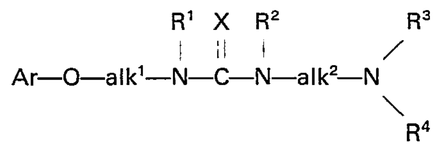

worin

Ar eine 1- oder 2-Naphthyl-, 2,3-Dihydro-1-H-inden-4-(oder 5)-yl oder Phenylgruppe darstellt oder eine substituierte Phenylgruppe darstellt, wobei die substituierte Phenylgruppe durch 1 bis 3 Gruppen substituiert ist, die gleich oder verschieden ausgewählt sein können aus Halogenatomen oder Niederalkyl-, Niederalkoxy-, Trifluormethyl-, Amino-, Nitro-, Niederalkylthio-, Niederalkylsulfinyl-, Niederalkylsulfonyl-, Niederalkanoyl-, Aroylamino- oder Acylaminogruppen;

$R^1$ und $R^2$ je ein Wasserstoffatom oder eine Niederalkyl-, Cycloalkyl-, Phenyl- oder Phenylniederalkylgruppe darstellen;

X ein Sauerstoff- oder ein Schwefelatom darstellt;

$R^3$ und $R^4$ je ein Wasserstoffatom oder eine Niederalkyl-, Phenyl- oder Phenylalkylgruppe, worin Phenyl durch ein Halogenstom oder eine Niederalkyl- oder Niederalkoxygruppe substituiert sein kann, darstellen, und $R^3$ und $R^4$ gleich oder verschieden sein können oder $R^3$ und $R^4$ zusammen mit dem anliegenden Stickstoff eine heterocyclische Gruppe bilden können;

$alk^1$ und $alk^2$ je eine Niederalkylen- oder Niederalkylenniederalkylgruppe darstellen und gleich oder verschieden sein können, anders als

N'-[2-(Diethylamino)-ethyl]-N-methyl-,
N-[2-(Phenoxy)-ethyl]-harnstoff und
N-Methyl-N-[2-(phenoxy)ethyl]-N'-[2-(pyrrolidinyl)-ethyl]-harnstoff,

und die pharmazeutisch annehmbaren Additionssalze oder Hydrate davon.

3. Verbindung nach Anspruch 1 oder 2, worin Ar eine substituierte Phenylgruppe darstellt.

4. Verbindung nach Anspruch 3, worin Ar eine Dichlorphenylgruppe darstellt.

5. Verbindung nach Anspruch 4, worin Ar eine $3,4—Cl_2—C_6H_3$- oder eine $3,5—Cl_2—C_6H_3$-Gruppe darstellt.

6. Verbindung nach einem der Ansprüche 1 bis 4, worin $R^1$ eine $—CH(CH_3)_2$-Gruppe darstellt.

7. Verbindung nach einem der Ansprüche 1 bis 6, worin X ein Schwefelatom darstellt.

8. Verbindung nach Anspruch 3, worin Ar eine 3,5-Dichlorphenylgruppe, $alk^1$ und $alk^2$ je eine $—(CH_2)_2$-Gruppe und $R^3$ und $R^4$ je eine Methylgruppe darstellen.

9. Verbindung nach Anspruch 1 oder 2, welche ist:

N-[2-(3,4-Dichlorphenoxy)ethyl]-N'-[2-(dimethylamino)ethyl]-N-(1-methylethyl)harnstoff oder
N-[2-(3,4-Dichlorphenoxy)ethyl]-N'-[2-(dimethylamino)ethyl]-N-(1-methylethyl)harnstoffmonohydrochloridmonohydrat,
N-[2-(3,4-Dichlorphenoxy)ethyl]-N'-[3-(dimethylamino)propyl]-N-(1-methylethyl)harnstoff oder
N-[2-(3,4-Dichlorphenoxy)ethyl]-N'-[3-(dimethylamino)-propyl]-N-(1-methylethyl)harnstoffoxalat [1:1].

10. Verbindung nach Anspruch 1 oder 2, welche ist:

N-[2-(3,5-Dichlorphenoxy)ethyl]-N'-[2-(dimethylamino)ethyl]-N-(1-methylethyl)harnstoff,
N-[2-(3,5-Dichlorphenoxy)ethyl]-N'-[2-(dimethylamino)ethyl]-N-(1-methylethyl)harnstoffmaleat [1:1],
N-[3-(3,5-Dichlorphenoxy)propyl]-N'-[2-(dimethylamino)ethyl]-N-(1-methylethyl)harnstoff,
N-[3-(3,5-Dichlorphenoxy)propyl]-N'-[2-(dimethylamino)ethyl]-N-(1-methylethyl)harnstoffoxalat [1:1],
N-[3-(3,5-Dichlorphenoxy)propyl]-N'-[3-(dimethylamino)propyl]-N-(1-methylethyl)harnstoff,
N-[3-(3,5-Dichlorphenoxy)propyl]-N'-[3-(dimethylamino)propyl]-N-(1-methylethyl)harnstoffcitrat [1:1],
N-[2-(3,5-Dichlorphenoxy)ethyl]-N'-[3-(dimethylamino)propyl]-N-(1-methylethyl)harnstoff,

N-[2-(3,5-Dichlorphenoxy)ethyl]-N'-[3-(dimethylamino)propyl]-N-(1-methylethyl)harnstoffcitrat [1:1],
N-[2-(3,5-Dichlorphenoxy)ethyl]-N-(1-methylethyl)-N'-[2-[bis-(1-methylethyl)amino]ethyl]harnstoff,
N-[2-(3,5-Dichlorphenoxy)ethyl]-N-(1-methylethyl)-N'-[2-[bis-(1-methylethyl)amino]ethyl]harnstoff-hydrochlorid [1:1],
N-[2-(3,-Dichlorphenoxy)ethyl]-N'-[2-(dimethylamino)ethyl]-N-phenylharnstoff,
N-[2-(3,5-Dichlorphenoxy)ethyl]-N'-[2-(dimethylamino)ethyl]-N-phenylharnstoffmonohydrochlorid,
N-[2-(3,5-Dichlorphenoxy)ethyl]-N'-[2-(dimethylamino)ethyl]-N'-methyl-N-(1-methylethyl)harnstoff,
N-[2-(3,5-Dichlorphenoxy)ethyl]-N'-[2-(dimethylamino)ethyl]-N'-methyl-N-(1-methylethyl)harnstoff-citrat [1:1],
N-[2-(3,5-Dichlorphenoxy)ethyl]-N'-[2-(dimethylamino)ethyl]harnstoff,
N-[2-(3,5-Dichlorphenoxy)ethyl]-N'-[2-(dimethylamino)ethyl]harnstoffumarat [1:1],
N-[2-(3,5-Dichlorphenoxy)ethyl]-N-(1-methylethyl)-N'-[2-(4-morpholinyl)ethyl]harnstoff,
N-[2-(3,5-Dichlorphenoxy)ethyl]-N-(1-methylethyl)-N'-[2-(4-morpholinyl)ethyl]harnstoffumarat [1:1],
N-[2-(3,5-Dichlorphenoxy)ethyl]-N'-[2-(dimethylamino)ethyl]-N-methylharnstoff,
N-[2-(3,5-Dichlorphenoxy)ethyl]-N'-[2-(dimethylamino)ethyl]-N-methylharnstoffmaleat [1:1],
N-[2-(3,5-Dichlorphenoxy)ethyl]-N'-methyl-N'-[2-(methylamin)ethyl]-N-(1-methylethyl)harnstoff,
N-[2-(3,5-Dichlorphenoxy)ethyl]-N'-methyl-N'-[2-(methylamino)ethyl]-N-(1-methylethyl)harnstoff-maleat [1:1],
N-Butyl-N-[2-(3,5-dichlorphenoxy)ethyl]-N'-[2-(dimethylamino)ethyl]harnstoff,
N-Butyl-N-[2-(3,5-dichlorphenoxy)ethyl]-N'-[2-(dimethylamino)ethyl]-harnstoffmaleat [1:1],
N-[2-(3,-Dichlorphenoxy]ethyl]-N'-[2-(dimethylamino)ethyl]-N-propylharnstoff,
N-[2-(3,5-Dichlorphenoxy]ethyl]-N'-[2-(dimethylamino)ethyl]-N-propylharnstoffmaleat [1:1],
N-Cyclohexyl-N-[2-(3,5-dichlorphenoxy)ethyl]-N'-[2-(dimethylamino)ethylharnstoff,
N-Cyclohexyl-N-[2-(3,5-dichlorphenoxy)ethyl]-N'-[2-(dimethylamino)ethyl]harnstoffmaleat [1:1],
N-[2-(3,5-Dichlorphenoxy)ethyl]-N'-[2-(dimethylamino)ethyl]-N-(2-methylpropyl)harnstoff,
N-[2-(3,5-Dichlorphenoxy)ethyl]-N'-[2-dimethylamino)ethyl]-N-(2-methylpropyl)harnstoffmaleat [1:1],
N'-[2-(3,5-Dichlorphenoxy)ethyl]-N-[2-(dimethylamino)ethyl]-N-methylharnstoff,
N'-[2-(3,5-Dichlorphenoxy)ethyl]-N-[2-(dimethylamino)ethyl]-N-methylharnstoffmaleat [1:1],
N-[2-(3,5-Dichlorphenoxy)ethyl]-N-(1-methylethyl)-N'-(2-aminoethyl)harnstoff,
N-[2-(3,5-Dichlorphenoxy)ethyl]-N-(1-methylethyl)-N'-[2-aminoethyl)harnstoffmaleat [1:1],
N-[2-(3,5-Dichlorphenoxy)ethyl]-N'-[2-(diethylamino)ethyl]-N-(1-methylethyl)harnstoff,
N-[2-(3,5-Dichlorphenoxy)ethyl]-N'-[2-(diethylamino)ethyl]-N-(1-methylethyl)harnstofftartrat [1:1],
N-[2-(3,5-Dichlorphenoxy)ethyl]-N'-[2-(dimethylamino)ethyl]-N-(1-methylethyl)harnstoffhydrat [2:1],
N-[2-[(3,5-Dichlorphenyl)oxy]ethyl]-N'-[2-(dimethylamino)ethyl]-N'-(1-methylethyl)harnstoff oder
N-[2-[(3,5-Dichlorphenyl)oxy]ethyl]-N'-[2-(dimethylamino)ethyl]-N'-(1-methylethyl)harnstoffmaleat.

11. Verbindung nach Anspruch 1 oder 2, welche ist:
N-[2-(2,4-Dichlorphenoxy)ethyl]-N'-[2-(dimethylamino)ethyl]-N-(1-methylethyl)harnstoff oder
N-[2-(2,4-Dichlorphenoxy)ethyl]-N'-[2-(dimethylamino)ethyl]-N-(1-methylethyl)harnstoffmaleat [1:1].

12. Verbindung nach Anspruch 1 oder 2, welche ist:
N-[2-(2,6-Dichlorphenoxy)ethyl]-N'-[2-(dimethylamino)ethyl]-N-(1-methylethyl)harnstoff.

13. Verbindung nach Anspruch 1 oder 2, welche ist:
N-[2-(2,5-Dichlorphenoxyethyl]-N'-[2-(dimethylamino)ethyl]-N-(1-methylethyl)harnstoff oder
N-[2-(2,5-Dichlorphenoxyethyl]-N'-[2-(dimethylamino)ethyl]-N-(1-methylethyl)harnstoffmaleat [1:1].

14. Verbindung nach Anspruch 1 oder 2, welche ist:
N-[2-(2,3-Dichlorphenoxy)ethyl]-N'-[2-(dimethylamino)ethyl]-N-(1-methylethyl)harnstoff oder
N-[2-(2,3-Dichlorphenoxy)ethyl]-N'-[-(dimethylamino)ethyl]-N-(1-methylethyl)harnstoffmaleat [1:1].

15. Verbindung nach Anspruch 1 oder 2, welche ist:
N'-[2-(Dimethylamino)ethyl]-N-[2-(3,4-dimethylphenoxy)ethyl]-N-(1-methylethyl)harnstoff,
N'-[2-(dimethylamino)ethyl]-N-[2-(3,4-dimethylphenoxy)ethyl]-N-(1-methylethyl)harnstoffumarat [1:1,5],
N'-[2-(Dimethylamino)ethyl]-N-[2-(3,4-dimethylphenoxy)ethyl]-N-(1-methylethyl)harnstoff oder
N'-[2-(Dimethylamino)ethyl]-N-[2-(3,4-dimethylphenoxy)ethyl]-N-(1-methylethyl)harnstoffumarat [1:1].

16. Verbindung nach Anspruch 1 oder 2, welche ist:
N'-[2-(Dimethylamino)ethyl]-N-[2-(3,5-dimethylphenoxy)ethyl]-N-(1-methylethyl)harnstoff oder
N'-[2-(Dimethylamino)ethyl]-N-[2-(3,5-dimethylphenoxy)ethyl]-N-(1-methylethyl)harnstoffumarat [1:1].

17. Verbindung nach Anspruch 1 oder 2, welche ist:
N-[2-(3,5-Dimethoxyphenoxy)ethyl]-N'-[2-(dimethylamino)ethyl]-N-(1-methylethyl)harnstoff oder
N-[2-(3,5-dimethoxyphenoxy)ethyl]-N'-[2-(dimethylamino)ethyl]-N-(1-methylethyl)harnstoffumarat [1:1].

18. Verbindung nach Anspruch 1 oder 2, welche ist:
N-[2-(3-Chlorphenoxy)ethyl]-N'-[2-(dimethylamino)ethyl]-N-(1-methylethyl)harnstoff,
N-[2-(3-Chlorphenoxy)ethyl]-N'-[2-(dimethylamino)ethyl]-N-(1-methyethyl)harnstoffmaleat [1:1],
N-[2-(3-Chlorphenoxy)ethyl]-N'-[2-(dimethylamino)ethyl]-N-(1-methylethyl)harnstoff,

N-[2-(3-Chlorphenoxy)ethyl]-N'-[2-dimethylamino)ethyl]-N-(1-methylethyl)harnstoffmaleat [1:1],
N-[2-(2-Chlorphenoxy)ethyl]-N'-[2-(dimethylamino)ethyl]-N-(1-methylethyl)harnstoff oder
N-[2-(2-Chlorphenoxy)ethyl]-N'-[2-(dimethylamino)ethyl]-N-(1-methylethyl)harnstoffmaleat [1:1].

19. Verbindung nach Anspruch 1 oder 2, welche ist:
N'-[2-(Dimethylamino)ethyl]-N-(2-(3-fluorphenoxy)ethyl]-N-(1-methylethyl)harnstoff oder
N'-[2-(Dimethylamino)ethyl]-N-[2-(3-fluorphenoxy)ethyl]-N-(1-methylethyl)harnstoffumarat [1:1].

20. Verbindung nach Anspruch 1 oder 2, welche ist:
N'-[2-(Dimethylamino)ethyl]-N-(1-methylethyl)-N-[2-(3-trifluormethylphenoxy)ethyl]harnstoff oder
N'-[2-(Dimethylamino)ethyl]-N-(1-methylethyl)-N-[2-(3-trifluormethylphenoxy)ethyl]harnstoffumarat [1:1].

21. Verbindung nach Anspruch 1 oder 2, welche ist:
N'-[2-(Dimethylamino)ethyl]-N-(1-methylethyl)-N-[2-(1-naphthalinyloxy)ethyl]harnstoff,
N'-[2-(Dimethylamino)ethyl]-N-(1-methylethyl)-N-[2-(1-naphthalinyloxy)ethyl]harnstoffmaleat [1:1],
N'-[2-(Dimethylamino)ethyl]-N-(l-methylethyl)-N-[2-(2-naphthalinyloxy)ethyl]harnstoff oder
N'-[2-(Dimethylamino)ethyl]-N-(1-methylethyl)-N-[2-(2-naphthalinyloxy)ethyl]harnstoffhydrochlorid.

22. Verbindung nach Anspruch 1 oder 2, welche ist:
N'-[2-(Dimethylamino)ethyl]-N-[2-[(2,3-dihydro-1H-inden-5-yl)oxy]-ethyl]-N-(1-methylethyl)harnstoff,
N'-[2-(Dimethylamino)ethyl]-N-[2-[(2,3-dihydro-1H-inden-5-yl)oxy]ethyl]-N-(1-methylethylharnstoff)-maleat [1:1],
N'-[2-(Dimethylamino)ethyl]-N-[2-[(2,3-dihydro-1H-inden-4-yl)oxy]-ethyl]-N-(1-methylethyl)harnstoff,
N'-[2-(Dimethylamino)ethyl]-N-[2-[(2,3-dihydro-1H-inden-4-yl)oxy]ethyl]-N-(1-methylethyl)harnstoff-maleat [1:1],
N'-[2-[(2,3-Dihydro-1H-inden-4-yl)oxy]ethyl]-N-[2-(methylamino)ethyl]-N-methylharnstoff,
N'-[2-[(2,3-Dihydro-1H-inden-4-yl)oxy]ethyl]-N-[2-(methylamino)ethyl]-N-methylharnstoffumarat [1:1],
N-[2-[(2,3-Dihydro-1H-inden-4-yl)oxy]ethyl]-N'-[2-(dimethylamino]ethyl]-N'-(1-methylethyl)harnstoff oder
N-[2-[(2,3-dihydro-1H-inden-4-yl)oxy]ethyl]-N'-[2-(dimethylamino)ethyl]-N'-(1-methylethyl)harnstoff-maleat [1:1].

23. Verbindung nach Anspruch 1 oder 2, welche ist:
N-[2-(Dimethylamino)ethyl]-N'-(1-methylethyl)-N'-(2-phenoxyethyl)harnstoff oder
N-[2-(Dimethylamino)ethyl]-N'-(1-methylethyl)-N'-(2-phenoxyethyl)-harnstoffumarat [1:1].

24. Verbindung nach Anspruch 1 oder 2, welche ist:
N-[2-(3,5-Dichlorphenoxy)ethyl]-N'-[2-(dimethylamino)ethyl]-N-(1-methylethyl)thioharnstoff,
N-[2-(3,5-Dichlorphenoxy)ethyl]-N'-[2-(dimethylamino)ethyl]-N-(1-methylethyl)thioharnstoffumarat-hydrat [2:1],
N-[2-(Diethylamino)ethyl]-N'-(1-methylethyl)-N'-(2-phenoxyethyl)-thioharnstoff,
N-[2-(Diethylamino)ethyl]-N'-(1-methylethyl)-N'-2-phenoxyethyl)-thioharnstoffoxalat [1:1],
N-[2-(Dimethylamino)ethyl]-N-(1-methylethyl)-N-[2-(1-naphthalinyloxy)ethyl]thioharnstoff,
N'-[2-(Dimethylamino)ethyl]-N-(1-methylethyl)-N-[2-(1-nathalinyloxy)ethylthioharnstof maleat [1:1],
N'-[2-(Dimethylamino)ethyl]-N-methyl-N-(2-phenoxyethyl)thioharnstoff oder
N'-[2-(Diethylamino)ethyl]-N-methyl-N-(2-phenoxyethyl)thioharnstoffcitrathemihydrat.

25. Therapeutische Zusammensetzung mit Hemmwirkung gegen Herzarrhythmien, welche umfaßt:
(a) eine wirksame Menge einer Verbindung nach einem der Ansprüche 1 bis 24,
(b) einen pharmazeutischen Trägerstoff hiefür.

26. Kapseln, Tabletten, Ampullen oder Suppositorien, enthaltend:
(a) Verbindungen der Formel

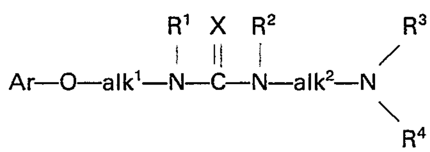

worin:

Ar eine 1- oder 2-Naphthyl-, 2,3-Dihydro-1-H-inden-4-(oder 5)-yl oder Phenylgruppe darstellt oder eine substituierte Phenylgruppe darstellt, wobei die substituierte Phenylgruppe durch 1 bis 3 Gruppen substituiert ist, die gleich oder verschieden ausgewählt sein können aus Halogenatomen oder Niederalkyl-, Niederalkoxy-, Trifluormethyl-, Amino-, Nitro-, Niederalkylthio-, Niederalkylsulfinyl-, Niederalkylsulfonyl-, Niederalkanoyl-, Aroylamino- oder Acylaminogruppen;

$R^1$ und $R^2$ je ein Wasserstoffatom oder eine Niederalkyl-, Cycloalkyl-, Phenyl- oder Phenylniederalkylgruppe darstellen;

X ein Sauerstoff- oder ein Schwefelatom darstellt;

$R^3$ und $R^4$ je ein Wasserstoffatom oder eine Niederalkyl-, Phenyl- oder Phenylalkylgruppe, worin Phenyl durch ein Halogenatom oder eine Niederalkyl- oder Niederalkoxygruppe substituiert sein kann, darstellen,

und $R^3$ und $R^4$ gleich oder verschieden sein könne oder $R^3$ und $R^4$ zusammen mit dem anliegenden Stickstoff eine heterocyclische Gruppe bilden können;

alk[1] und alk[2] je eine Niederalkylen- oder Niederalkylenniederalkylgruppe darstellen und gleich oder verschieden sein könne, oder ein pharmazeutisch annehmbares Additionssalz oder Hydrate davon, und

(b) einen pharmazeutischen Trägerstoff hiefür.

27. Verbindungen der Formel

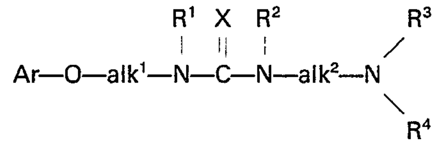

worin:

Ar eine 1- oder 2-Naphthyl-, 2,3-Dihydro-1-H-inden-4-(oder 5)-yl oder Phenylgruppe darstellt oder eine substituierte Phenylgruppe darstellt, wobei die substituierte Phenylgruppe durch 1 bis 3 Gruppen substituiert ist, die gleich oder verschieden ausgewählt sein können aus Halogenatomen oder Niederalkyl-, Niederalkoxy-, Trifluormethyl-, Amino-, Nitro-, Niederalkylthio-, Niederalkylsulfinyl-, Niederalkylsulfonyl-, Niederalkanoyl-, Aroylamino- oder Acylaminogruppen;

$R^1$ und $R^2$ je ein Wasserstoffatom oder eine Niederalkyl-, Cycloalkyl-, Phenyl- oder Phenylniederalkylgruppe darstellen;

X ein Sauerstoff- oder ein Schwefelatom darstellt;

$R^3$ und $R^4$ je ein Wasserstoffatom oder eine Niederalkyl-, Phenyl- oder Phenylalkylgruppe, worin Phenyl durch ein Halogenatom oder eine Niederalkyl- oder Niederalkoxygruppe substituiert sein kann, darstellen, und $R^3$ knd $R^4$ gleich oder verschieden sein können oder $R^3$ und $R^4$ zusammen mit dem anliegenden Stickstoff eine heterocyclische Gruppe bilden können;

alk[1] und alk[2] je eine Niederalkylen- oder Niederalkylenniederalkylgruppe darstellen und gleich oder verschieden sein können, anders als

N'-[2-(Diethylamino)-ethyl]-N-methyl-,
N-[2-(Phenoxy)-ethyl]-harnstoff und
N-Methyl-N-[2-(phenoxy)ethyl]-N'-[2-pyrrolidinyl)ethyl]-harnstoff

und die pharmazeutisch annehmbaren Additionssalze und Hydrate davon für die Verwendung bei der Behandlung von Arrhythmien.

**Patentansprüche für den Vertragsstaat: AT**

1. Verfahren zur Herstellung einer Verbindung der allgemeinen Formel

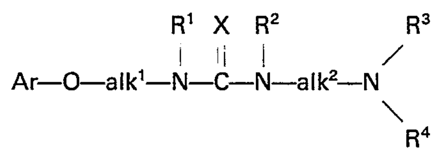

,(I)

worin:

Ar eine 1- oder 2-Naphthyl-, 2,3-Dihydro-1-H-inden-4-(oder 5)-yl oder Phenylgruppe darstellt oder eine substituierte Phenylgruppe darstellt, wobei die substituierte Phenylgruppe durch 1 bis 3 Gruppen substituiert ist, die gleich oder verschieden ausgewählt sein können aus Halogenatomen oder Niederalkyl-, Niederalkoxy-, Trifluormethyl-, Amino-, Nitro-, Niederalkylthio-, Niederalkylsulfinyl-, Niederalkylsulfonyl-, Niederalkanoyl-, Aroylamino- oder Acylaminogruppen;

$R^1$ und $R^2$ je ein Wasserstoffatom oder eine Niederalkyl-, Cycloalkyl-, Phenyl- oder Phenylniederalkylgruppe darstellen;

X ein Sauerstoff- oder ein Schwefelatom darstellt;

$R^3$ und $R^4$ je ein Wasserstoffatom oder eine Niederalkyl-, Phenyl- oder Phenylalkylgruppe, worin Phenyl durch ein Halogenatom oder eine Niederalkyl- oder Niederalkoxygruppe substituiert sein kann, darstellen, und $R^3$ und $R^4$ gleich oder verschieden sein können oder $R^3$ und $R^4$ zusammen mit dem anliegenden Stickstoff eine heterocyclische Gruppe bilden können;

alk[1] und alk[2] je eine Niederalkylen- oder Niederalkylenniederalkylgruppe darstellen und gleich oder verschieden sein könne, anders als

N'-[2-(Diethylamino)ethyl]-N-methyl-,
N-[2-(Phenoxy)-ethyl]-harnstoff und
N-Methyl-N-[2-(phenoxy)-ethyl]-N'-[2-(pyrrolidinyl)ethyl]-harnstoff,

oder eines pharmazeutisch annehmbaren Additionssalzes oder Hydrates davon, gekennzeichnet durch:

(A) Umsetzen einer Verbindung der allgemeinen Formel

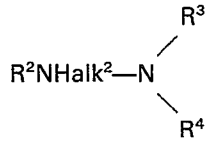

(IIa)

worin $R^2$, $alk^2$, $R^3$ und $R^4$ wie für die Formel (I) definiert sind, mit der Maßgabe, daß dann, wenn $R^2$ von einem Wasserstoffatom verschieden ist, $R^3$ und $R^4$ je von einem Wasserstoffatom verschieden sein müssen, oder $R^2$ gleich ist $R^3$ und $R^4$ ein Wasserstoffatom bedeutet, mit einer Verbindung der allgemeinen Formel

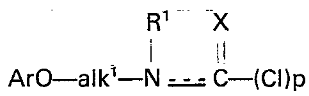

(III)

worin Ar, $alk^1$, $R^1$ und X wie für die allgemeine Formel (I) definiert sind, und wenn $R^1$ ein Wasserstoffatom darstellt, p gleich Null ist und die unterbrochene Linie eine ein Isocyanat bildende Doppelbindung ist; alternativ p gleich 1 ist und die unterbrochene Linie keine Bedeutung hat;

(B)—wenn X ein Sauerstoffatom darstellt und $R^2$ ein Wasserstoffatom darstellt—Umsetzen einer Verbindung der allgemeinen Formel

$$H_2N—alk^2NR^3R^4 \qquad (IIb)$$

worin $alk^2$, $R^3$ und $R^4$ wie für die allgemeine Formel (I) definiert sind, und einer Verbindung der allgemeinen Formel

$$ArO—alk^1—NHR^1 \qquad (IVa)$$

worin Ar, $alk^1$ und $R^1$ wie für die allgemeine Formel (I) definiert sind, mit 1,1'-Carbonyldiimidazol;

(C)—wenn X ein Sauerstoffatom und $R^1$ ein Wasserstoffatom darstellen—Umsetzen einer Verbindung der allgemeinen Formel

$$R^2NH—alk^2—NR^3R^4 \qquad (IIb')$$

worin $R^2$, $alk^2$, $R^3$ und $R^4$ wie für die allgemeine Formel (I) definiert sind, und einer Verbindung der allgemeinen Formel

$$Ar—O—alk^1—NH_2 \qquad (IVb)$$

worin Ar und $alk^1$ wie für die allgemeine Formel (I) definiert sind, mit 1,1'-Carbonyldiimidazol; oder

(D) Umsetzen einer Verbindung der allgemeinen Formel

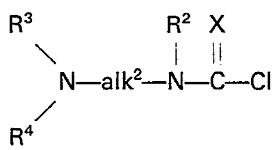

(V)

worin X, $R^2$, $alk^2$, $R^3$ und $R^4$ wie für die allgemeine Formel (I) definiert sind, mit der Ausnahme, daß $R^2$, $R^3$ und $R^4$ immer von einem Wasserstoffatom verschieden sind, und eine Verbindung der allgemeinen Formel

$$Ar—O—alk^1—NHR^1 \qquad (IVa)$$

worin Ar, $alk^1$ und $R^1$ wie für die obige allgemeine Formel (I) definiert sind, mit Triethylamin, und gewünschtenfalls nachfolgendes Bilden eines pharmazeutisch annehmbaren Additionssalzes oder Hydrates einer so gebildeten Verbindung.

2. Verfahren nach Anspruch 1, worin Ar eine substituierte Phenylgruppe darstellt.

3. Verfahren nach Anspruch 2, worin Ar eine Dichlorphenylgruppe darstellt.

4. Verfahren nach Anspruch 3, worin Ar eine 3,4—$Cl_2$—$C_6H_3$- oder eine 3,5—$Cl_2$—$C_6H_3$-Gruppe darstellt.

5. Verfahren nach Anspruch 1, 2 oder 3, worin $R^1$ eine —$CH(CH_3)_2$-Gruppe darstellt.

6. Verfahren nach einem der Ansprüche 1 bis 5, worin X ein Schwefelatom darstellt.

7. Verfahren nach Anspruch 2, worin Ar eine 3,5-Dichlorphenylgruppe, $alk^1$ und $alk^2$ je eine —$(CH_2)_2$-Gruppe und $R^3$ und $R^4$ je eine Methylgruppe darstellen.

8. Verfahren nach Anspruch 1 zur Herstellung von:

N-[2-(3,4-Dichlorphenoxy)ethyl]-N'-[2-(dimethylamino)ethyl]-N-(1-methylethyl)harnstoff oder

N-[2-(3,4-Dichlorphenoxy)ethyl]-N'-[2-(dimethylamino)ethyl]-N-(1-methylethyl)harnstoffmonohydro-chloridmonohydrat,
N-[2-(3,4-Dichlorphenoxy)ethyl]-N'-[3-(dimethylamino)propyl]-N-(1-methylethyl)harnstoff oder
N-[2-(3,4-Dichlorphenoxy)ethyl]-N'-[3-(dimethylamino)-propyl]-N-(1-methylethyl)harnstoffoxalat [1:1].

9. Verfahren nach Anspruch 1 zur Herstellung von:
N-[2-(3,5-Dichlorphenoxy)ethyl]-N'-[2-(dimethylamino)ethyl]-N-(1-methylethyl)harnstoff,
N-[2-(3,5-Dichlorphenoxy)ethyl]-N'-[2-(dimethylamino)ethyl]-N-(1-methylethyl)harnstoffmaleat [1:1],
N-[-(3,5-Dichlorphenoxy)propyl]-N'-[2-(dimethylamino)ethyl]-N-(1-methylethyl)harnstoff,
N-[3-(3,5-Dichlorphenoxy)propyl]-N'-[2-(dimethylamino)ethyl]-N-(1-methylethyl)harnstoffoxalat [1:1],
N-[3-(3,5-Dichlorphenoxy)propyl]-N'-[3-(dimethylamino)propyl]-N-(1-methylethyl)harnstoff,
N-[3-(3,5-Dichlorphenoxy)propyl]-N'-[3-(dimethylamino)propyl]-N-(1-methylethylharnstoffcitrat [1:1],
N-[2-(3,5-Dichlorphenoxy)ethyl]-N'-[3-(dimethylamino)propyl]-N-(1-methylethyl)harnstoff,
N-[2-(3,5-Dichlorphenoxy)ethyl]-N'-[3-(dimethylamino)propyl]-N-(1-methylethyl)harnstoffcitrat [1:1],
N-[2-(3,5-Dichlorphenoxy)ethyl]-N-(1-methylethyl)-N'-[2-[bis-(1-methylethyl)amino]ethyl]harnstoff,
N-[2-(3,5-Dichlorphenoxy)ethyl]-N-(1-methylethyl)-N'-[2-[bis-(1-methylethyl)amino]ethyl]harnstoff-hydrochlorid [1:1],
N-[2-(3,5-Dichlorphenoxy)ethyl]-N'-[2-(dimethylamino)ethyl]-N-phenylharnstoff,
N-[2-(3,5-Dichlorphenoxy)ethyl]-N'-[2-(dimethylamino)ethyl]-N-phenylharnstoffmonohydrochlorid,
N-[2-(3,5-Dichlorphenoxy)ethyl]-N'-[2-(dimethylamino)ethyl]-N'-methyl-N-(1-methylethyl)harnstoff,
N-[2-(3,5-Dichlorphenoxy)ethyl]-N'-[2-(dimethylamino)ethyl]-N'-methyl-N-(1-methylethyl)harnstoff-citrat [1:1],
N-[2-(3,5-Dichlorphenoxy)ethyl]-N'-[2-(dimethylamino)ethyl]harnstoff,
N-[2-(3,5-Dichlorphenoxy)ethyl]-N'-[2-(dimethylamino)ethylharnstoffumarat [1:1],
N-[2-(3,5-Dichlorphenoxy)ethyl]-N-(1-methylethyl)-N'-[2-4-morpholinyl)ethyl]harnstoff,
N-[2-(3,5-Dichlorphenoxy)ethyl]-N-(1-methylethyl)-N'-[2-(4-morpholinyl)ethyl]harnstoffumarat [1:1],
N-[2-(3,5-Dichlorphenoxy)ethyl]-N'-[2-(dimethylamino)ethy]-N-methylharnstoff,
N-[2-(3,5-Dichlorphenoxy)ethyl]-N'-[2-(dimethylamino)ethyl]-N-methylharnstoffmaleat [1:1],
N-[2-(3,5-Dichlorphenoxy)ethyl]-N'-methyl-N'-[2-(methylamino)-ethyl]-N-(1-methylethyl)harnstoff,
N-[2-(3,5-Dichlorphenoxy)ethyl]-N'-methyl-N'-[2-(methylamino)ethyl]-N-(1-methylethyl)harnstoff-maleat [1:1],
N-Butyl-N-[2-(3,5-dichlorphenoxy)ethyl]-N'-[2-(dimethylamino)ethyl]harnstoff,
N-Butyl-N-[2-(3,5-dichlorphenoxy)ethyl]-N'-[2-(dimethylamino)ethyl]-harnstoffmaleat [1:1],
N-[2-(3,5-Dichlorphenoxy)ethyl]-N'-[2-(dimethylamino)ethyl]-N-propylharnstoff,
N-[2-(3,5-Dichlorphenoxy)ethyl]-N'-[2-(dimethylamino)ethyl]-N-propylharnstoffmaleat [1:1],
N-Cyclohexyl-N-[2-(3,5-dichlorphenoxy)ethyl]-N'-[2-(dimethylamino)ethyl]harnstoff,
N-Cyclohexyl-N-[2-(3,-dichlorphenoxy)ethyl]-N'-[2-(dimethylamino)ethyl]harnstoffmaleat [1:1],
N-[2-(3,5-Dichlorphenoxy)ethyl]-N'-[2-(dimethylamino)ethyl]-N-(2-methylpropyl)harnstoff,
N-[2-(3,5-Dichlorphenoxy)ethyl]-N'-[2-dimethylamino)ethyl]-N-(2-methylpropyl)harnstoffmaleat [1:1],
N'-[2-(3,5-Dichlorphenoxy)ethyl]-N-[2-(dimethylamino)ethyl]-N-methylharnstoff,
N'-[2-(3,5-Dichlorphenoxy)ethyl]-N-[2-(dimethylamino)ethyl]-N-methylharnstoffmaleat [1:1],
N-[2-(3,5-Dichlorphenoxy)ethyl]-N-(1-methylethyl-N'-(2-aminoethyl)harnstoff,
N-[2-(3,5-Dichlorphenoxy)ethyl]-N-(1-methylethyl)-N'-[2-aminoethyl)harnstoffmaleat [1:1]
N-[2-(3,5-Dichlorphenoxy)ethyl]-N'-[2-(diethylamino)ethyl]-N-(1-methylethyl)harnstoff,
N-[2-(3,5-Dichlorphenoxy)ethyl]-N'-[2-(diethylamino)ethyl]-N-(1-methylethyl)harnstofftartrat [1:1],
N-[2-(3,5-Dichlorphenoxy)ethyl]-N'-[2-(dimethylamino)ethyl]-N-(1-methylethyl)harnstoffhydrat [2:1],
N-[2-[(3,5-Dichlorphenyl)oxy]ethyl]-N'-[2-(dimethylamino)ethyl]-N'-(1-methylethyl)harnstoff oder
N-[2-[(3,5-Dichlorphenyl)oxy]ethyl]-N'-[2-(dimethylamino)ethyl]-N'-(1-methylethyl)harnstoffmaleat.

10. Verfahren nach Anspruch 1 zur Herstellung von:
N-[2-(2,4-Dichlorphenoxy)ethyl]-N'-[2-(dimethylamino)ethyl]-N-(1-methylethyl)harnstoff oder
N-[2-(2,4-Dichlorphenoxy)ethyl]-N'-[2-(dimethylamino)ethyl]-N-(1-methylethyl)harnstoffmaleat [1:1].

11. Verfahren nach Anspruch 1 zur Herstellung von:
N-[2-(2,1-Dichlorphenoxy)ethyl]-N'-[2-(dimethylamino)ethyl]-N-(1-methylethyl)harnstoff.

12. Verfahren nach Anspruch 1 zur Herstellung von:
N-[(2,5-Dichlorphenoxyethyl]-N'-[2-(dimethylamino)ethyl]-N-(1-methylethyl)harnstoff oder
N-[2-(2,5-Dichlorphenoxyethyl]-N'-[2-(dimethylamino)ethyl]-N-(1-methylethyl)harnstoffmaleat [1:1].

13. Verfahren nach Anspruch 1 zur Herstellung von:
N-[2-(2,3-Dichlorphenoxy)ethyl]-N'-[2-(dimethylamino)ethyl]-N-(1-methylethyl)harnstoff oder
N-[2-(2,3-Dichlorphenoxy)ethyl]-N'-[2-(dimethylamino)ethyl]-N-(1-methylethyl)harnstoffmaleat [1:1].

14. Verfahren nach Anspruch 1 zur Herstellung von:
N-[2-(Dimethylamino)ethyl]-N-[2-(3,4-dimethylphenoxy)ethyl]-N-(1-methylethyl)harnstoff,
N'-[2-(Dimethylamino)ethyl]-N-[2-(3,4-dimethylphenoxy)ethyl]-N-(1-methylethyl)harnstoffumarat [1:1,5],
N'-[2-(Dimethylamino)ethyl]-N-[2-(3,4-dimethylphenoxy)ethyl]-N-(1-methylethyl)harnstoff oder
N'-[2-(Dimethylamino)ethyl]-N-[2-(3,4-dimethylphenoxy)ethyl]-N-(1-methylethyl)harnstoffumarat [1:1].

15. Verfahren nach Anspruch 1 zur Herstellung von:
N'-[2-(Dimethylamino)ethyl]-N-[2-(3,5-dimethylphenoxy)ethyl]-N-(1-methylethyl)harnstoff oder
N'-[2-(Dimethylamino)ethyl]-N-[2-(3,5-dimethylphenoxy)ethyl]-N-(1-methylethyl)harnstoffumarat [1:1].

16. Verfahren nach Anspruch 1 zur Herstellung von:
N-[2-(3,5-Dimethoxyphenoxy)ethyl]-N'-[2-(dimethylamino)ethyl]-N-(1-methylethyl)harnstoff oder
N-[2-(3,5-Dimethoxyphenoxy)ethyl]-N'-[2-(dimethylamino)ethyl]-N-(1-methylethyl)harnstoffumarat [1:1].

17. Verfahren nach Anspruch 1 zur Herstellung von:
N-[2-(3-Chlorphenoxy)ethyl]-N'-[2-(dimethylamino)ethyl]-N-(1-methylethyl)harnstoff,
N-[2-(3-Chlorphenoxy)ethyl]-N'-[2-(dimethylamino)ethyl]-N-(1-methylethyl)harnstoffmaleat [1:1],
N-[2-(3-Chlorphenoxy)ethyl]-N'-[2-(dimethylamino)ethyl]-N-(1-methylethyl)harnstoff,
N-[2-(3-Chlorphenoxy)ethyl]-N'-[2-dimethylamino)ethyl]-N-(1-methylethyl)harnstoffmaleat [1:1],
N-[2-(2-Chlorphenoxy)ethyl]-N'-[2-(dimethylamino)ethyl]-N-(1-methylethyl)harnstoff oder
N-[2-(2-Chlorphenoxy)ethyl]-N'-[2-(dimethylamino)ethyl]-N-(1-methylethyl)harnstoffmaleat [1:1].

18. Verfahren nach Anspruch 1 zur Herstellung von:
N-[2-(Dimethylamino)ethyl]-N-[2-(3-fluorphenoxy)ethyl]-N-(1-methylethyl)harnstoff oder
N'-[2-(Dimethylamino)ethyl]-N-[2-(3-fluorphenoxy)ethyl]-N-(1-methylethyl)harnstoffumarat [1:1].

19. Verfahren nach Anspruch 1 zur Herstellung von:
N'-[2-(Dimethylamino)ethyl]-N-(1-methylethyl)-N-[2-(3-trifluormethylphenoxy)ethyl]harnstoff oder
N'-[2-(Dimethylamino)ethyl]-N-(1-methylethyl)-N-[2-(3-trifluormethylphenoxy)ethyl]harnstoffumarat [1:1].

20. Verfahren nach Anspruch 1 zur Herstellung von:
N-[2-(Dimethylamino)ethyl]-N-(1-methylethyl)-N-[2-(1-naphthalinyloxy)ethylharnstoff,
N'-[2-(Dimethylamino)ethyl]-N-(1-methylethyl)-N-[2-(1-naphthalinyloxy)ethyl]harnstoffmaleat [1:1],
N'-[2-(Dimethylamino)ethyl]-N-(1-methylethyl)-N-[2-(2-naphthalinyloxy)ethylharnstoff oder
N'-[2-(Dimethylamino)ethyl]-N-(1-methylethyl)-N-[2-(2-naphthalinyloxy)ethyl]harnstoffhydrochlorid.

21. Verfahren nach Anspruch 1 zur Herstellung von:
N'-[2-(Dimethylamino)ethyl]-N-[2-[(2,3-dihydro-1H-inden-5-yl)oxy]-ethyl]-N-(1-methylethyl)harnstoff,
N'-[2-(Dimethylamino)ethyl]-N-[2-[(2,3-dihydro-1H-inden-5-yl)oxy]-ethyl]-N-(1-methylethylharnstoff)-maleat [1:1],
N'-[2-(Dimethylamino)ethyl]-N-[2-[(2,3-dihydro-1H-inden-4-yl)oxy]-ethyl]-N-(1-methylethyl)harnstoff,
N'-[2-(Dimethylamino)ethyl]-N-[2-[(2,3-dihydro-1H-inden-4-yl)oxy]-ethyl]-N-(1-methylethyl)harnstoff-maleat [1:1],
N'-[2-[(2,3-Dihydro-1H-inden-4-yl)oxy]ethyl]-N-[2-(methylamino)-ethyl]-N-methylharnstoff,
N'-[2-[(2,3-Dihydro-1H-inden-4-yl)oxy]ethyl]-N-[2-(methylamino)-ethyl]-N-methylharnstoffumarat [1:1],
N-[2-[(2,3-Dihydro-1H-inden-4-yl)oxy]ethyl]-N'-[2-(dimethylamino)-ethyl]-N'-(1-methylethyl)harnstoff oder
N-[2-[2,3-Dihydro-1H-inden-4-yl)oxy]ethyl]-N'-[2-(dimethylamino)-ethyl]-N'-(1-methylethyl)harnstoff-maleat [1:1].

22. Verfahren nach Anspruch 1 zur Herstellung von:
N-[2-(Dimethylamino)ethyl]-N'-(1-methylethyl)-N'-(2-phenoxyethyl)harnstoff oder
N-[2-(Dimethylamino)ethyl]-N'-(1-methylethyl)-N'-(2-phenoxyethyl)-harnstoffumarat [1:1].

23. Verfahren nach Anspruch 1 zur Herstellung von:
N-[2-(3,5-Dichlorphenoxy)ethyl]-N'-[2-(dimethylamino)ethyl]-N-(1-methylethyl)thioharnstoff,
N-[2-(3,5-Dichlorphenoxy)ethyl]-N'-[2-(dimethylamino)ethyl]-N-(1-methylethyl)thioharnstoffumarat-hydrat [2:1],
N-[2-(Diethylamino)ethyl]-N'-(1-methylethyl)-N'-(2-phenoxyethyl)-thioharnstoff,
N-[2-(Diethylamino)ethyl]-N'-(1-methylethyl)-N'-2-phenoxyethyl)-thioharnstoffoxalat [1:1],
N-[2-(Dimethylamino)ethyl]-N-(1-methylethyl)-N-[2-(1-naphthalinyl-oxy)ethyl]thioharnstoff,
N'-[2-(Dimethylamino)ethyl]-N-(1-methylethyl)-N-[2-(1-naphtalinyloxy]ethyl thioharnstoffmaleat [1:1],
N'-[2-(Dimethylamino)ethyl]-N-methyl-N-(2-phenoxyethyl)thioharnstoff oder
N'-[2-(Diethylamino)ethyl]-N-methyl-N-(2-phenoxyethyl)thioharnstoffcitrat hemihydrat.

24. Verfahren zur Herstellung einer therapeutischen Zusammensetzung mit Hemmwirkung gegen Herzarrythmien, umfassend das Mischen:

(a) einer wirksamen Menge einer Verbindung der Formel

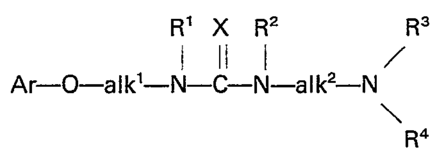

worin:

Ar eine 1- oder 2-Naphthyl-, 2,3-Dihydro-1-H-inden-4-(oder 5)-yl oder Phenylgruppe darstellt oder eine

substituierte Phenylgruppe darstellt, wobei die substituierte Phenylgruppe durch 1 bis 3 Gruppen substituiert ist, die gleich oder verschieden ausgewählt sein können aus Halogenatomen oder Niederalkyl-, Niederalkoxy-, Trifluormethyl-, Amino-, Nitro-, Niederalkylthio-, Niederalkylsulfinyl-, Niederalkylsulfonyl-, Niederalkanoyl-, Aroylamino- oder Acylaminogruppen;

$R^1$ und $R^2$ je ein Wasserstoffatom oder eine Niederalkyl-, Cycloalkyl-, Phenyl- oder Phenylniederalkylgruppe darstellen;

X ein Sauerstoff- oder ein Schwefelatom darstellt;

$R^3$ und $R^4$ je ein Wasserstoffatom oder eine Niederalkyl-, Phenyl- oder Phenylalkylgruppe, worin Phenyl durch ein Halogenatom oder eine Niederalkyl- oder Niederalkoxygruppe substutiert sein kann, darstellen, und $R^3$ und $R^4$ gleich oder verschieden sein können oder $R^3$ und $R^4$ zusammen mit dem anliegenden Stickstoff eine heterocyclische Gruppe bilden können;

alk[1] und alk[2] je eine Niederalkylen- oder Niederalkylenniederalkylgruppe darstellen und gleich oder verschieden sein können, oder eines Salzes oder Hydrates davon, und

(b) eines pharmazeutisch annehmbaren Trägerstoffes hiefür.

25. Verfahren zur Herstellung einer therapeutischen Zusammensetzung mit Hemmwirkung gegen Herzarrythmien, umfassend das Mischen einer nach einem Verfahren nach einem der Ansprüche 1 bis 24 hergestellten Verbindung mit einem pharmazeutisch annehmbaren Trägerstoff hiefür.

26. Verfahren zur Herstellung von Kapseln, Tabletten, Ampullen oder Suppositorien mit Hemmwirkung gegen Herzarryhthmie, umfassend das Mischen:

(a) von Verbindungen der Formel

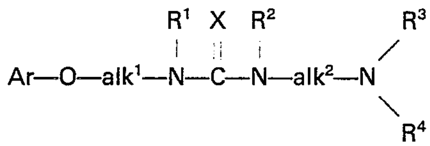

worin:

Ar eine 1- oder 2-Naphthyl-, 2,3-Dihydro-1-H-inden-4-(oder 5)-yl oder Phenylgruppe darstellt oder eine substituierte Phenylgruppe darstellt, wobei die substituierte Phenylgruppe durch 1 bis 3 Gruppen substituiert ist, die gleich oder verschieden ausgewählt sein können aus Halogenatomen oder Niederalkyl-, Niederalkoxy-, Trifluormethyl-, Amino-, Nitro-, Niederalkylthio-, Niederalkylsulfinyl-, Niederalkylsulfonyl-, Niederalkanoyl-, Aroylamino- oder Acylaminogruppen;

$R^1$ und $R^2$ je ein Wasserstoffatom oder eine Niederalkyl-, Cycloalkyl-, Phenyl- oder Phenylniederalkylgruppe darstellen;

X ein Sauerstoff- oder ein Schwefelatom darstellt;

$R^3$ und $R^4$ je ein Wasserstoffatom oder eine Niederalkyl-, Phenyl- oder Phenylalkylgruppe, worin Phenyl durch ein Halogenatom oder eine Niederalkyl- oder Niederalkoxygruppe substituiert sein kann, darstellen, und $R^3$ und $R^4$ gleich oder verschieden sein können oder $R^3$ und $R^4$ zusammen mit dem anliegenden Stickstoff eine heterocyclische Gruppe bilden können;

alk[1] und alk[2] je eine Niederalkylen- oder Niederalkylenniederalkylgruppe darstellen und gleich oder verschieden sein können, oder eines pharmazeutisch annehmbaren Additionssalzes oder Hydrates davon und

(b) einem pharmazeutischen Trägerstoff hiefür; und das nachfolgende Herstellen von Kapsenl, Tabletten, Ampullen oder Suppositorien aus dem Gemisch.

**Revendications pour les Etats Contractants: BE, CH, DE, FR, GB, IT, LI, LU, NL, SE**

1. Utilisation d'un composé de formule:

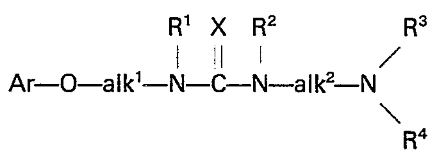

où Ar représente un radical 1- ou 2-naphtyle, 2,3-dihydro-1-H-indén-4(ou 5)yle, phényle ou phényle substitué, ce radical phényle substitué étant substitué par 1—3 radicaux qui peuvent être identiques ou différents et sont choisis parmi les atomes d'halogène et radicaux alkyle inférieures, alkoxy inférieurs, trifluorométhyle, amino, nitro, alkylthio inférieurs, alkylsulfinyle inférieurs, alkylsulfonyle inférieurs, alkanoyle inférieurs, aroylamino et acylamino;

$R^1$ et $R^2$ représentent chacun un atome d'hydrogène ou un radical alkyle inférieur, cycloalkyle, phényle ou phényl-alkyle inférieur;

X représente un atome d'oxygène ou un atome de soufre;

$R^3$ et $R^4$ représentent chacun un atome d'hydrogène ou un radical alkyle inférieur, phényle ou

phénylalkyle où le radical phényle peut être substitué par un atome d'halogène ou un radical alkyle inférieur ou alkoxy inférieur et $R^3$ et $R^4$ peuvent être identiques ou différents ou bien $R^3$ et $R^4$ pris ensemble avec l'atome d'azote adjacent peuvent former un reste hétérocyclique;

alk$^1$ et alk$^2$ représentent chacun un radical alkylène inférieur ou alkylène inférieur-alkyle inférieur et peuvent être identiques ou différents, ou d'un sel ou hydrate de ce composé dans la préparation d'un médicament pour le traitement d'arythmies.

2. Composés de formule

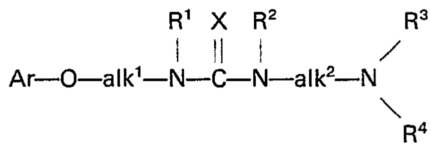

où Ar représente un radical 1- ou 2-naphtyle, 2,3-dihydro-1-H-indén-4(ou 5)yle, phényle ou phényle substitué, ce radical phényle substitué étant substitué par 1—3 radicaux qui peuvent être identiques ou différents et sont choisis parmi les atomes d'halogène et radicaux alkyle inférieurs, alkoxy inférieurs, trifluorométhyle, amino, nitro, alkylthio inférieurs, alkylsulfinyle inférieurs, alkylsulfonyle inférieurs, alkanoyle inférieurs, aroylamino et acylamino;

$R^1$ et $R^2$ représentent chacun un atome d'hydrogène ou un radical alkyle inférieur, cycloalkyle, phényle ou phényl-alkyle inférieur;

X représente un atome d'oxygène ou un atome de soufre;

$R^3$ et $R^4$ représentent chacun un atome d'hydrogène ou un radical alkyle inférieur, phényle ou phénylalkyle où le radical phényle peut être substitué par un atome d'halogène ou un radical alkyle inférieur ou alkoxy inférieur et $R^3$ et $R^4$ peuvent être identiques ou différents ou bien $R^3$ et $R^4$ pris ensemble avec l'atome d'azote adjacent peuvent former un reste hétérocyclique;

alk$^1$ et alk$^2$ représentent chacun un radical alkylène inférieur ou alkylène inférieur-alkyle inférieur et peuvent être identiques ou différents, autres que la

N'-[2-(diéthylamino)éthyl]-N-méthyl-N-[2-(phénoxy)éthyl]urée et la

N-méthyl-N-[2-(phénoxy)éthyl]-N'-[2-(pyrrolidinyl)éthyl]urée,

et leurs sels d'addition pharmaceutiquement acceptables et hydrates.

3. Composé suivant la revendication 1 ou 2, dans lequel Ar représente un radical phényle substitué.

4. Composé suivant la revendication 3, dans lequel Ar représente un radical dichlorophényle.

5. Composé suivant la revendication 4, dans lequel Ar représente un radical $3,4—Cl_2—C_6H_3—$ ou $3,5—Cl_2C_6h_3—$.

6. Composé suivant l'une quelconque des revendications 1 à 4, dans lequel $R^1$ représente un radical $—CH(CH_3)_2$.

7. Composé suivant l'une quelconque des revendications 1 à 6, dans lequel X représente un atome de soufre.

8. Composé suivant la revendication 3, dans lequel Ar représente un radical 3,5-dichlorophényle, alk$^1$ et alk$^2$ représentent tous deux un radical $—(CH_2)_2—$ et $R^3$ et $R^4$ représentent tous deux un radical méthyle.

9. Composé suivant la revendication 1 ou 2, qui est

la N-[2-(3,4-dichlorophénoxy)éthyl]-N'-[2-(diméthylamino)éthyl]-N-(1-méthyléthyl)urée ou

le monochlorhydrate de N-[2-(3,4-dichlorophénoxy)éthyl]-N'-[2-(diméthylamino)éthyl]-N-(1-méthyléthyl)urée monohydraté,

la N-[2-(3,4-dichlorophénoxy)éthyl]-N'-[3-(diméthylamino)propyl]-N-(1-méthyléthyl)urée ou

le [1:1] oxalate de N-[2-(3,4-dichlorophénoxy)éthyl]-N'-[3-(diméthylamino)propyl]-N-(1-méthyléthyl)-urée.

10. Composé suivant la revendication 1 ou 2 qui est

la N-[2-(3,5-dichlorophénoxy)éthyl]-N']-[2-(diméthylamino)éthyl]-N-(1-méthyl)urée,

le [1:1] maléate de N-[2-(3,5-dichlorophénoxy)éthyl]-N'-[2-(diméthylamino)éthyl]-N-(1-méthyléthyl)-urée,

la N-[3-(3,5-dichlorophénoxy)propyl]-N'-[2-(diméthylamino)éthyl]-N-(1-méthyléthyl)urée,

le [1:1] oxalate de N-[3-(3,5-dichlorophénoxy)propyl]-N'-[2-(diméthylamino)éthyl]-N-(1-méthyléthyl)-urée,

la N-[3-(3,5-dichlorophénoxy)propyl]-N'-[3-(diméthylamino)propyl]-N-(1-méthyléthyl)urée,

le [1:1] citrate de N-[3-(3,5-dichlorophénoxy)propyl]-N'-[3-(diméthylamino)propyl]-N-(1-méthyléthyl)-urée,

la N-[2-(3,5-dichlorophénoxy)éthyl]-N'-[3-(diméthylamino)propyl]-N-(1-méthyléthyl)urée,

le [1:1] citrate de N-[2-(3,5-dichlorophénoxy)éthyl]-N'-[3-(diméthylamino)propyl]-N-(1-méthyléthyl)-urée,

la N-[2-(3,5-dichlorophénoxy)éthyl]-N-(1-méthyléthyl)-N'-[2-[bis(1-méthyléthyl)amino]éthyl]urée,

le [1:1] chlorhydrate de N-[2-(3,5-dichlorophénoxy)éthyl]-N-(1-méthyléthyl)-N'-[2-[bis(1-méthyléthyl)-amino]éthyl]urée,

la N-[2-(3,5-dichlorophénoxy)éthyl]-N'-[2-(diméthylamino)éthyl]-N-phénylurée,

le monochlorhydrate de N-[2-(3,5-dichlorophénoxy)éthyl]-N'-[2-(diméthylamino)éthyl]-N-phénylurée,
la N-[2-(3,5-dichlorophénoxy)éthyl]-N'-[2-(diméthylamino)éthyl]-N'-méthyl-N-(1-méthyléthyl)urée,
le [1:1] citrate de N-[2-(3,5-dichlorophénoxy)éthyl]-N'-[2-(diméthylamino)éthyl]-N'-méthyl-N-(1-méthyléthyl)urée,
la N-[2-(3,5-dichlorophénoxy)éthyl]-N'-[2-(diméthylamino)éthyl]urée,
le [1:1] fumarate de N-[2-(3,5-dichlorophénoxy)éthyl]-N'-[2-(diméthylamino)éthyl]urée,
la N-[2-(3,5-dichlorophénoxy)éthyl]-N-(1-méthyléthyl)-N'-[2-(4-morpholinyl)éthyl]urée,
le [1:1] fumarate de N-[2-(3,5-dichlorophénoxy)éthyl]-N-(1-méthyléthyl)-N'-[2-(4-morpholinyl)éthyl]-urée,
la N-[2-(3,5-dichlorophénoxy)éthyl]-N'-[2-(diméthylamino)éthyl]-N-méthylurée,
le [1:1] maléate de N-[2-(3,5-dichlorophénoxy)éthyl]-N'-[2-(diméthylamino)éthyl]-N-méthylurée,
la N-[2-(3,5-dichlorophénoxy)éthyl]-N'-méthyl-N'-[2-(méthylamino)éthyl]-N-(1-méthyléthyl)urée,
le [1:1] maléate de N-[2-(3,5-dichlorophénoxy)éthyl]-N'-[2-(méthylamino)éthyl]-N-(1-méthyléthyl)urée,
la N-butyl-N-[2-(3,5-dichlorophénoxy)éthyl]-N'-[2-(diméthylamino)éthyl]urée,
le [1:1] maléate N-butyl-N-[2-(3,5-dichlorophénoxy)éthyl]-N'-[2-(diméthylamino)éthyl]urée,
la N-[2-(3,5-dichlorophénoxy)éthyl]-N'-[2-(diméthylamino)éthyl]-N-propylurée,
le [1:1] maléate de N-[2-(3,5-dichlorophénoxy)éthyl]-N'-[2-(diméthylamino)éthyl]-N-propylurée,
la N-cyclohexyl-N-[2-(3,5-dichlorophénoxy)éthyl]-N'-[2-(diméthylamino)éthyl]urée,
le [1:1] maléate de N-cyclohexyl-N-[2-(3,5-dichlorophénoxy)éthyl]-N'-[2-(diméthylamino)éthyl]urée,
la N-[2-(3,5-dichlorophénoxy)éthyl]-N'-[2-(diméthylamino)éthyl]-N-(2-méthylpropyl)urée,
le [1:1] maléate de N-[2-(3,5-dichlorophénoxy)éthyl]-N'-[2-(diméthylamino)éthyl]-N-(2-méthylpropyl)-urée,
la N'-[2-(3,5-dichlorophénoxy)éthyl]-N-[2-(diméthylamino)éthyl]-N-méthylurée,
le [1:1] maléate de N'-[2-(3,5-dichlorophénoxy)éthyl]-N-[2-(diméthylamino)éthyl]-N-méthylurée,
la N-[2-(3,5-dichlorophénoxy)éthyl]-N-(1-méthyléthyl)-N'-(2-aminoéthyl)urée,
le [1:1] maléate de N-[2-(3,5-dichlorophénoxy)éthyl]-N-(1-méthyléthyl)-N'-(2-aminoéthyl)urée,
la N-[2-(3,5-dichlorophénoxy)éthyl]-N'-[2-(diéthylamino)éthyl]-N-(1-méthyléthyl)urée,
le [1:1] tartrate de N-[2-(3,5-dichlorophénoxy)éthyl]-N'-[2-(diéthylamino)éthyl]-N-(1-méthyléthyl)urée,
la N-[2-(3,5-dichlorophénoxy)éthyl]-N'-[2-(diméthylamino)éthyl]-N-(1-méthyléthyl)urée hydratée [2:1],
la N-[2-[(3,5-dichlorophényl)oxy]-éthyl]-N'-[2-(diméthylamino)éthyl]-N'-(1-méthyléthyl)urée ou
le maléate de N-[2-[(3,5-dichlorophényl)oxy]éthyl]-N'-[2-(diméthylamino)éthyl]-N'-(1-méthyléthyl)-urée.

11. Composé suivant la revendication 1 ou 2 qui est
la N-[2-(2,4-dichlorophénoxy)éthyl]-N'-[2-(diméthylamino)éthyl]-N-(1-méthyléthyl)urée ou
le [1:1] maléate de N-[2-(2,4-dichlorophénoxy)éthyl]-N'-[2-(diméthylamino)éthyl]-N-(1-méthyléthyl)-urée.

12. Composé suivant la revendication 1 ou 2, qui est
la N-[2-(2,6-dichlorophénoxy)éthyl]-N'-[2-(diméthylamino)éthyl]-N-(1-méthyléthyl)urée.

13. Composé suivant la revendication 1 ou 2, qui est
la N-[2-(2,5-dichlorophénoxy)éthyl]-N'-[2-(diméthylamino)éthyl]-N-(1-méthyléthyl)urée ou
le [1:1] maléate de N-[2-(2,5-dichlorophénoxy)éthyl]-N'-[2-(diméthylamino)éthyl]-N-(1-méthyléthyl)-urée.

14. Composé suivant la revendication 1 ou 2, qui est
la N-[2-(2,3-dichlorophénoxy)éthyl]-N'-[2-(diméthylamino)éthyl]-N-(1-méthyléthyl)urée ou
le [1:1] maléate de N-[2-(2,3-dichlorophénoxy)éthyl]-N'-[2-(diméthylamino)éthyl]-N-(1-méthyléthyl)-urée.

15. Composé suivant la revendication 1 ou 2, qui est
la N'-[2-(diméthylamino)éthyl]-N-[2-(3,4-diméthylphénoxy)éthyl]-N-(1-méthyléthyl)urée,
le [1,5:1] fumarate de N'-[2-(diméthylamino)éthyl]-N-[2-(3,4-diméthylphénoxy)éthyl]-N-(1-méthyl-éthyl)urée,
la N'-[2-(diméthylamino)éthyl]-N-[2-(3,4-diméthylphénoxy)éthyl]-N-(1-méthyléthyl)urée ou
le [1:1] fumarate de N'-[2-(diméthylamino)éthyl]-N-[2-(3,4-diméthylphénoxy)éthyl]-N-(1-méthyléthyl)-urée.

16. Composé suivant la revendication 1 ou 2, qui est
la N'-[2-(diméthylamino)éthyl]-N-[2-(3,5-diméthylphénoxy)éthyl]-N-(1-méthyléthyl)urée ou
le [1:1] fumarate de N'-[2-(diméthylamino)éthyl]-N-[2-(3,5-diméthylphénoxy)éthyl]-N-(1-méthyléthyl)-urée.

17. Composé suivant la revendication 1 ou 2, qui est
la N-[2-(3,5-diméthoxyphénoxy)éthyl]-N'-[2-(diméthylamino)éthyl]-N-(1-méthyléthyl)urée ou
le [1:1] fumarate de N-[2-(3,5-diméthoxyphénoxy)éthyl]-N'-[2-(diméthylamino)éthyl]-N-(1-méthyl-éthyl)urée.

18. Composé suivant la revendication 1 ou 2, qui est
la N-[2-(3-chlorophénoxy)éthyl]-N'-[2-(diméthylamino)éthyl]-N-(1-méthyléthyl)urée,
le [1:1] maléate de N-[2-(3-chlorophénoxy)éthyl]-N'-[2-(diméthylamino)éthyl]-N-(1-méthyléthyl)urée,

la N-[2-(3-chlorophénoxy)éthyl]-N'-[2-(diméthylamino)éthyl]-N-(1-méthyléthyl)urée,
le [1:1] maléate de N-[2-(3-chlorophénoxy)éthyl]-N'-[2-(diméthylamino)éthyl]-N-(1-méthyléthyl)urée,
la N-[2-(2-chlorophénoxy)éthyl]-N'-[2-(diméthylamino)éthyl]-N-(1-méthyléthyl)urée ou
le [1:1] maléate de N-[2-(2-chlorophénoxy)éthyl]-N'-[2-(diméthylamino)éthyl]-N-(1-méthyléthyl)urée.

19. Composé suivant la revendication 1 ou 2, qui est
la N'-[2-(diméthylamino)éthyl]-N-[2-(3-fluorophénoxy)éthyl]-N-(1-méthyléthyl)urée ou
le [1:1] fumarate de N'-[2-(diméthylamino)éthyl]-N-[2-(3-fluorophénoxy)éthyl]-N-(1-méthyléthyl)urée.

20. Composé suivant la revendication 1 ou 2, qui est
la N'-[2-(diméthylamino)éthyl]-N-(1-méthyléthyl)-N-[2-(3-trifluorométhylphénoxy)éthyl]urée ou
le [1:1] fumarate de N'-[2-(diméthylamino)éthyl]-N-(1-méthyl)-N-[2-(3-trifluorométhylphénoxy)éthyl]-urée.

21. Composé suivant la revendication 1 ou 2, qui est
la N'-[2-(diméthylamino)éthyl]-N-(1-méthyléthyl)-N-[2-(1-naphtalényloxy)éthyl]urée,
le [1:1] maléate de N'-[2-(diméthylamino)éthyl]-N-(1-méthyléthyl)-N-[2-(1-naphtalényloxy)éthyl]urée,
la N'-[2-(diméthylamino)éthyl]-N-(1-méthyléthyl)-N-[2-(2-naphtalényloxy)éthyl]urée ou
le chlorhydrate de N'-[2-(diméthylamino)éthyl]-N-(1-méthyléthyl)-N-[2-(2-naphtalényloxy)éthyl]urée.

22. Composé suivant la revendication 1 ou 2, qui est
la N'-[2-(diméthylamino)éthyl]-N-[2-[(2,3-dihydro-1H-indén-5-yl)oxy]éthyl]-N-(1-méthyléthyl)urée,
le [1:1] maléate de N'-[2-(diméthylamino)éthyl]-N-[2-[(2,3-dihydro-1H-indén-5-yl)oxy]éthyl]-N-(1-méthyléthylurée),
la N'-[2-(diméthylamino)éthyl]-N-[2-[(2,3-dihydro-1H-indén-4-yl)oxy]éthyl]-N-(1-méthyléthyl)urée,
le [1:1] maléate de N'-[2-(diméthylamino)éthyl]-N-[2-[(2,3-dihydro-1H-indén-4-yl)oxy]éthyl]-N-(1-méthyléthyl)urée,
la N'-[2-[(2,3-dihydro-1H-indén-4-yl)oxy]éthyl]-N-[2-(méthylamino)éthyl]-N-méthylurée,
le [1:1] fumarate de N'-[2-[(2,3-dihydro-1H-indén-4-yl)oxy]éthyl]-N-[2-(méthylamino)éthyl]-N-méthyl-urée,
la N-[2-[(2,3-dihydro-1H-indén-4-yl)oxy]éthyl]-N'-[2-(diméthylamino)éthyl]-N'-(1-méthyléthyl)urée ou
le [1:1] maléate de N-[2-[(2,3-dihydro-1H-indén-4-yl)oxy]éthyl]-N'-[2-(diméthylamino)éthyl]-N'-(1-méthyléthyl)urée.

23. Composé suivant la revendication 1 ou 2, qui est
la N-[2-(diméthylamino)éthyl]-N'-(1-méthyléthyl)-N'-(2-phénoxyéthyl)urée, ou
le [1:1] fumarate de N-[2-(diméthylamino)éthyl]-N'-(1-méthyléthyl)-N'-(2-phénoxyéthyl)urée.

24. Composé suivant la revendication 1 ou 2, qui est
la N-[2-(3,5-dichlorophénoxy)éthyl]-N'-[2-(diméthylamino)éthyl]-N-(1-méthyléthyl)thiourée,
le [1:2] fumarate de N-[2-(3,5-dichlorophénoxy)éthyl]-N'-[2-(diméthylamino)éthyl]-N-(1-méthyléthyl)-thiourée hydraté,
la N-[2-(diéthylamino)éthyl]-N'-(1-méthyléthyl)-N'-(2-phénoxyéthyl)thiourée,
le [1:1] oxalate de N-[2-(diéthylamino)éthyl]-N'-(1-méthyléthyl)-N'-(2-phénoxyéthyl)thiourée,
la N-[2-(diméthylamino)éthyl]-N-(1-méthyléthyl)-N-[2-(1-naphtalényloxyéthyl]thiourée,
le [1:1] maléate de N'-[2-(diméthylamino)éthyl]-N-(1-méthyléthyl)-N-[2-(1-naphtalényloxy)éthyl]-thiourée,
la N'-[2-(diméthylamino)éthyl]-N-méthyl-N-(2-phénoxyéthyl)thiourée ou
le citrate de N'-[2-(diéthylamino)éthyl]-N-méthyl-N-(2-phénoxyéthyl)thiourée hémihydraté.

25. Composition thérapeutique ayant une activité d'inhibition de l'arythmie cardiaque, qui comprend (a) une quantité efficace d'un composé suivant l'une quelconque des revendications 1 à 24 (b) un excipient pharmaceutiquement acceptable pour ce composé.

26. Capsules, comprimés, ampoules ou suppositoires contenant
(a) des composés de formule

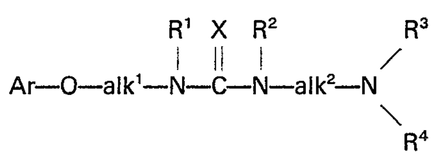

où Ar représente un radical 1- ou 2-naphtyle, 2,3-dihydro-1-H-indén-4(ou 5)yle, phényle ou phényle substitué, ce radical phényle substitué étant substitué par 1—3 radicaux qui peuvent être identiques ou différents et sont choisis parmi les atomes d'halogène et radicaux alkyle inférieurs, alkoxy inférieurs, trifluorométhyle, amino, nitro, alkylthio inférieurs, alkylsulfinyle inférieurs, alkylsulfonyle inférieurs, alkanoyle inférieurs, aroylamino et acylamino;

$R^1$ et $R^2$ représentent chacun un atome d'hydrogène ou un radical alkyle inférieur, cycloalkyle, phényle ou phényl-alkyle inférieur;

X représente un atome d'oxygène ou un atome de soufre;

$R^3$ et $R^4$ représentent chacun un atome d'hydrogène ou un radical alkyle inférieur, phényle ou phénylalkyle où le radical phényle peut être substitué par un atome d'halogène ou un radical alkyle

inférieur ou alkoxy inférieur et $R^3$ et $R^4$ peuvent être identiques ou différents ou bien $R^3$ et $R^4$ pris ensemble avec l'atome d'azote adjacent peuvent former un reste hétérocyclique;

alk[1] et alk[2] représentent chacun un radical alkylène inférieur ou alkylène inférieur-alkyle inférieur et peuvent être identiques ou différents ou un sel d'addition pharmaceutiquement acceptable ou hydrates de ceux-ci et

(b) un excipient pharmaceutique pour ces composés.

27. Composés de formule:

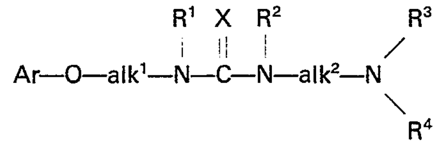

où Ar représente un radical 1- ou 2-naphtyle, 2,3-dihydro-1-H-indén-4(ou 5)yle, phényle ou phényle substitué, ce radical phényle substitué étant substitué par 1—3 radicaux qui peuvent être identiques ou différents et sont choisis parmi les atomes d'halogène et radicaux alkyle inférieurs, alkoxy inférieurs, trifluorométhyle, amino, nitro, alkylthio inférieurs, alkylsulfinyle inférieurs, alkylsulfonyle inférieurs, alkanoyle inférieurs, aroylamino et acylamino;

$R^1$ et $R^2$ représentent chacun un atome d'hydrogène ou un radical alkyle inférieur, cycloalkyle, phényle ou phényl-alkyle inférieur;

X représente un atome d'oxygène ou un atome de soufre;

$R^3$ et $R^4$ représentent chacun un atome d'hydrogène ou un radical alkyle inférieur, phényle ou phénylalkyle où le radical phényle peut être substitué par un atome d'halogène ou un radical alkyle inférieur ou alkoxy inférieur et $R^3$ et $R^4$ peuvent être identiques ou différents ou bien $R^3$ et $R^4$ pris ensemble avec l'atome d'azote adjacent peuvent former un reste hétérocyclique;

alk[1] et alk[2] représentent chacun un radical alkylène inférieur ou alkylène inférieur-alkyle inférieur et peuvent être identiques ou différents,

autres que la N'-[2-(diéthylamino)éthyl]-N-méthyl-N-[2-(phénoxy)éthyl]urée et

la N-méthyl-N-[2-(phénoxy)éthyl]-N-[2-(pyrrolidinyl)éthyl]urée,

et leurs sels d'addition pharmaceutiquement acceptables et hydrates,

à utiliser pour le traitement d'arythmies.

**Revendications pour l'Etat Contractant: AT**

1. Procédé de préparation d'un composé de formule générale I

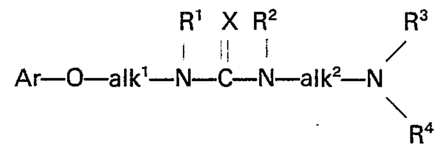

(I)

où Ar représente un radical 1- ou 2-naphtyle, 2,3-dihydro-1-H-indén-4(ou 5)yle, phényle ou phényle substitué, ce radical phényle substitué étant substitué par 1—3 radicaux qui peuvent être identiques ou différents et sont choisis parmi les atomes d'halogène et radicaux alkyle inférieurs, alkoxy inférieurs, trifluorométhyle, amino, nitro, alkylthio inférieurs, alkylsulfinyle inférieurs, alkylsulfonyle inférieurs, alkanoyle inférieurs, aroylamino et acylamino;

$R^1$ et $R^2$ représentent chacun un atome d'hydrogène ou un radical alkyle inférieur, cycloalkyle, phényle ou phényl-alkyle inférieur;

X représente un atome d'oxygène ou un atome de soufre;

$R^3$ et $R^4$ représentent chacun un atome d'hydrogène ou un radical alkyle inférieur, phényle ou phénylalkyle où le radical phényle peut être substitué par un atome d'halogène ou un radical alkyle inférieur ou alkoxy inférieur et $R^3$ et $R^4$ peuvent être identiques ou différents ou bien $R^3$ et $R^4$ pris ensemble avec l'atome d'azote adjacent peuvent former un reste hétérocyclique;

alk[1] et alk[2] représentent chacun un radical alkylène inférieur ou alkylène inférieur-alkyle inférieur et peuvent être identiques ou différents,

autre que la N'-[2-(diéthylamino)éthyl]-N-méthyl-N-[2-(phénoxy)éthyl]urée et

la N-méthyl-N-[2-(phénoxy)éthyl]-N'-[2-(pyrrolidinyl)éthyl]urée,

ou d'un sel d'addition pharmaceutiquement acceptable ou hydrate de celui-ci, le procédé comprenant:

(A) la réaction d'un composé de formule générale IIa

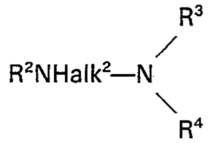

(IIa)

où $R^2$, $alk^2$, $R^3$ et $R^4$ sont tels que définis à propos de la formule I, avec la restriction que lorsque $R^2$ ne représente pas un atome d'hydrogène, $R^3$ et $R^4$ doivent représenter autre chose qu'un atome d'hydrogène, ou bien $R^2$ est le même que $R^3$ et $R^4$ représente un atome d'hydrogène, avec un composé de formule générale III

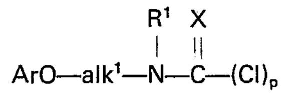

(III)

où Ar, $alk^1$, $R^1$ et X sont tels que définis à propos de la formule générale I et lorsque $R^1$ représente un atome d'hydrogène, p représente zéro et la ligne en pointillés est une double liaison formant un isocyanate; au cas contraire, p représente un et la ligne en pointillés est sans signification;

(B) lorsque X représente un atome d'oxygène et que $R^2$ représente un atome d'hydrogène, la réaction d'un composé de formule générale IIb

$$H_2N—alk^2NR^3R^4 \qquad (IIb)$$

où $alk^2$, $R^3$ et $R^4$ sont tels que définis à propos de la formule générale I, et d'un composé de formule générale IVa

$$ArO—alk^1—NHR^1 \qquad (IVa)$$

où Ar, $alk^1$ et $R^1$ sont tels que définis à propos de la formule générale I, avec le 1,1'-carbonyldiimidazole;

(C) lorsque X représente un atome d'oxygène et que $R^1$ représente un atome d'hydrogène, la réaction d'un composé de formule générale IIb'

$$R^2NH—alk^2—NR^3R^4 \qquad (IIb')$$

où $R^2$, $alk^2$, $R^3$ et $R^4$ sont tels que définis à propos de la formule générale I, et d'un composé de formule générale IVb

$$Ar—O—alk^1—NH_2 \qquad (IVb)$$

où Ar et $alk^1$ sont tels que définis à propos de la formule générale I, avec le 1,1'-carbonyldiimidazole, ou

(D) la réaction d'un composé de formule générale V

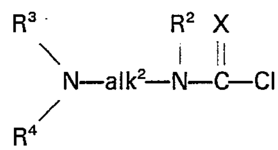

(V)

où X, $R^2$, $alk^2$, $R^3$ et $R^4$ sont tels que définis à propos de la formule générale I, sauf que $R^2$, $R^3$ et $R^4$ ne représentent jamais un atome d'hydrogène, et d'un composé de formule générale IVa

$$Ar—O—alk^1—NHR^1 \qquad (IVa)$$

où Ar, $alk^1$ et $R^1$ sont tels que définis à propos de la formule générale I ci-dessus, avec la triéthylamine et ensuite, si la chose est souhaitée, la formation d'un sel d'addition d'acide pharmaceutiquement acceptable ou hydrate d'un composé ainsi formé.

2. Procédé suivant la revendication 1, dans lequel Ar représente un radical phényle substitué.

3. Procédé suivant la revendication 2, dans lequel Ar représente un radical dichlorophényle.

4. Procédé suivant la revendication 3, dans lequel Ar représente un radical, $3,4—Cl_2—C_6H_3—$ ou $3,5—Cl_2C_6H_3—$.

5. Procédé suivant la revendication 1, 2 ou 3, dans lequel $R^1$ représente un radical $—CH(CH_3)_3$.

6. Procédé suivant l'une quelconque des revendications 1 à 5, dans lequel X représente un atome de soufre.

7. Procédé suivant la revendication 2, dans lequel Ar représente un radical, 3,5-dichlorophényle, $alk^1$ et $alk^2$ représentent tous deux un radical $—(CH_2)_2—$ et $R^3$ er $R^4$ représentent tous deux un radical méthyle.

8. Procédé suivant la revendication 1 de préparation
de la N-[2-(3,4-dichlorophénoxy)éthyl]-N'-[2-(diméthylamino)-éthyl]-N-(1-méthyléthyl)urée ou
du monochlorhydrate de N-[2-(3,4-dichlorophénoxy)éthyl]-N'-[2-(diméthylamino)éthyl]-N-(1-méthyléthyl)urée monohydraté,
de la N-[2-(3,4-dichlorophénoxy)éthyl]-N'-[3-(diméthylamino)propyl]-N-(1-méthyléthyl)urée ou
du [1:1] oxalate de N-[2-(3,4-dichlorophénoxy)éthyl]-N'-[3-(diméthylamino)propyl]-N-(1-méthyléthyl)-urée.

9. Procédé suivant la revendication 1 de préparation
de la N-[2-(3,5-dichlorophénoxy)éthyl]-N'-[2-(diméthylamino)éthyl]-N-(1-méthyléthyl)urée,
du [1:1] maléate de N-[2-(3,5-dichlorophénoxy)éthyl]-N'-[2-(diméthylamino)éthyl]-N-(1-méthyléthyl)-urée,
de la N-[3-(3,5-dichlorophénoxy)propyl]-N'-[2-(diméthylamino)éthyl]-N-(1-méthyléthyl)urée,
du [1:1] oxalate de N-[3-(3,5-dichlorophénoxy)propyl]-N'-[2-(diméthylamino)éthyl]-N-(1-méthyléthyl)-urée,
de la N-[3-(3,5-dichlorophénoxy)propyl]-N'-[3-(diméthylamino)propyl]-N-(1-méthyléthyl)urée,
du [1:1] citrate de N-[3-(3,5-dichlorophénoxy)propyl]-N'-[3-(diméthylamino)propyl]-N-(1-méthyléthyl)-urée,
de la N-[2-(3,5-dichlorophénoxy)éthyl]-N'-[3-(diméthylamino)propyl]-N-(1-méthyléthyl)urée,
du [1:1] citrate de N-[2-(3,5-dichlorophénoxy)éthyl]-N'-[3-(diméthylamino)propyl]-N-(1-méthyléthyl)-urée,
de la N-[2-(3,5-dichlorophénoxy)éthyl]-N-(1-méthyléthyl)-N'-[2-[bis(1-méthyléthyl)amino]éthyl]urée,
du [1:1] chlorhydrate de N-[2-(3,5-dichlorophénoxy)éthyl]-N-(1-méthyléthyl)-N'-[2-[bis(1-méthyléthyl)-amino]éthyl]urée,
de la N-[2-(3,5-dichlorophénoxy)éthyl]-N'-[2-(diméthylamino)éthyl]-N-phénylurée,
du monochlorhydrate de N-[2-(3,5-dichlorophénoxy)éthyl]-N'-[2-(diméthylamino)éthyl]-N-phénylurée,
de la N-[2-(3,5-dichlorophénoxy)éthyl]-N'-[2-(diméthylamino)éthyl]-N'-méthyl-N-(1-méthyléthyl)urée,
du [1:1] citrate de N-[2-(3,5-dichlorophénoxy)éthyl]-N'-[2-(diméthylamino)éthyl]-N'-méthyl-N-(1-méthyléthyl)urée,
de la N-[2-(3,5-dichlorophénoxy)éthyl]-N'-[2-(diméthylamino)éthyl]urée,
du [1:1] fumarate de N-[2-(3,5-dichlorophénoxy)éthyl]-N'-[2-(diméthylamino)éthyl]urée,
de la N-[2-(3,5-dichlorophénoxy)éthyl]-N-(1-méthyléthyl)-N'-[2-(4-morpholinyl)éthyl]urée,
du [1:1] fumarate de N-[2-(3,5-dichlorophénoxy)éthyl]-N-(1-méthyléthyl)-N'-[2-(4-morpholinyl)éthyl]-urée,
de la N-[2-(3,5-dichlorophénoxy)éthyl]-N'-[2-(diméthylamino)éthyl]-N-méthylurée,
du [1:1] maléate de N-[2-(3,5-dichlorophénoxy)éthyl]-N'-[2-(diméthylamino)éthyl]-N-méthylurée,
de la N-[2-(3,5-dichlorophénoxy)éthyl]-N'-méthyl-N'-[2-(méthylamino)éthyl]-N-(1-méthyléthyl)urée,
du [1:1] maléate de N-[2-(3,5-dichlorophénoxy)éthyl]-N'-méthyl-N'-[2-(méthylamino)éthyl]-N-(1-méthyléthyl)urée,
de la N-butyl-N-[2-(3,5-dichlorophénoxy)éthyl]-N'-[2-(diméthylamino)éthyl]urée,
du [1:1] maléate de N-butyl-N-[2-(3,5-dichlorophénoxy)éthyl]-N'-[2-(diméthylamino)éthyl]urée,
de la N-[2-(3,5-dichlorophénoxy)éthyl]-N'-[2-(diméthylamino)éthyl]-N-propylurée,
du [1:1] maléate de N-[2-(3,5-dichlorophénoxy)éthyl]-N'-[2-(diméthylamino)éthyl]-N-propylurée,
de la N-cyclohexyl-N-[2-(3,5-dichlorophénoxy)éthyl]-N'-[2-(diméthylamino)éthyl]urée,
du [1:1] maléate de N-cyclohexyl-N-[2-(3,5-dichlorophénoxy)éthyl]-N'-[2-(diméthylamino)éthyl]urée,
de la N-[2-(3,5-dichlorophénoxy)éthyl]-N'-[2-(diméthylamino)éthyl]-N-(2-méthylpropyl)urée,
du [1:1] maléate de N-[2-(3,5-dichlorophénoxy)éthyl]-N'-[2-(diméthylamino)éthyl]-N-(2-méthylpropyl)-urée,
de la N'-[2-(3,5-dichlorophénoxy)éthyl]-N-[2-(diméthylamino)éthyl]-N-méthylurée,
du [1:1] maléate de N'-[2-(3,5-dichlorophénoxy)éthyl]-N-[2-(diméthylamino)éthyl]-N-méthylurée,
de la N-[2-(3,5-dichlorophénoxy)éthyl]-N-(1-méthyléthyl)-N'-(2-aminoéthyl)urée,
du [1:1] maléate de N-[2-(3,5-dichlorophénoxy)éthyl]-N-(1-méthyléthyl)-N'-(2-aminoéthyl)urée,
de la N-[2-(3,5-dichlorophénoxy)éthyl]-N'-[2-(diéthylamino)éthyl]-N-(1-méthyléthyl)urée,
du [1:1] tartrate de N-[2-(3,5-dichlorophénoxy)éthyl]-N'-[2-(diéthylamino)éthyl]-N-(1-méthyléthyl)urée,
de la N-[2-(3,5-dichlorophénoxy)éthyl]-N'-[2-(diméthylamino)éthyl]-N-(1-méthyléthyl)urée hydratée [2:1],
de la N-[2-[(3,5-dichlorophényl)oxy]éthyl]-N'-[2-(diméthylamino)éthyl]-N'-(1-méthyléthyl)urée ou
du maléate de N-[2-[(3,5-dichlorophényl)oxy]éthyl]-N'-[2-(diméthylamino)éthyl]-N'-(1-méthyléthyl)-urée.

10. Procédé suivant la revendication 1 de préparation
de la N-[2-(2,4-dichlorophénoxy)éthyl]-N'-[2-(diméthylamino)-éthyl]-N-(1-méthyléthyl)urée ou
du [1:1] maléate de N-[2-(2,4-dichlorophénoxy)éthyl]-N'-[2-(diméthylamino)éthyl]-N-(1-méthyléthyl)-urée.

11. Procédé suivant la revendication 1 de préparation
de la N-[2-(2,6-dichlorophénoxy)éthyl]-N'-[2-(diméthylamino)éthyl]-N-(1-méthyléthyl)urée.

12. Procédé suivant la revendication 1 de préparation

de la N-[2-(2,5-dichlorophénoxy)éthyl]-N'-[2-(diméthylamino)éthyl]-N-(1-méthyléthyl)urée ou
du [1:1] maleate de N-[2-(2,5-dichlorophénoxy)éthyl]-N'-[2-(diméthylamino)éthyl]-N-(1-méthyléthyl)-urée.

13. Procédé suivant la revendication 1 de préparation
de la N-[2-(2,3-dichlorophénoxy)éthyl]-N'-[2-(diméthylamino)éthyl]-N-(1-méthyléthyl)urée ou
du [1:1] maléate de N-[2-(2,3-dichlorophénoxy)éthyl]-N'-[2-(diméthylamino)éthyl]-N-(1-méthyléthyl)-urée.

14. Procédé suivant la revendication 1 de préparation
de la N'-[2-(diméthylamino)éthyl]-N-[2-(3,4-diméthylphénoxy)éthyl]-N-(1-méthyléthyl)urée,
du [1,5:1] fumarate de N'-[2-(diméthylamino)éthyl]-N-[2-(3,4-diméthylphénoxy)éthyl]-N-(1-méthyléthyl)urée,
de la N'-[2-(diméthylamino)éthyl]-N-[2-(3,4-diméthylphénoxy)éthyl]-N-(1-méthyléthyl)urée ou
du [1:1] fumarate de N'-[2-(diméthylamino)éthyl]-N-[2-(3,4-diméthylphénoxy)éthyl]-N-(1-méthyléthyl)-urée.

15. Procédé suivant la revendication 1 de préparation
de la N'-[2-(diméthylamino)éthyl]-N-[2-(3,5-diméthylphénoxy)éthyl]-N-(1-méthyléthyl)urée ou
du [1:1] fumarate de N'-[2-(diméthylamino)éthyl]-N-[2-(3,5-diméthylphénoxy)éthyl]-N-(1-méthyléthyl)-urée.

16. Procédé suivant la revendication 1 de préparation
de la N-[2-(3,5-diméthoxyphénoxy)éthyl]-N'-[2-(diméthylamino)éthyl]-N-(1-méthyléthyl)urée ou
du [1:1] fumarate de N-[2-(3,5-diméthoxyphénoxy)éthyl]-N'-[2-(diméthylamino)éthyl]-N-(1-méthyléthyl)urée.

17. Procédé suivant la revendication 1 de préparation
de la N-[2-(3-chlorophénoxy)éthyl]-N'-[2-(diméthylamino)éthyl]-N-(1-méthylethyl)urée,
du [1:1] maléate de N-[2-(3-chlorophénoxy)éthyl]-N'-[2-(diméthylamino)éthyl]-N-(1-méthyléthyl)urée,
de la N-[2-(3-chlorophénoxy)éthyl]-N'-[2-(diméthylamino)éthyl]-N-(1-méthyléthyl)urée,
du [1:1] maléate de N-[2-(3-chlorophénoxy)éthyl]-N'-[2-(diméthylamino)éthyl]-N-(1-méthyléthyl)urée,
de la N-[2-(2-chlorophénoxy)éthyl]-N'-[2-(diméthylamino)éthyl]-N-(1-méthyléthyl)urée ou
du [1:1] maléate de N-[2-(2-chlorophénoxy)éthyl]-N'-[2-(diméthylamino)éthyl]-N-(1-méthyléthyl)urée.

18. Procédé suivant la revendication 1 de préparation
de la N'-[2-(diméthylamino)éthyl]-N-[2-(3-fluorophénoxy)éthyl]-N-(1-méthyléthyl)urée ou
du [1:1] fumarate de N'-[2-(diméthylamino)éthyl]-N-[2-(3-fluorophénoxy)éthyl]-N-(1-méthyléthyl)urée.

19. Procédé suivant la revendication 1 de préparation
de la N'-[2-(diméthylamino)éthyl]-N-(1-méthyléthyl)-N-[2-(3-trifluorométhylphénoxy)éthyl]urée ou
du [1:1] fumarate de N'-[2-(diméthylamino)éthyl]-N-(1-méthyl)-N-[2-(3-trifluorométhylphénoxy)éthyl]-urée.

20. Procédé suivant la revendication 1 de préparation
de la N'-[2-(diméthylamino)éthyl]-N-(1-méthyléthyl)-N-[2-(1-naphtalényloxy)éthyl]urée,
du [1:1] maléate de N'-[2-(diméthylamino)éthyl]-N-(1-méthyléthyl)-N-[2-(1-naphtalényloxy)éthyl]urée,
de la N'-[2-(diméthylamino)éthyl]-N-(1-méthyléthyl)-N-[2-(2-naphtalényloxy)éthyl]urée ou
du chlorhydrate de N'-[2-(diméthylamino)éthyl]-N-(1-méthyléthyl)-N-[2-(2-naphtalényloxy)éthyl]urée.

21. Procédé suivant la revendication 1 de préparation
de la N'-[2-(diméthylamino)éthyl]-N-[2-[(2,3-dihydro-1H-indén-5-yl)oxy]éthyl]-N-(1-méthyléthyl)urée,
du [1:1] maléate de N'-[2-(diméthylamino)éthyl]-N-[2-[(2,3-dihydro-1H-indén-5-yl)oxy]éthyl]-N-(1-méthyléthylurée),
de la N'-[2-(diméthylamino)éthyl]-N-[2-[(2,3-dihydro-1H-indén-4-yl)oxy]éthyl]-N-(1-méthyléthyl)urée,
du [1:1] maléate de N'-[2-(diméthylamino)éthyl]-N-[2-[(2,3-dihydro-1H-indén-4-yl)oxy]éthyl]-N-(1-méthyléthyl)urée,
de la N'-[2-[(2,3-dihydro-1H-indén-4-yl)oxy]éthyl]-N-[2-(méthylamino)éthyl]-N-méthylurée,
du [1:1] fumarate de N'-[2-[(2,3-dihydro-1H-indén-4-yl)oxy]éthyl]-N-[2-(méthylamino)éthyl]-N-méthyl-urée,
de la N-[2-[(2,3-dihydro-1H-indén-4-yl)oxy]éthyl]-N'-[2-(diméthylamino)éthyl]-N'-(1-méthyléthyl)urée ou
du [1:1] maléate de N-[2-[(2,3-dihydro-1H-indén-4-yl)oxy]éthyl]-N'-[2-(diméthylamino)éthyl]-N'-(1-méthyléthyl)urée.

22. Procédé suivant la revendication 1 de préparation
de la N-[2-(diméthylamino)éthyl]-N'-(1-méthyléthyl)-N'-(2-phénoxyéthyl)urée, ou
du [1:1] fumarate de N-[2-(diméthylamino)éthyl]-N'-(1-méthyléthyl)-N'-(2-phénoxyéthyl)urée.

23. Procédé suivant la revendication 1 de préparation
de la N-[2-(3,5-dichlorophénoxy)éthyl]-N'-[2-(diméthylamino)-éthyl]-N-(1-méthyléthyl)thiourée,
du [1:1] fumarate de N-[2-(3,5-dichlorophénoxy)éthyl]-N'-[2-(diméthylamino)éthyl]-N-(1-méthyléthyl)-thiourée,
de la N-[2-(diéthylamino)éthyl]-N'-(1-méthyléthyl)-N'-(2-phénoxyéthyl)thiourée,
du [1:1] oxalate de N-[2-(diéthylamino)éthyl]-N'-(1-méthyléthyl)-N'-(2-phénoxyéthyl)thiourée,
de la N-[2-(diméthylamino)éthyl]-N-(1-méthyléthyl)-N-[2-(1-naphtalényloxy)éthyl]thiourée,

du [1:1] maléate de N'-[2-(diméthylamino)éthyl]-N-(1-méthyléthyl)-N-[2-(1-naphtalényloxy)éthyl]thiourée,

de la N'-[2-(diméthylamino)éthyl]-N-méthyl-N-(2-phénoxyéthyl)thiourée ou

du citrate de N'-[2-(diéthylamino)éthyl]-N-méthyl-N-(2-phénoxyéthyl)thiourée hémihydraté.

24. Procédé de préparation d'une composition thérapeutique ayant une activité d'inhibition de l'arythmie cardiaque, qui comprend le mélange

(a) d'une quantité efficace d'un composé du formule:

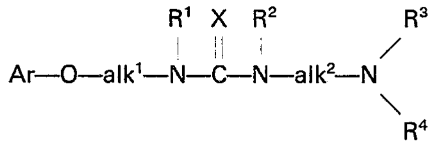

où Ar représente un radical 1- ou 2-naphtyle, 2,3-dihydro-1-H-indén-4(ou 5)yle, phényle ou phényle substitué, ce radical phényle substitué étant substitué par 1—3 radicaux qui peuvent être identiques ou différents et sont choisis parmi les atomes d'halogène et radicaux alkyle inférieurs, alkoxy inférieurs, trifluorométhyle, amino, nitro, alkylthio, inférieurs, alkylsulfinyle inférieurs, alkylsulfonyle inférieurs, alkanoyle inférieurs, aroylamino et acylamino;

$R^1$ et $R^2$ représentent chacun un atome d'hydrogène ou un radical alkyle inférieur, cycloalkyle, phényle ou phényl-alkyle inférieur;

X représente un atome d'oxygène ou un atome de soufre;

$R^3$ et $R^4$ représentent chacun un atome d'hydrogène ou un radical alkyle inférieur, phényle ou phénylalkyle où le radical phényle peut être substitué par un atome d'halogène ou un radical alkyle inférieur ou alkoxy inférieur et $R^3$ et $R^4$ peuvent être identiques ou différents ou bien $R^3$ et $R^4$ pris ensemble avec l'atome d'azote adjacente peuvent former un reste hétérocyclique;

$alk^1$ et $alk^2$ représentent chacun un radical alkylène inférieur ou alkylène inférieur-alkyle inférieur et peuvent être identiques ou différents, ou d'un sel ou hydrate de ce composé et

(b) d'un excipient pharmaceutiquement acceptable pour ce composé.

25. Procédé de préparation d'une composition thérapeutique ayant une activité d'inhibition de l'arythmie cardiaque, qui comprend le mélange d'un composé préparé par un procédé suivant l'une quelconque des revendications 1 à 24 avec un excipient pharmaceutiquement acceptable pour ce composé.

26. Procédé de préparation de capsule, de comprimés, d'ampoules ou de suppositoires ayant une activité d'inhibition de l'arythmie cardiaque, le procédé comprenant le mélange

(a) de composés de formule:

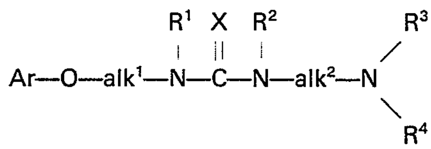

où Ar représente un radical 1- ou 2-naphtyle, 2,3-dihydro-1-H-indén-4(ou 5)yle, phényle ou phényle substitué, ce radical phényle substitué étant substitué par 1—3 radicaux qui peuvent être identiques ou différents et sont choisis parmi les atomes d'halogène et radicaux alkyle inférieurs, alkoxy inférieurs, trifluorométhyle, amino, nitro, alkylthio inférieurs, alkylsulfinyle inférieurs, alkylsulfonyle inférieurs, alkanoyle inférieurs, aroylamino et acylamino;

$R^1$ et $R^2$ représentent chacun un atome d'hydrogène ou un radical alkyle inférieur, cycloalkyle, phényle ou phényl-alkyle inférieur;

X représente un atome d'oxygène ou un atome de soufre;

$R^3$ et $R^4$ représentent chacun un atome d'hydrogène ou un radical alkyle inférieur, phényle ou phénylalkyle où le radical phényle peut être substitué par un atome d'halogène ou un radical alkyle inférieur ou alkoxy inférieur et $R^3$ et $R^4$ peuvent être identiques ou différents ou bien $R^3$ et $R^4$ pris ensemble avec l'atome d'azote adjacent peuvent former un reste hétérocyclique;

$alk^1$ et $alk^2$ représentent chacun un radical alkylène inférieur ou alkylène inférieur-alkyle inférieur et peuvent être identiques ou différents, ou d'un sel ou hydrate de ces composés et

(b) d'un excipient pharmaceutique pour ces composés et ensuite la préparation de capsules, comprimés, ampoules ou suppositoires à partir du mélange.